# EUROPEAN PATENT APPLICATION

(11) **EP 4 410 963 A1**
(43) Date of publication of application: **07.08.2024**
(21) Application number: 22876262.1
(22) Date of filing: 27.09.2022
(51) Int. Cl.: C12N 5/071, C12N 5/10

(54) **METHOD FOR PRODUCING CELL MASS INCLUDING PITUITARY TISSUE AND CELL MASS**

(30) Priority: 30.09.2021 JP 2021162255; 21.07.2022 JP 2022116716
(71) Applicant: Sumitomo Chemical Company, Limited, Tokyo 103-6020 (JP); Sumitomo Pharma Co., Ltd., Osaka 541-0045 (JP); National University Corporation Tokai National Higher Education and Research System, Nagoya-shi, Aichi 464-8601 (JP)
(72) Inventor: NAKANO, Tokushige, Osaka-shi, Osaka 554-8558 (JP); TAGA, Shiori, Kobe-shi, Hyogo 650-0047 (JP); KUWAHARA, Atsushi, Kobe-shi, Hyogo 650-0047 (JP); SUGA, Hidetaka, Nagoya-shi, Aichi 464-8601 (JP)
(74) Representative: J A Kemp LLP
(86) International application number: PCT/JP2022/036019
(87) International publication number: WO 2023/054396

(57) **Abstract**

The present invention aims to provide a method for efficiently producing a cell population containing pituitary tissue from pluripotent stem cells. The method for producing a cell population containing pituitary tissue includes the following steps (1) and (2):
(1) a first step of culturing the pluripotent stem cells in the presence of a c-jun N-terminal kinase (JNK) signal transduction pathway inhibiting substance and a Wnt signal transduction pathway inhibiting substance,
(2) a second step of culturing the cell population obtained in the first step in the presence of a BMP signal transduction pathway activating substance and a Sonic hedgehog signal transduction pathway activating substance, thereby obtaining a cell population containing pituitary tissue.

## Description

### [Technical Field]

The present invention relates to a method for producing a cell population containing pituitary tissue, and the cell population. Furthermore, it relates to a cell population containing neural cells or neural tissue, pituitary tissue, and mesenchymal cells.

The pituitary gland is an endocrine organ located in the head and produces various pituitary hormones that are important for the maintenance and growth of the body, such as adrenocorticotropic hormone (ACTH) and growth hormone. When pituitary dysfunction is caused by diseases such as pituitary hypoplasia, hypopituitarism, pituitary adenoma and the like, severe symptoms similar to growth disorder, genital-related abnormality, and abnormalities of the adrenal gland and thyroid gland occur. In general, disordered pituitary tissue rarely regenerates naturally and recovers function.

Patent Literature 1 and non-patent Literatures 1, 2 report that cranial placode-derived cells including pituitary cells were produced by inducing differentiation of human pluripotent stem cells in the presence of a BMP signal transduction pathway inhibiting substance or activating substance, a Sonic hedgehog (sometimes referred to as Shh in the present specification) signal transduction pathway activating substance, and a TGF-β signal transduction pathway inhibitor. However, the cells produced were those two-dimensionally cultured and the structure of complicated pituitary tissue of the body which is important for exhibiting functions has not been reproduced. Therefore, a method capable of efficiently producing three-dimensional pituitary tissue has been desired.

In Patent Literatures 2 to 4 and Non Patent Literatures 3, 4, the present inventors produced three-dimensional pituitary tissue from pluripotent stem cells and reported that the tissue has pituitary hormone production capacity.

### [Citation List]

### [Patent Literature]

[Patent Literature 1]
   JP-A- 2016-538856
[Patent Literature 2]
   WO2013/065763
[Patent Literature 3]
   WO2016/013669
[Patent Literature 4]
   WO2019/103129

### [Non Patent Literature]

[Non Patent Literature 1]
   Dincer et al. Cell Reports 5, 1387-1402, 2013.
[Non Patent Literature 2]
   Zimmer et al. Stem Cell Reports 6, 858-872, 2016.
[Non Patent Literature 3]
   Suga et al. Nature. 2011 Nov 9; 480(7375): 57-62.
[Non Patent Literature 4]
   Ozone et al. Nature communications 7.10351 (2016): 1-10.

### [Summary of Invention]

### [Technical Problem]

An object of the present invention is to provide a method for efficiently producing a cell population containing pituitary tissue from pluripotent stem cells. Particularly, the object is to provide methods for efficiently producing a cell population containing high quality pituitary tissue suitable for transplantation into human and regenerative medicine. Furthermore, the object is to identify and produce a cell population corresponding to an intermediate product suitable for the production and purification of final products. Specifically, it is to provide a method for efficiently producing a cell population which may use a feeder-free cultured pluripotent stem cell as a starting material, and permits reduction of the amount of expensive recombinant protein to be used to produce at a lower cost.

The present inventors have conducted intensive studies in an attempt to solve the above-mentioned problems and found that a cell population containing pituitary tissue can be efficiently produced by culturing pluripotent stem cells in the presence of a c-jun N-terminal kinase (JNK) signal transduction pathway inhibiting substance, and adding a BMP signal transduction pathway activating substance and a Sonic hedgehog signal transduction pathway activating substance. Furthermore, they have found that a cell population containing pituitary tissue can be produced more efficiently by simultaneously adding the aforementioned JNK signal transduction pathway inhibiting substance and a Wnt signal transduction pathway inhibiting substance.

That is, the present invention relates to the following.

[1] A method for producing a cell population containing pituitary tissue, comprising the following steps (1) and (2):
   (1) a first step of culturing the pluripotent stem cells in the presence of a c-jun N-terminal kinase (JNK) signal transduction pathway inhibiting substance and a Wnt signal transduction pathway inhibiting substance to obtain a cell population,
   (2) a second step of culturing the cell population obtained in the first step in the presence of a BMP signal transduction pathway activating substance and a Sonic hedgehog signal transduction pathway activating substance, thereby obtaining a cell population containing pituitary tissue.
[2] The production method of [1], wherein the pluripotent stem cell is subjected to the following step (a) before the first step:
   (a) step a of culturing pluripotent stem cells in the absence of feeder cells and in a medium containing 1) a TGFβ family signal transduction pathway inhibiting substance and/or a Sonic hedgehog signal transduction pathway activating substance, and 2) a factor for maintaining an undifferentiated state.
[2-1] The production method of [2], wherein the TGFβ family signal transduction pathway inhibiting substance to be added in the step (a) is an Alk5/TGFβR1 inhibitor and the Alk5/TGFβR1 inhibitor comprises at least one selected from the group consisting of SB431542, SB505124, SB525334, LY2157299, GW788388, LY364947, SD-208, EW-7197, A83-01, A77-01, RepSox, BIBF-0775, TP0427736, TGFBR1-IN-1, SM-16, TEW-7197, LY3200882, LY2109761, KRCA 0008, GSK 1838705, Crizotinib, Ceritinib, ASP 3026, TAE684, AZD3463, and derivatives of these.
[3] The production method of [1], wherein the culture in the first step is further in the presence of a Sonic hedgehog signal transduction pathway activating substance, and a period of the culture in the presence of a Sonic hedgehog signal transduction pathway activating substance in the first step and the second step is 30 days.
[4] The production method of any of [1], [2], [2-1], and [3], wherein the cell population obtained in the second step is subjected to the following step (3):
   (3) a third step of culturing the cell population obtained in the second step in the absence of a Sonic hedgehog signal transduction pathway activating substance to obtain a cell population containing pituitary tissue.
[5] The production method of [4], wherein the cell population obtained in the second step is subjected to the following step (b) before the third step:
   (b) step b of culturing the cell population obtained in the second step, in the presence of a BMP signal transduction pathway inhibiting substance.
[5-1] The production method of [5], wherein the BMP signal transduction pathway inhibiting substance to be added in the aforementioned step (b) comprises a type I BMP receptor inhibitor.
[5-2] The production method of [5-1], wherein the type I BMP receptor inhibitor comprises at least one selected from the group consisting of K02288, Dorsomorphin, LDN-193189, LDN-212854, LDN-214117, ML347, DMH1, DMH2, compound 1, VU5350, OD52, E6201, Saracatinib, BYL719, and derivatives of these.
[6] The production method of any of [1] to [5], [2-1], [5-1], and [5-2], wherein the JNK signal transduction pathway inhibiting substance comprises a JNK inhibitor.
[6-1] The production method of [6], wherein the JNK inhibitor comprises at least one selected from the group consisting of SP600125, JNK-IN-8, DB07268, IQ-1S, Tanzisertib, Bentamapimod, BI-78D3, BI-87G3, CC-401, TCS JNK 5a, AS601245, CV-65, D-JNK1, ER-358063, ER-409903, ER-417258, CC-359, CC-930, SB203580, and derivatives of these.
[6-2] The production method of [6] or [6-1], wherein the JNK inhibitor comprises JNK-IN-8 or SP600125, and the step (1) is initiated in a medium containing 1 nM to 50 µM of JNK-IN-8 or 1 nM to 25 µM of SP600125.
[7] The production method of any of [1] to [5], [2-1], [5-1], and [5-2], wherein the aforementioned JNK signal transduction pathway inhibiting substance comprises a Rac inhibitor.
[7-1] The production method of [7], wherein the Rac inhibitor comprises at least one selected from the group consisting of NSC23766, EHop-016, ZCL278, MBQ-167, KRpep-2d, ARS-853, Salirasib, ML141, EHT1864, and derivatives of these.
[8] The production method of any of [1] to [7], [2-1], [5-1], [5-2], [6-1], [6-2], and [7-1], wherein the aforementioned Wnt signal transduction pathway inhibiting substance comprises a substance with inhibitory activity against non-classical Wnt pathway.
[8-1] The production method of [8], wherein the substance with inhibitory activity against non-classical Wnt pathway is a PORCN inhibitor and the PORCN inhibitor comprises at least one selected from the group consisting of IWP-2, IWP-3, IWP-4, IWP-L6, IWP-12, IWP-O1, LGK-974, Wnt-C59, ETC-131, ETC-159, GNF-1331, GNF-6231, Porcn-IN-1, RXC004, CGX1321, and derivatives of these.
[9] The production method of any of [1] to [8], [2-1], [5-1], [5-2], [6-1], [6-2], [7-1], and [8-1], wherein a TGF signal transduction pathway inhibiting substance is further present in any one or more of the first step, second step, step b, and the third step.
[9-1] The production method of any of [1] to [9], [2-1], [5-1], [5-2], [6-1], [6-2], [7-1], and [8-1], wherein the Sonic hedgehog signal transduction pathway activating substance to be added in any one or more of the step a, the first step, the second step, and step b comprises at least one selected from the group consisting of SAG, Purmorphamine, and GSA-10.
[10] The production method of any of [1] to [9], [2-1], [5-1], [5-2], [6-1], [6-2], [7-1], [8-1], and [9-1], wherein a TAK1 inhibiting substance is further present in any one or more of the first step, the second step, step b, and the third step.
[10-1] The production method of [10], wherein the TAK1 inhibiting substance comprises at least one selected from the group consisting of (5Z)-7-Oxozeaenol, N-Des(aminocarbonyl)AZ-TAK1 inhibitor, Takinib, NG25, Sarsasapogenin, and derivatives of these.
[11] The production method of any of [1] to [10], [2-1], [5-1], [5-2], [6-1], [6-2], [7-1], [8-1], [9-1], and [10-1], wherein a FGF signal transduction pathway activating substance is further present in any one or more of the second step, step b, and the third step.
[11-1] The production method of [11], wherein the FGF signal transduction pathway activating substance comprises at least one selected from the group consisting of FGF2, FGF3, FGF8, FGF10, and variants thereof.
[12] The production method of any of [1] to [11], [2-1], [5-1], [5-2], [6-1], [6-2], [7-1], [8-1], [9-1], [10-1], and [11-1], wherein a substance having the action of reducing oxidative stress is further present in any one or more of the second step, step b, and the third step.
[12-1] The production method of [12], wherein the substance having the action of reducing oxidative stress comprises at least one selected from the group consisting of ascorbic acid, N-acetyl-L-cysteine, nicotine amide, and derivatives of these.
[13] The production method of any of [1] to [12], [2-1], [5-1], [5-2], [6-1], [6-2], [7-1], [8-1], [9-1], [10-1], [11-1], and [12-1], wherein an inhibiting substance against a stress response signal transduction pathway is further present in any one or more of the second step, step b, and the third step.
[14] The production method of any of [1] to [13], [2-1], [5-1], [5-2], [6-1], [6-2], [7-1], [8-1], [9-1], [10-1], [11-1], and [12-1], wherein cells are cultivated while shaking in any one or more of the second step, step b, and the third step.
[15] The production method of any of [1] to [14], [2-1], [5-1], [5-2], [6-1], [6-2], [7-1], [8-1], [9-1], [10-1], [11-1], and [12-1], wherein the cell population obtained in the first step is a cell aggregate.
[16] The production method of any of [1] to [15], [2-1], [5-1], [5-2], [6-1], [6-2], [7-1], [8-1], [9-1], [10-1], [11-1], and [12-1], wherein any one or more of the first step, the second step, step b, and the third step is/are conducted in a culture vessel in which at least one well is formed, the well is divided into a plurality of microwells, and suspension culturing is performed so that one cell mass is formed in each microwell.
[17] A method for producing a pituitary tissue, comprising collecting the pituitary tissue from a cell population containing a pituitary tissue, which is obtained by the production method of any of [1] to [16], [2-1], [5-1], [5-2], [6-1], [6-2], [7-1], [8-1], [9-1], [10-1], [11-1], and [12-1].

### [Advantageous Effects of Invention]

According to the present invention, the induction efficiency of differentiation into ectoderm becomes high due to the effect of a JNK signal transduction pathway inhibiting substance, and a cell population containing pituitary tissue with high hormone production capacity can be produced efficiently from pluripotent stem cells.

### [Brief Description of Drawings]

[Fig. 1]

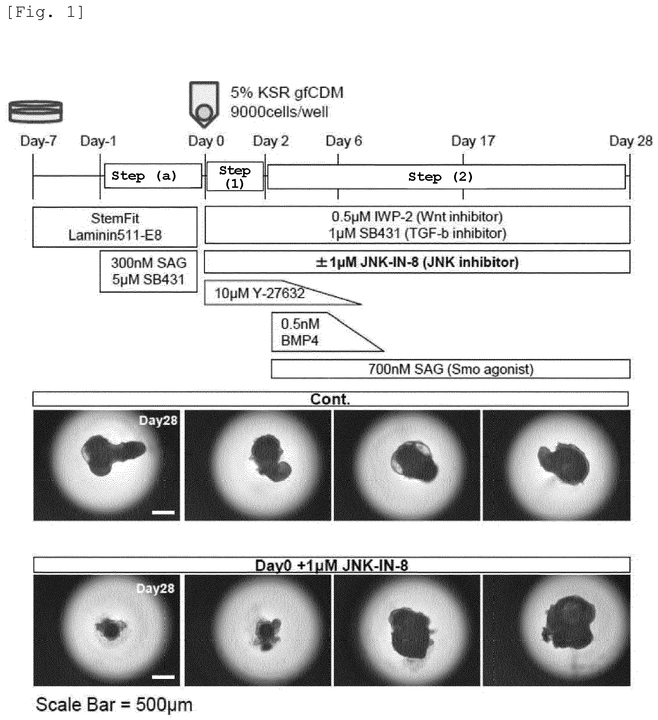

   Fig. 1 presents a diagram (top diagram) schematically showing the method for producing a cell population containing pituitary tissue of the present invention (embodiment performed in step (a), step (1), and step (2): Example 1) and a diagram (bottom diagram) showing the morphology of the obtained cell population (cell mass).
[Fig. 2A]

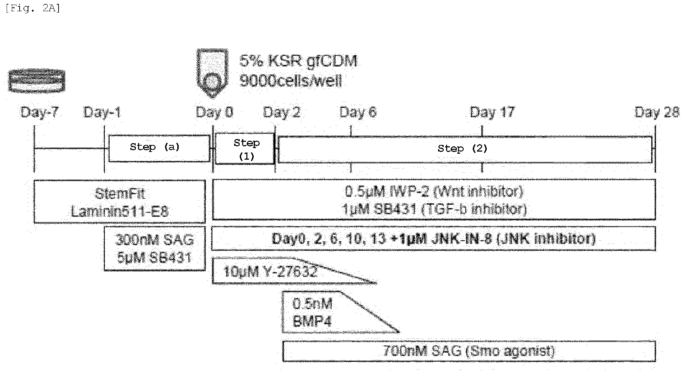

   Fig. 2A is a diagram schematically showing the method for producing a cell population containing pituitary tissue of the present invention (embodiment performed in step (a), step (1), and step (2): Example 2).
[Fig. 2B]

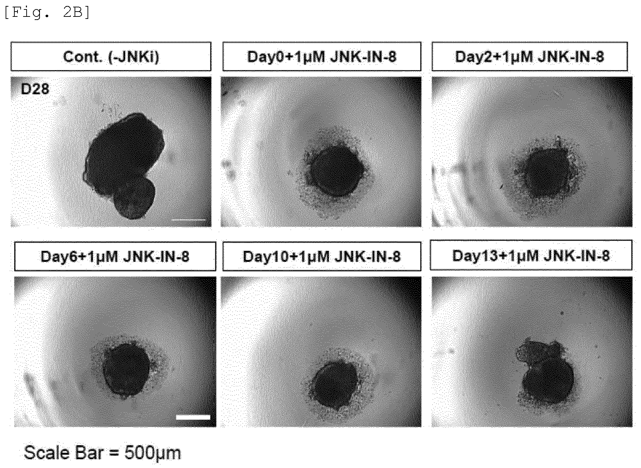

   Fig. 2B is a diagram showing the morphology of a cell population (cell mass) produced by the step shown in Fig. 2A.
[Fig. 3A]

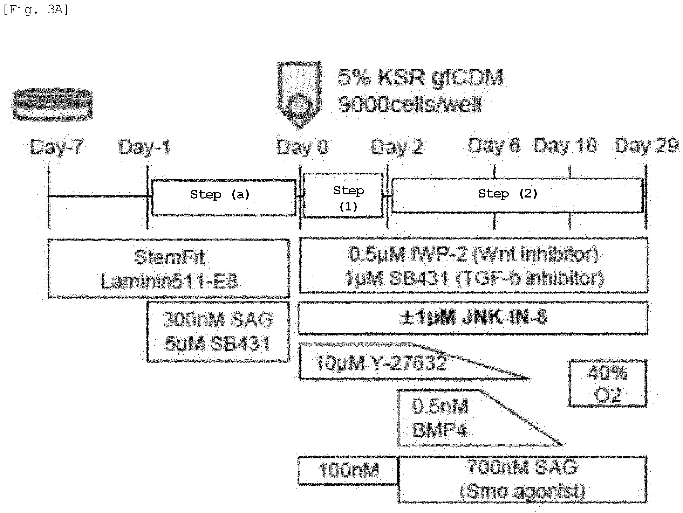

   Fig. 3A is a diagram schematically showing the method for producing a cell population containing pituitary tissue of the present invention (embodiment performed in step (a), step (1), and step (2): Example 3).
[Fig. 3B]

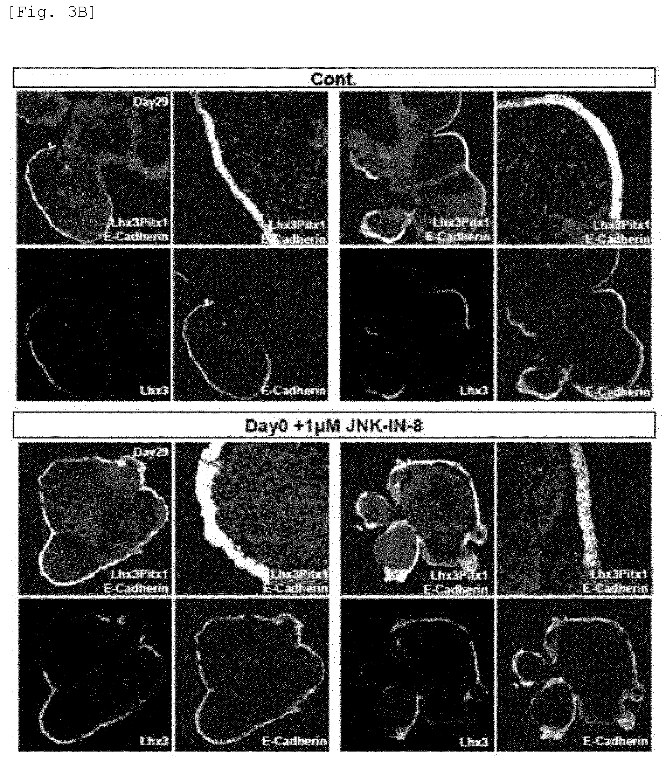

   Fig. 3B presents diagrams showing the expression status of Lhx3, Pitx1, and E-cadherin in the cell population (cell mass) produced by the step shown in Fig. 3A.
[Fig. 4A]

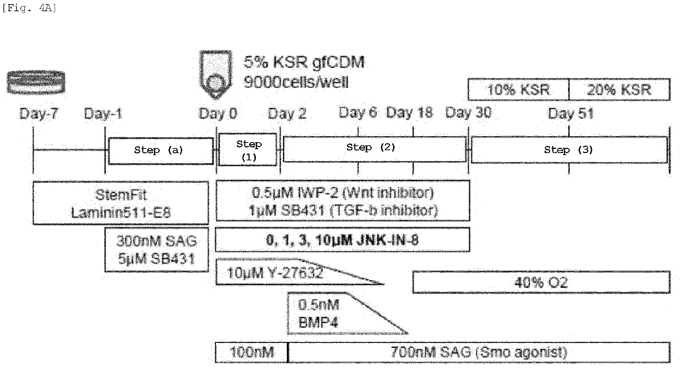

   Fig. 4A is a diagram schematically showing the method for producing a cell population containing pituitary tissue of the present invention (embodiment performed in step (a), step (1), step (2), and step (3): Example 4).
[Fig. 4B]

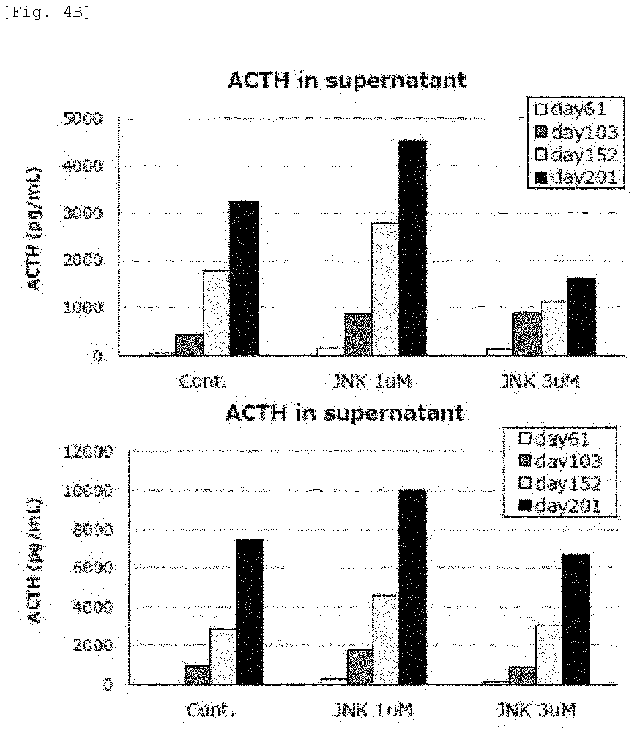

   Fig. 4B presents graphs showing the results of adrenocorticotropic hormone (ACTH) secretory capacity on days 61, 103, 152, and 201 after the differentiation induction of pituitary tissue induced to differentiate from a cell population (cell mass) produced by the step shown in Fig. 4A.
[Fig. 5]

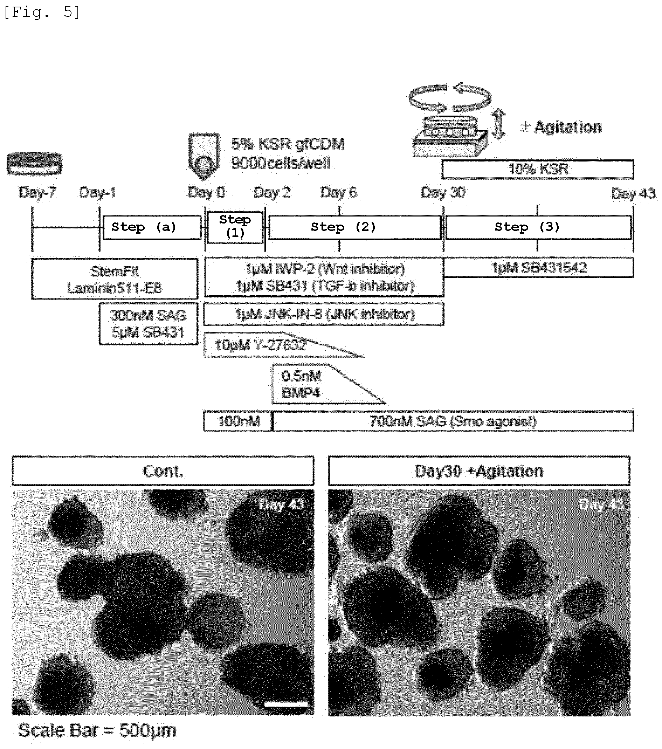

   Fig. 5 presents a diagram (top diagram) schematically showing the method for producing a cell population containing pituitary tissue of the present invention (embodiment performed in step (a), step (1), step (2), and step (3): Example 5) and a diagram (bottom diagram) showing the morphology of the obtained cell population (cell mass). Step (3) was performed by shaking culture.
[Fig. 6]

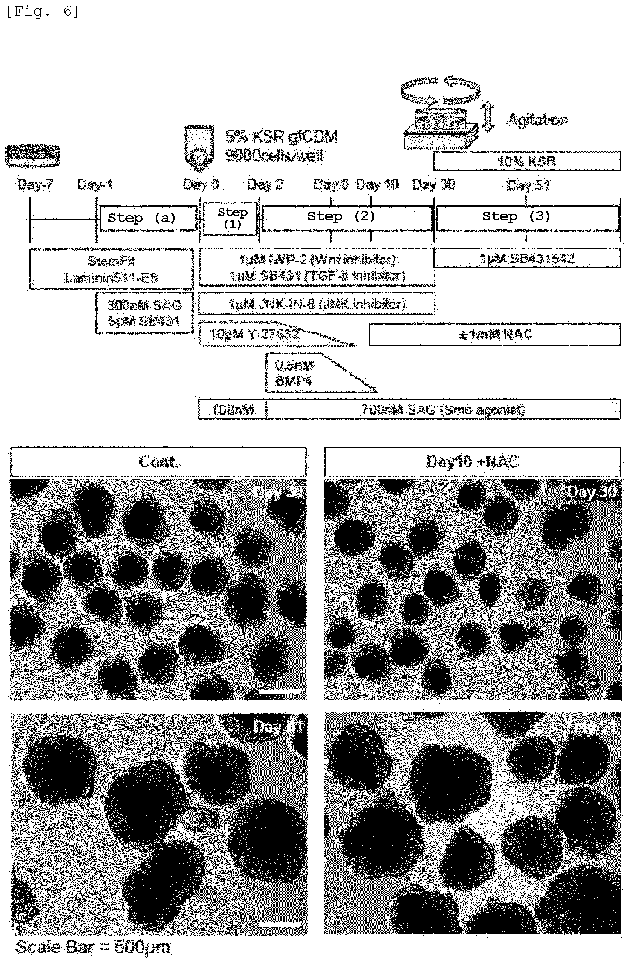

   Fig. 6 presents a diagram (top diagram) schematically showing the method for producing a cell population containing pituitary tissue of the present invention (embodiment performed in step (a), step (1), step (2), and step (3): Example 6) and a diagram (bottom diagram) showing the morphology of the obtained cell population (cell mass). Step (3) was performed by shaking culture. N-acetylcysteine was added or not added in step (3).
[Fig. 7A]

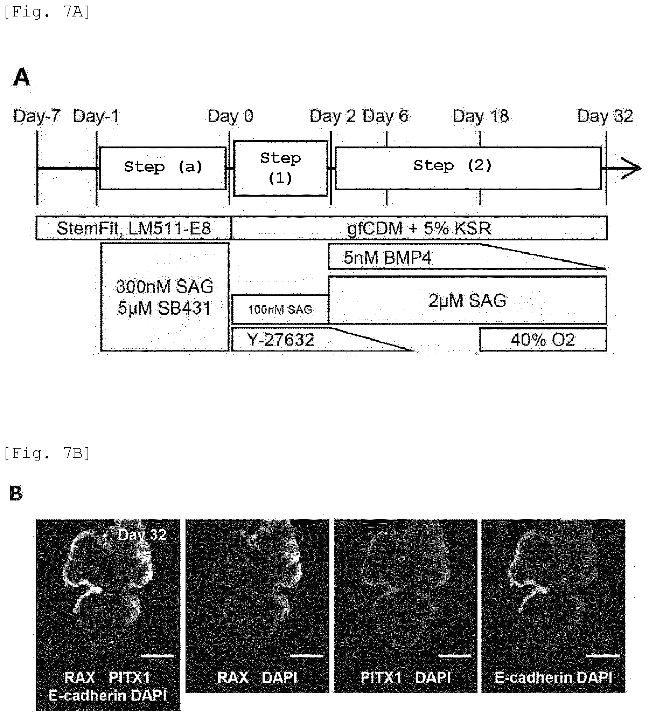

   Fig. 7A is a diagram schematically showing the step (condition without addition of JNK inhibitor in step (1)) of Reference Example 1 of the production method of a cell population containing pituitary tissue from human ES cells.
[Fig. 7B]
   Fig. 7B is a diagram showing the expression status of RAX, PITX1, and E-cadherin on day 32 in the cell aggregate produced by the step shown in Fig. 7A.
[Fig. 8A]

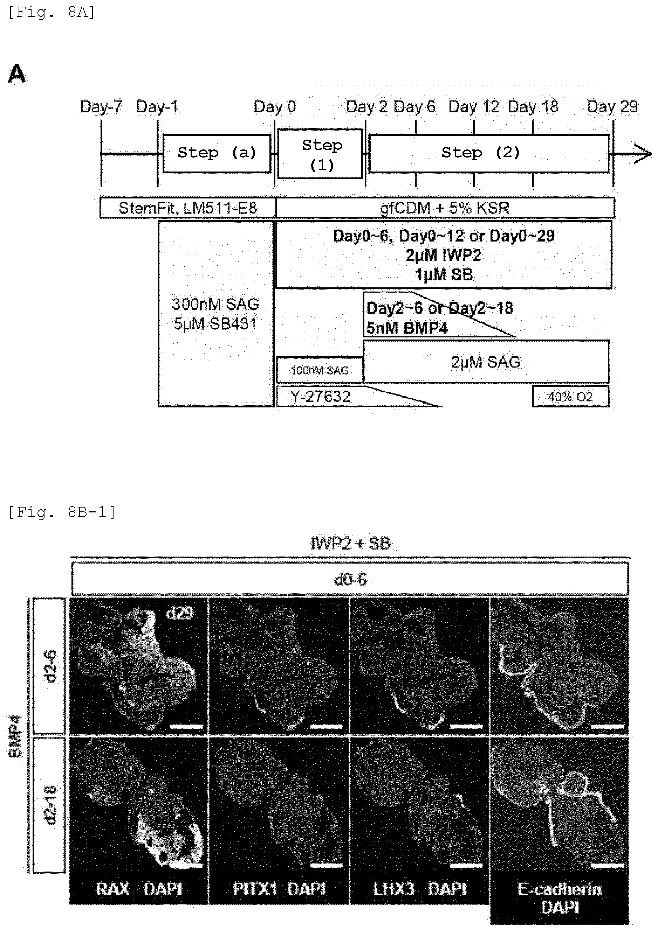

   Fig. 8A is a diagram schematically showing the step of Reference Example 2 (study of addition period of IWP2, SB431542, and BMP4 in step (1) and step (2)) of the production method of a cell population containing pituitary tissue from human iPS cells.
[Fig. 8B-1]

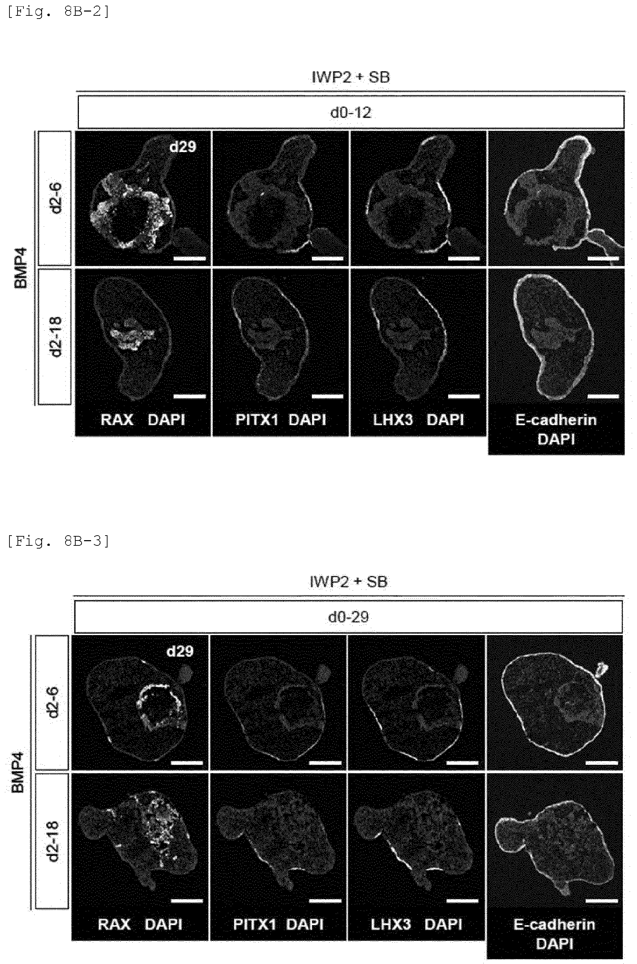

   Fig. 8B-1 is a diagram showing the expression status of RAX, PITX1, LHX3, and E-cadherin on day 29 in the cell aggregate produced by the step (addition period of IWP2, SB431542: d0-6) shown in Fig. 8A. The upper panel shows the case with BMP4 addition period of d2-6, and the lower panel shows the case with BMP4 addition period of d2-18.
[Fig. 8B-2]
   Fig. 8B-2 is a diagram showing the expression status of RAX, PITX1, LHX3, and E-cadherin on day 29 in the cell aggregate produced by the step (addition period of IWP2, SB431542: d0-12) shown in Fig. 8A. The upper panel shows the case with BMP4 addition period of d2-6, and the lower panel shows the case with BMP4 addition period of d2-18.
[Fig. 8B-3]
   Fig. 8B-3 is a diagram showing the expression status of RAX, PITX1, LHX3, and E-cadherin on day 29 in the cell aggregate produced by the step (addition period of IWP2, SB431542: d0-29) shown in Fig. 8A. The upper panel shows the case with BMP4 addition period of d2-6, and the lower panel shows the case with BMP4 addition period of d2-18.
[Fig. 9]
   Top diagram (A) is a diagram schematically showing the step (study of addition concentration of IWP2, BMP4, and SAG in step (1), step (2), and step (3)) of Reference Example 3 of the production method of a cell population containing pituitary tissue from human ES cells. Bottom diagram (B) is a graph showing the results of ACTH secretory capacity on days 61, 103, 152, 201, and 250 after the start of differentiation induction of a cell aggregate produced by the step shown in A. □ shows the results of low concentration group and ■ shows the results of high concentration group.
[Fig. 10]
   Top diagram (A) is a diagram schematically showing the method for producing a cell population containing pituitary tissue of the present invention (embodiment performed in step (a), step (1), step (2), and step (3): Example 7). Bottom diagram (B) is a diagram showing the expression status of ACTH and E-cadherin on day 103 after the start of differentiation induction of a cell aggregate produced in the step shown in A. The upper panel shows the results when JNK inhibitor was not added, and the lower panel shows the results when JNK inhibitor was added.
[Fig. 11]

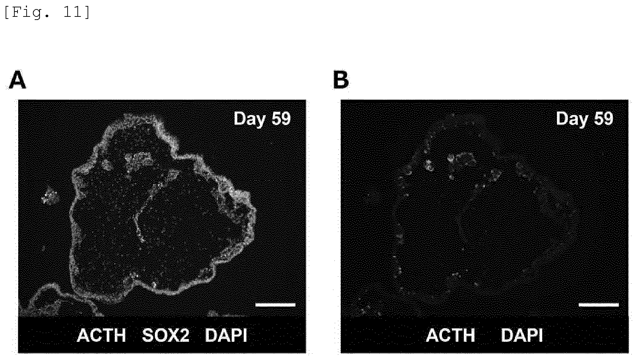

   Fig. 11 presents diagrams showing the expression status of ACTH and SOX2 on day 59 after the start of differentiation induction of a cell aggregate obtained by the production method (embodiment performed in step (a), step (1), step (2), and step (3): Example 8) of the cell population containing pituitary tissue of the present invention.
[Fig. 12]
   Top diagram (A) is a diagram schematically showing the method for producing a cell population containing pituitary tissue of the present invention (embodiment performed in step (a), step (1), step (2), and step (3): Example 9). Bottom diagram (B) shows comparison of ACTH secretory capacity between cell aggregates produced by treating with SAG until day 30 after the start of differentiation induction and by continuously treating with SAG also thereafter, as shown in A. □ shows the results of treatment with SAG until day 30, and ■ shows the results of continuous treatment with SAG even after day 30. *:p<0.05, Student's t-test.
[Fig. 13A]

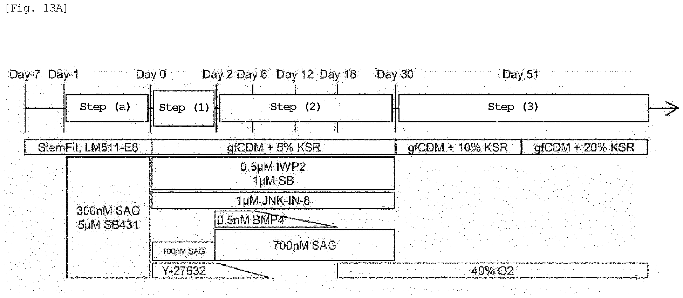

   Fig. 13A is a diagram schematically showing the method for producing a cell population containing pituitary tissue of the present invention (embodiment performed in step (a), step (1), step (2), and step (3): Example 10).
[Fig. 13B]

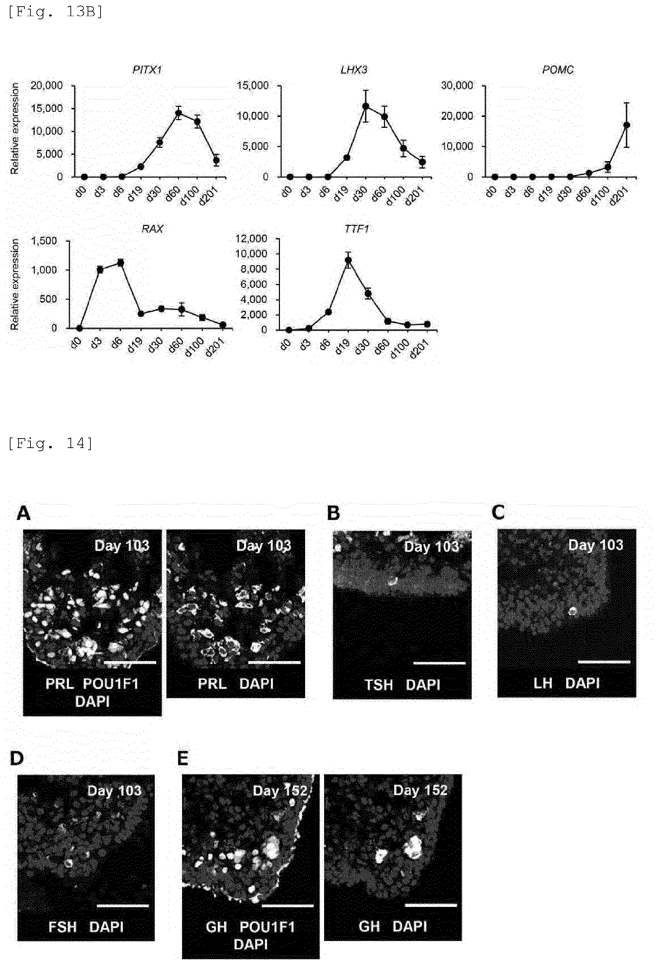

   Fig. 13B is a diagram showing the quantification results of the expression levels of various cell markers on days 3, 6, 19, 30, 60, 100, and 201 after the start of differentiation induction of cell aggregates produced by the step shown in Fig. 13A. The upper panel shows the results of PITX1, LHX3, and POMC. The lower panel shows the results of RAX and TTF1.
[Fig. 14]
   Fig. 14 presents diagrams showing the expression status of PRL, POU1F1, TSH, LH, FSH, and GH in cell aggregates produced by the step shown in Fig. 13A (embodiment performed in step (a), step (1), step (2), and step (3); Example 11). A: triple staining with PRL, POU1F1, and DAPI (left diagram) on day 103 after the start of suspension culturing and double staining with PRL and DAPI on day 103 after the start of suspension culturing (right diagram), B: double staining with TSH and DAPI on day 103 after the start of suspension culturing, C: double staining with LH and DAPI on day 103 after the start of suspension culturing, D: double staining with FSH and DAPI on day 103 after the start of suspension culturing, E: triple staining with GH, POU1F1, and DAPI on day 152 after the start of suspension culturing (left diagram), and double staining with GH and DAPI on day 152 after the start of suspension culturing (right diagram).
[Fig. 15]

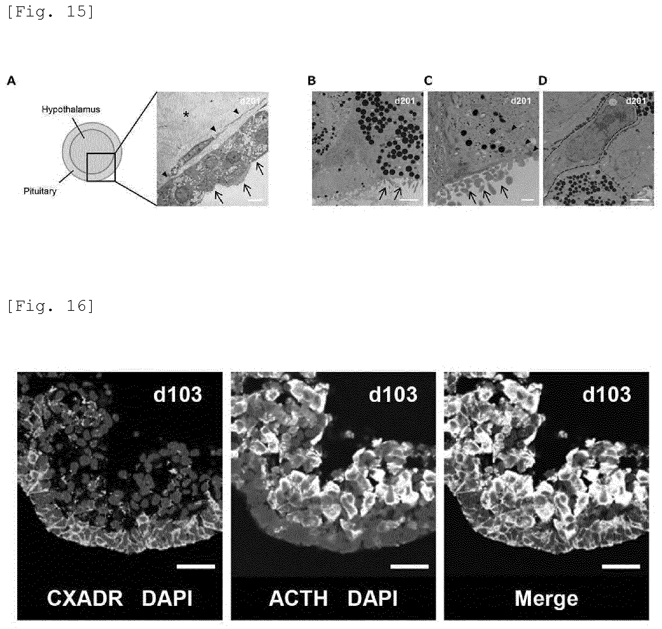

   Fig. 15 shows cell aggregates produced by the step shown in Fig. 13A (embodiment performed in step (a), step (1), step (2), and step (3); Example 12) on day 201 after the start of differentiation induction as observed under an electron microscope.
[Fig. 16]
   Fig. 16 presents diagrams confirming the expression of ACTH and CXADR on day 103 after the start of differentiation induction, in cell aggregates produced by the step shown in Fig. 13A (embodiment performed in step (a), step (1), step (2), and step (3); Example 13).
[Fig. 17]
   A is a diagram showing the ACTH secretory capacity on days 29, 61, 103, 152, 201, and 250 after the start of differentiation induction, of a cell aggregate produced by the step shown in Fig. 13A (embodiment performed in step (a), step (1), step (2), and step (3); Example 14). **:p<0.01, ***:p<0.001, one-way ANOVA with post hoc Tukey. B and C show the results of ACTH stimulation test by CRH (B) or dexamethasone (C) in cell aggregates produced by the step shown in Fig. 13A (embodiment performed in step (a), step (1), step (2), and step (3); Example 14). **:p<0.01, paired t-test.
[Fig. 18A]

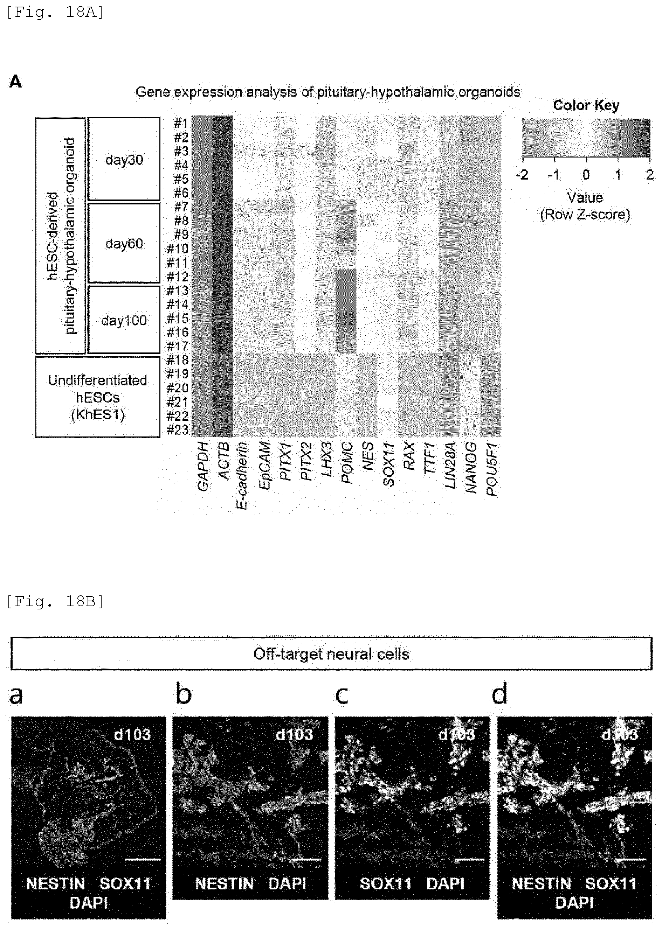

   Fig. 18A is a diagram confirming the expression of each cell marker on days 30, 60, and 100 after the start of differentiation induction, in a cell aggregate produced by the step shown in Fig. 13A (embodiment performed in step (a), step (1), step (2), and step (3); Example 15) and in undifferentiated cells.
[Fig. 18B]
   Fig. 18B presents diagrams showing the expression status of NESTIN and SOX11 on day 103 from the differentiation induction, in cell aggregates produced by the step shown in Fig. 13A (embodiment performed in step (a), step (1), step (2), and step (3); Example 15). a: triple staining with NESTIN, SOX11, and DAPI, b: double staining with NESTIN and DAPI, c: double staining with SOX11 and DAPI, d: triple staining with NESTIN, SOX11, and DAPI.
[Fig. 19A]

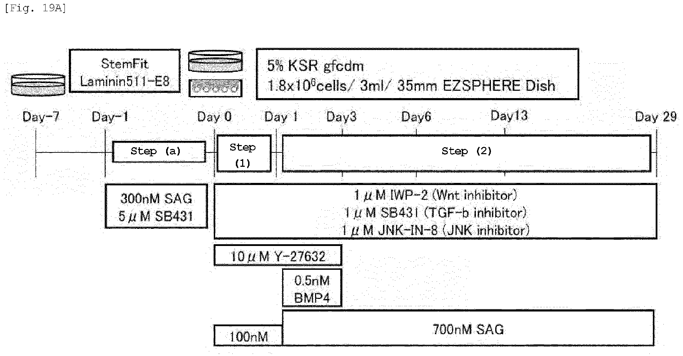

   Fig. 19A is a diagram schematically showing the production method (embodiment performed in step (a), step (1), and step (2): Example 16) of a pituitary organoid from human iPS cells by using a culture vessel with divided microwells.
[Fig. 19B]

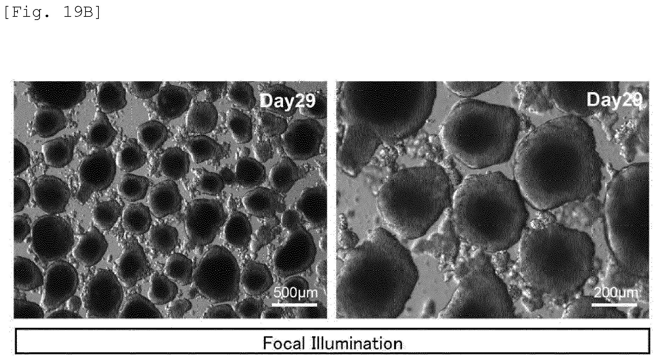

   Fig. 19B presents diagrams showing the shape of a cell aggregate produced by the step shown in Fig. 19A, on day 29 after the start of differentiation induction by oblique illumination observation. A pituitary organoid having a placode-like pituitary tissue was formed.
[Fig. 20A]

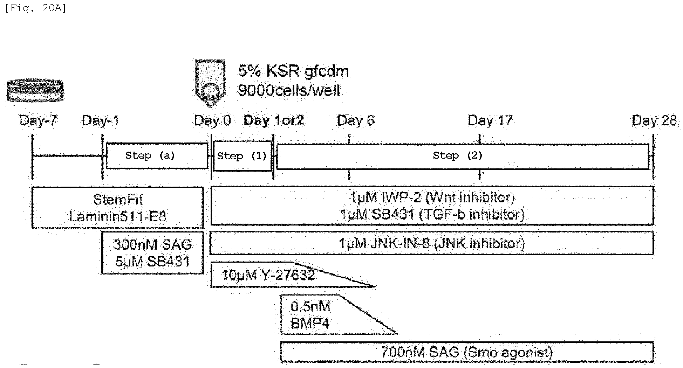

   Fig. 20A is a diagram schematically showing the method for producing a cell population containing pituitary tissue of the present invention (embodiment performed in step (a), step (1), and step (2): Example 17) from human iPS cells.
[Fig. 20B]

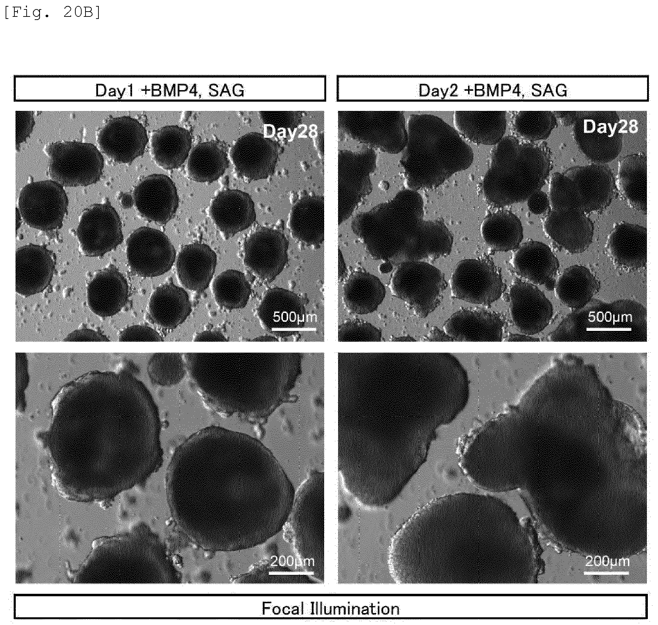

   Fig. 20B presents diagrams showing the shape by oblique illumination observation of cell aggregates on day 28 after the start of differentiation induction, which were produced by the step shown in Fig. 20A. Pituitary organoids having a placode-like pituitary tissue were formed under BMP4 addition conditions on both days 1 and 2 after the start of differentiation induction.

### 1. Definition

In the present specification, the definitions of the terms are as follows. The "stem cell" means an undifferentiated cell having differentiation potency and proliferative capacity (particularly self-renewal competence). The stem cell includes pluripotent stem cell, multipotent stem cell, unipotent stem cell and the like according to the differentiation potency. The "pluripotent stem cell" refers to a stem cell capable of being cultured in vitro and having a potency to differentiate into any cell constituting living organisms (pluripotency). Any cell refers to cells derived from three germ layers of ectoderm, mesoderm, and endoderm. The "multipotent stem cell" means a stem cell having a potency to differentiate into plural types of tissues or cells, though not all kinds. The "unipotent stem cell" means a stem cell having a potency to differentiate into a particular tissue or cell.

Pluripotent stem cell can be induced from fertilized egg, clone embryo, germ stem cell, stem cell in a tissue, somatic cell or the like. Examples of the pluripotent stem cell include embryonic stem cell (ES cell), EG cell (embryonic germ cell), and induced pluripotent stem cell (iPS cell). Muse cell (Multi-lineage differentiating stress enduring cell) obtained from mesenchymal stem cell (MSC), and GS cell produced from reproductive cell (e.g., testis) are also encompassed in the pluripotent stem cell. Human embryonic stem cells were established from human embryos within 14 days of fertilization.

Embryonic stem cell was first established in 1981, and has also been applied to the generation of knockout mouse since 1989. In 1998, human embryonic stem cell was established, which is also being utilized for regenerative medicine. ES cell can be produced by culturing an inner cell population on a feeder cell or in a medium containing leukemia inhibitory factor (LIF). The production methods of ES cell are described in, for example, WO 96/22362, WO 02/101057, US Patent No. 5843780, US Patent No. 6200806, US Patent No. 6280718, and the like. Embryonic stem cells are available from given organizations, or a commercially available product can be purchased. For example, human embryonic stem cells, KhES-1, KhES-2 and KhES-3, are available from Kyoto University's Institute for Frontier Medical Sciences. EB5 cell, which is a mouse embryonic stem cell, is available from Incorporated Administrative Agency RIKEN, and D3 cell line, which is a mouse embryonic stem cell, is available from American Type Culture Collection (ATCC). Nuclear transfer ES cell (ntES cell), which is one of the ES cells, can be established from a clone embryo produced by transplanting the cell nucleus of a somatic cell into a cell nucleus-removed egg.

EG cell can be produced by culturing a primordial germ cell in a medium containing mouse stem cell factor (mSCF), LIF, and basic fibroblast growth factor (bFGF) (Cell, 70: 841-847, 1992) .

The "induced pluripotent stem cell" is a cell induced to have pluripotency by reprogramming a somatic cell by a known method and the like. Specifically, as the induced pluripotent stem cell, a cell induced to have pluripotency by reprogramming differentiated somatic cells such as fibroblast, and peripheral blood mononuclear cell by the expression of a plurality of genes selected from the group consisting of reprogramming genes including Oct3/4, Sox2, Klf4, Myc (c-Myc, N-Myc, L-Myc), Glis1, Nanog, Sall4, lin28, and Esrrb can be mentioned. Induced pluripotent stem cell was established by Yamanaka et al. in mouse cell in 2006 (Cell, 2006, 126(4), pp.663-676). In 2007, Induced pluripotent stem cell was also established from human fibroblast, and has pluripotency and self-renewal competence similar to those of embryonic stem cells (Cell, 2007, 131(5), pp.861-872; Science, 2007, 318(5858), pp.1917-1920; Nat. Biotechnol., 2008, 26(1), pp.101-106). Besides the production method based on direct reprogramming by gene expression, induced pluripotent stem cell can also be obtained from somatic cell by the addition of a compound and the like (Science, 2013, 341, pp. 651-654).

While the somatic cell used for producing induced pluripotent stem cell is not particularly limited, tissue-derived fibroblast, blood-lineage cells (e.g., peripheral blood mononuclear cell, T cell), hepatocyte, pancreatic cell, intestinal epithelial cell, and smooth muscle cell can be mentioned.

When induced pluripotent stem cell is produced by reprogramming by the expression of several kinds of genes (e.g., 4 factors of Oct3/4, Sox2, Klf4, and Myc), the means for gene expression is not particularly limited. Examples of the aforementioned means for gene expression include an infection method using a virus vector (e.g., retrovirus vector, lentivirus vector, Sendaivirus vector, adenovirus vector, adeno-associated virus vector), a gene transfer method using a plasmid vector (e.g., plasmid vector, episomal vector) (e.g., calcium phosphate method, lipofection method, retronectin method, electroporation method), a gene transfer method using an RNA vector (e.g., calcium phosphate method, lipofection method, electroporation method), and a method with direct injection of protein.

It is also possible to obtain established induced pluripotent stem cells. For example, human induced pluripotent cell lines established in Kyoto University, such as 201B7 cell, 201B7-Ff cell, 253G1 cell, 253G4 cell, 1201C1 cell, 1205D1 cell, 1210B2 cell, 1231A3 cell, and the like, are available from Kyoto University and iPS Academia Japan, Inc. As induced pluripotent stem cells strain, for example, Ff-I01 cell, Ff-I14 cell, and QHJI01s04 cell, established in Kyoto University, are available from Kyoto University.

The pluripotent stem cells may be genetically modified. Genetically-modified pluripotent stem cells can be produced by using, for example, a homologous recombination technique. Examples of the gene on the chromosome to be modified include a cell marker gene, a histocompatibility antigen gene, a gene related to a disease due to a disorder of neural cell and so on. A target gene on the chromosome can be modified using the methods described in Manipulating the Mouse Embryo, A Laboratory Manual, Second Edition, Cold Spring Harbor Laboratory Press (1994); Gene Targeting, A Practical Approach, IRL Press at Oxford University Press (1993); Biomanual Series 8, Gene Targeting, Making of Mutant Mouse using ES cell, YODOSHA CO., LTD. (1995); and so on.

To be specific, for example, the genome gene of the target gene to be modified (e.g., cell marker gene, histocompatibility antigen gene, disease-related gene and so on) is isolated, and a targeting vector used for homologous recombination of the target gene is produced using the isolated genome gene. The produced targeting vector is introduced into stem cells and the cells that showed homologous recombination between the target gene and the targeting vector are selected, whereby stem cells having the modified gene on the chromosome can be produced.

Examples of the method for isolating genome gene of the target gene include known methods described in Molecular Cloning, A Laboratory Manual, Second Edition, Cold Spring Harbor Laboratory Press (1989), Current Protocols in Molecular Biology, John Wiley & Sons (1987-1997) and so on. Genomic DNA library screening system (manufactured by Genome Systems), Universal GenomeWalker Kits (manufactured by CLONTECH) and so on can be used.

Production of targeting vector used for homologous recombination of the target gene, and efficient selection of a homologous recombinant can be performed according to the methods described in Gene Targeting, A Practical Approach, IRL Press at Oxford University Press (1993); Biomanual Series 8, Gene Targeting, Making of Mutant Mouse using ES cell, YODOSHA CO., LTD. (1995); and so on. As the targeting vector, any of replacement type or insertion type can be used. As the selection method, methods such as positive selection, promoter selection, negative selection, polyA selection and so on can be used. Examples of a method for selecting the desired homologous recombinant from the selected cell lines include Southern hybridization method, PCR method and so on for the genomic DNA.

As the pluripotent stem cells, pluripotent stem cells that have undergone genome editing can also be used as pluripotent stem cells. The "genome editing" is a technique that intentionally modifies a target gene or genomic region by using principles such as site-specific cleavage of genomic DNA strands using nucleases or chemical conversion of bases. Site-specific nuclease includes zinc finger nuclease (ZFN), TALEN, CRISPR/Cas9, and the like. By using genome editing technique, knockout cell line in which a specific gene is deleted, knock-in cell line in which another sequence is artificially inserted into a specific gene locus, and the like can be produced.

Disease-specific pluripotent stem cells may be used as pluripotent stem cells. The "disease-specific pluripotent stem cell" refers to a pluripotent stem cell that has gene mutation or genetic background involved in the onset of disease. Disease-specific pluripotent stem cells can be produced by a method of establishing induced pluripotent stem cells from patients with the target disease or their close relatives by the aforementioned method and the like, or by a method of modifying the genome of already established pluripotent stem cells by using genome editing techniques such as zinc finger nuclease (ZFN), TALEN, and CRISPR.

The "mammal" encompasses rodents, ungulata, carnivora, lagomorpha, primates, etc. The rodents encompass mouse, rat, hamster, guinea pig, etc. Ungulata encompass swine, bovine, goat, horse, sheep, etc. Carnivora encompasses dog, cat, etc. Lagomorpha encompass rabbit and the like. The "primates" in the present invention refers to mammals belonging to the primate, and the primates include prosimian such as lemur, loris, and tupai, and anthropoidea such as monkey, ape, and human.

The pluripotent stem cells to be used in the present invention are mammalian pluripotent stem cells, preferably pluripotent stem cells of rodents (e.g., mouse, rat) or primates (e.g., human, monkey), most preferably a human pluripotent stem cell.

The "cell adhesion" includes adhesion between cells (cell-cell adhesion) and adhesion between cells and extracellular matrix (substrate) (cell-substrate adhesion). Adhesion of cells to culture vessels and the like that occurs under an artificial culture environment in vitro is also included in the cell adhesion. The bond formed in cell-cell adhesion is a cell-cell junction, and the bond formed in cell-substratum adhesion is a cell-substratum junction. As the kind of the cell adhesion, for example, anchoring junction, communicating junction, occluding junction can be mentioned.

Examples of cell-cell junction include "tight junction" and "adherence junction". The tight junction is a relatively strong cell-cell bond that can occur in epithelial cells. Whether a tight junction is present between tissues can be detected by, for example, methods such as immunohistochemistry and the like using an antibody (anti-claudin antibody, anti-ZO-1 antibody etc.) to a constituent component of the tight junction.

The "suspension culturing" refers to culturing while maintaining a state in which cells are suspended in a culture medium. That is, the suspension culturing is performed under conditions in which cells are not adhered to a culture vessel or feeder cells or the like on the culture vessel (hereinafter denoted as "culture vessel, etc.") and is distinguished from culturing performed under conditions in which cells are adhered to a culture vessel and the like (adhesion culture). More particularly, suspension culturing refers to culturing under conditions in which a strong cell-substrate junction is not formed between a cell and a culture vessel and the like. Those of ordinary skill in the art can easily determine whether the cells being cultured are in a suspension culture state or in an adhesion culturing by, for example, shaking the culture vessel during microscopic observation.

The "adhesion culture" refers to culturing while maintaining the state in which cells adhere to culture vessel and the like. As used herein, the adhesion of cells to culture vessel and the like means, for example, that a strong cell-substrate bond, which is one type of cell adhesion, is formed between cells and culture vessel and the like.

In a cell aggregate in suspension culturing, a plane attachment is formed between a cell and a cell. In a cell aggregate in suspension culturing, a strong cell-substrate junction is not formed between the cell and the culture vessel, etc. and a cell-substrate junction is hardly formed and, even if it is formed, its contribution is small. An inherent cell-substrate junction may be present inside the cell aggregates during suspension culturing. The "plane attachment between a cell and a cell" means that a cell attaches to another cell via planes. More particularly, the "plane attachment between a cell and a cell" means that, for example, not less than 1%, preferably not less than 3%, more preferably not less than 5%, of the surface area of a cell adheres to the surface of another cell. A surface of a cell can be observed by methods such as staining with a reagent (e.g., DiI) that stains membranes, immunostaining with cell adhesion factors (e.g., E-cadherin, N-cadherin, etc.), and the like.

The culture vessel to be used when performing adhesion culturing is not particularly limited as long as it enables adhesion culturing and those of ordinary skill in the art can appropriately determine same. Examples of such culture vessel include flask, tissue culture flask, dish, tissue culture dish, multidish, microplate, microwell plate, micropore, multiplate, multiwell plate, chamber slide, schale, tube, tray, culture bag, and biological function chips such as organ-on-chip and the like. As cell-adhesive culture vessel, a culture vessel with its surface artificially treated for the purpose of improving adhesion to cells and the like can be used. Examples of the artificial treatment include coating treatment with an extracellular matrix, polymer, and the like, and surface treatment such as gas plasma treatment, positive charge treatment, and the like. Examples of the extracellular matrix to which cells adhere include basement membrane preparation, laminin, entactin, collagen, gelatin, and the like. Examples of the polymer include polylysine, polyornithine, and the like. The culture surface of the culture vessel may be a flat bottom or may have concaves and convexes.

"Laminin" is a heterotrimer molecule consisting of α, β, γ chains and an extracellular matrix protein containing isoforms having different subunit chain compositions. Specifically, laminin has about 15 kinds of isoforms based on the combinations of heterotrimers with 5 kinds of α chains, 4 kinds of β chains and 3 kinds of γ chains. The name of laminin is determined by combining respective numbers of α chain (α1 - α5), β chain (β1 - β4) and γ chain (γ1 - γ4). For example, a laminin having a combination of α5 chain, β1 chain, γ1 chain is named laminin 511.

The culture vessel to be used when performing suspension culturing is not particularly limited as long as it enables "culturing in suspension" and those of ordinary skill in the art can appropriately determine same. Examples of such culture vessel include flask, tissue culture flask, dish, petri dish, tissue culture dish, multidish, microplate, microwell plate, micropore, multiplate, multiwell plate, chamber slide, schale, tube, tray, culture bag, spinner flask, and roller bottle. To enable suspension culturing, these culture vessels are preferably non-cell-adhesive. As the cell non-adhesive culture vessel, a culture vessel that has not been processed by artificial treatment as described above for the purpose of improving adhesion to cells, or the like can be used. As a non-cell-adhesive culture vessel, culture vessels whose surfaces have been artificially treated to decrease adhesiveness to the cells can also be used. The culture surface of the culture vessel may be a flat bottom, U-bottom, or V-bottom, or may have concaves and convexes. Examples of the treatment to decrease adhesiveness to the cells include superhydrophilic treatment by coating with 2-methacryloyloxyethyl phosphorylcholine (MPC) polymer, Poly(2-hydroxyethyl methacrylate) (Poly-HEMA), polyethylene glycol (PEG), and the like, and protein low adsorption treatment, and the like.

The "cultured with shaking" is a culture method in which the culture medium is stirred by shaking the culture vessel to promote oxygen supply to the culture medium, material exchange with the periphery of the cells, and the like. Stirring culture, channel culture, or the like can also be performed.

For the purpose of protecting cell aggregates from physical stress such as shearing force that occurs during suspension culture, increasing the local concentration of growth factors and cytokines secreted by cells, and promoting tissue development, suspension culturing can also be performed after embedding cell aggregates in a gel or encapsulating them in a substance-permeable capsule (Nature, 2013, 501.7467:373). The encapsulated cell aggregates mentioned above may be cultured with shaking. The gel or capsule used for embedding may be of biological origin or made of a synthetic polymer. Examples of the gels or capsules used for this purpose include Matrigel (manufactured by Corning), PuraMatrix (manufactured by 3D Matrix), VitroGel 3D (manufactured by TheWell Bioscience), collagen gel (manufactured by Nitta Gelatin Inc.), alginate gel (manufactured by PG research), Cell-in-a-Box (manufactured by Austrianova) and the like.

The medium to be used for culturing cells can be prepared from a medium generally used for culturing animal cells as a basal medium. Examples of the basal medium include Basal Medium Eagle (BME), BGJb medium, CMRL 1066 medium, Glasgow Minimum Essential Medium (Glasgow MEM), Improved MEM Zinc Option, Iscove's Modified Dulbecco's Medium (IMDM), Medium 199, Eagle Minimum Essential Medium (Eagle MEM), Alpha Modified Eagle Minimum Essential Medium (aMEM), Dulbecco's Modified Eagle Medium (DMEM), F-12 medium, DMEM/F12, IMDM/F12, Ham's medium, RPMI 1640, Fischer's medium, mixed media of these, and the like.

For culturing pluripotent stem cells, a medium for culturing pluripotent stem cells using the above-mentioned basal medium as the base, preferably a known medium for embryonic stem cells and/or induced pluripotent stem cells, a medium for culturing pluripotent stem cells under feeder free (feeder-free medium) and the like can be used. As feeder-free medium, many synthetic media have been developed and are commercially available and, for example, Essential 8 medium can be mentioned. Essential 8 medium is obtained by supplementing DMEM/F12 medium with L-ascorbic acid-2-phosphate magnesium (64 mg/l), sodium selenium (14 pg/1), insulin (19.4 mg/l), NaHCO₃ (543 mg/l), transferrin (10.7 mg/l), bFGF (100 ng/mL), and TGFβ family signal transduction pathway activating substance (TGFβ1 (2 ng/mL) or Nodal (100 ng/mL)) as additives (Nature Methods, 8, 424-429 (2011)). Examples of the commercially available feeder-free medium include Essential 8 (manufactured by Thermo Fisher Scientific), S-medium (manufactured by DS Pharma Biomedical Co., Ltd.), StemPro (manufactured by Thermo Fisher Scientific), hESF9, mTeSR1 (manufactured by STEMCELL Technologies), mTeSR2 (manufactured by STEMCELL Technologies), TeSR-E8 (manufactured by STEMCELL Technologies), mTeSR Plus (manufactured by STEMCELL Technologies), StemFit (manufactured by Ajinomoto Co., Inc.), ReproMed iPSC Medium (manufactured by REPROCELL), NutriStem XF (manufactured by Biological Industries), NutriStem V9 (manufactured by Biological Industries), Cellartis DEF-CS Xeno-Free Culture medium (manufactured by Takara Bio Inc.), Stem-Partner SF (manufactured by KYOKUTO PHARMACEUTICAL INDUSTRIAL CO., LTD), PluriSTEM Human ES/iPS Cell Medium (manufactured by Merck), StemSure hPSC MediumΔ (manufactured by FUJIFILM Wako Pure Chemical Corporation), and the like.

The serum-free medium may contain a serum replacement. Examples of the serum replacement include one appropriately containing albumin, transferrin, fatty acid, collagen precursor, trace element, 2-mercaptoethanol or 3' thiolglycerol, or equivalents of these, and so on. Such serum replacement may be prepared by, for example, the method described in WO 98/30679. The serum replacement may be a commercially available product. Examples of such commercially available serum replacement include Knockout Serum Replacement (manufactured by Thermo Fisher Scientific) (hereinafter sometimes also to be denoted as "KSR"), Chemically-defined Lipid concentrated (manufactured by Thermo Fisher Scientific), Glutamax (manufactured by Thermo Fisher Scientific), B27 Supplement (manufactured by Thermo Fisher Scientific), N2 Supplement (manufactured by Thermo Fisher Scientific) and the like.

The serum-free medium to be used for suspension culturing and adhesion culturing may appropriately contain a fatty acid or lipid, amino acid (e.g., non-essential amino acids), vitamin, growth factor, cytokine, antioxidant, 2-mercaptoethanol, pyruvic acid, buffering agent, inorganic salts and so on.

To avoid complicated preparation, a serum-free medium supplemented with an appropriate amount (e.g., about 0.5% to about 30%, preferably about 1% to about 20%) of commercially available KSR (manufactured by Thermo Fisher Scientific) (e.g., medium of 1:1 mixture of F-12 medium and IMDM medium supplemented with 1×chemically-defined Lipid concentrated, 5% KSR, and 450 µM 1-monothioglycerol) may be used as such serum-free medium. In addition, as a product equivalent to KSR, the medium disclosed in JP-A-2001-508302 can be mentioned.

The "serum-containing medium" means a medium containing unadjusted or unpurified serum. The medium may contain a fatty acid, lipid, amino acid (e.g., non-essential amino acids), vitamin, growth factor, cytokine, antioxidant, 2-mercaptoethanol, 1-monothioglycerol, pyruvic acid, buffering agent, inorganic salts and so on.

The culturing in the present invention is preferably performed under xeno-free conditions. The "xeno-free" means conditions eliminating components derived from species different from that of the cell to be cultured.

The medium to be used in the present invention is preferably a medium containing chemically determined components (Chemically defined medium; CDM) to avoid contamination with chemically undetermined components.

The "basement membrane structure" means a thin membrane structure composed of extracellular matrix. The basement membrane is formed on the basal side of epithelial cells in a living body. The components of the basement membrane include type IV collagen, laminin, heparan sulfate proteoglycan (perlecan), entactin/nidogen, cytokine, growth factor and the like. Whether a basement membrane is present in a tissue derived from a living body and in a cell population prepared by the production method of the present invention is determined by, for example, tissue staining such as PAM staining, and a method such as immunohistochemistry using an antibody against a constituent component of the basement membrane (anti-laminin antibody, anti-type IV collagen antibody, etc.).

The "basement membrane preparation" is one containing a basement membrane constituent component having functions to control epithelial cell-like cell morphology, differentiation, proliferation, motility, functional expression, and the like when desired cells having basement membrane formability are seeded thereon and cultured. When adhesion culturing of cells is performed in the present invention, the cells can be cultured in the presence of a basement membrane preparation. As used herein, the "basement membrane constituent component" refers to an extracellular matrix molecule as a thin membrane present between an epithelial cell layer and an interstitial cell layer and the like in animal tissues. A basement membrane preparation can be produced, for example, by removing cells adhered to the support via a basement membrane and having the ability to form the basement membrane from the support by using a solution having the ability to dissolve the lipids of the cells, an alkaline solution or the like. Examples of the basement membrane preparation include products commercially available as basement membrane products such as Matrigel (manufactured by Corning), Geltrex (manufactured by Thermo Fisher Scientific), and extracellular matrix molecules known as basement membrane components (e.g., laminin, type-IV collagen, heparan sulfate proteoglycan, and entactin etc.).

For culturing cells and tissues, a basement membrane preparation such as Matrigel (manufactured by Corning) which is extracted from a tissue or cell of Engelbreth-Holm-Swarm (EHS) mouse sarcoma and the like and solubilized can be used. Similarly, as a basement membrane component used for cell culturing, human solubilized amniotic membrane (manufactured by Bioresource Application Institute, Co.), human recombinant laminin produced by HEK293 cell (manufactured by BioLamina), human recombinant laminin fragment (manufactured by Nippi, Inc.), and human recombinant vitronectin (manufactured by Thermo Fisher Scientific) and the like can also be used. To avoid contamination with components derived from different organism species and to avoid the risk of infections, the basement membrane preparation is preferably a recombinant protein whose components are clear.

In the present specification, the "medium containing a substance X" and "in the presence of a substance X" respectively refer to a medium supplemented with an exogenous substance X or a medium containing an exogenous substance X, and in the presence of an exogenous substance X. The exogenous substance X is distinguished from the endogenous substance X which is the substance X endogenously expressed, secreted or produced by, for example, the cells or tissues present in the medium. Substance X in the medium may undergo a slight change in concentration due to decomposition of substance X or evaporation of the medium.

In the present specification, the start of culture in a medium where the concentration of substance X is Y preferably refers to the time point when the concentration of substance X in the medium becomes uniform at Y. When the culture container is sufficiently small (e.g., 96-well plate or culture with 200 µL or less of culture medium), the start of culture at concentration Y is the time point when the culture medium addition operation, half-volume medium exchange operation, or full-volume medium exchange operation described below is performed so that the concentration becomes Y. Also, that the concentration of substance X in the medium is Y includes when the average concentration of substance X over a certain culture period is Y, when the period containing substance X at a concentration of Y is 50% or more of the culture period, when the period containing substance X at a concentration of Y is longer than the shortest period among the culture periods assumed in each step, and the like.

In the present specification, "in the absence of a substance X" refers to a medium not supplemented with an exogenous substance X or a medium not containing an exogenous substance X, or a state in which an exogenous substance X is not present.

In the present specification, the "human protein X" means that protein X has the amino acid sequence of protein X naturally expressed in human in vivo.

Being "isolated" means that an operation to remove factors other than the intended component or cell has been performed, and the component or cell is no longer in a naturally occurring state. Therefore, "isolated protein X" does not include an endogenous protein X produced from the cells or tissues to be cultured, and contained in a cell or tissue or in the medium. The purity of the "isolated protein X" (percentage of the weight of protein X to the total protein weight) is generally not less than 70%, preferably not less than 80%, more preferably not less than 90%, further preferably not less than 99%, most preferably 100%.

In the present specification, the "derivative" refers to a group of compounds produced by substituting a part of the molecule of a specific compound with another functional group or other atom. In the present specification, a "variant" of a protein refers to a protein in which mutations such as deletion, addition, or substitution of amino acid residues have been made to the extent that the properties of the original protein can be maintained. The number of mutated amino acids is not particularly limited, and may be, for example, 1 to 4, 1 to 3, 1 or 2, or 1. A "variant" of a protein may be a protein having an amino acid sequence showing an identity of at least not less than 90%, not less than 91%, not less than 92%, not less than 93%, not less than 94%, not less than 95%, not less than 96%, not less than 97%, not less than 98%, not less than 99%, or not less than 99.5%, with that of the original protein. The amino acid mutated in the variant may be a non-natural type.

In the present specification, "after A hour (day A)" includes the A hour (day A) and refers to time and day after the A hour (day A). "Within B hour (day B)" includes the B hour (day B) and refers to time and day before B hour (day B).

The "feeder cell" refers to a cell other than a stem cell that co-exists when culturing the stem cell. Examples of the feeder cells used for culturing pluripotent stem cells while maintaining an undifferentiated state include mouse fibroblasts (MEF), human fibroblasts, and SNL cells. As the feeder cells, feeder cells that underwent a growth suppression treatment is preferable. Examples of the growth suppression treatment include treatment with a growth inhibitor (e.g., mitomycin C), and UV irradiation. Feeder cells used for culturing pluripotent stem cells while maintaining an undifferentiated state contributes to the maintenance of undifferentiated state of pluripotent stem cell by secretion of humoral factors (preferably factor for maintaining an undifferentiated state), or production of scaffolds for cell adhesion (extracellular matrix).

In the present specification, the absence of feeder cells (feeder-free) means culturing in the absence of a feeder cell. The absence of feeder cells means, for example, a condition without addition of a feeder cell or a condition substantially free of feeder cells (e.g., the ratio of the number of feeder cells relative to the total number of cells is not more than 3%).

The "cell aggregate" refers to a clump formed by assembly of cells, wherein the cells are adhered to each other. Cell populations, embryoid bodies, spheres, spheroids, and organoids are also encompassed in the cell aggregates. In cell aggregates, a plane attachment is preferably formed between a cell and a cell. In some embodiments, cells adhere to each other to form, for example, adhesionic junction (adherence junction) in some or all of the cell aggregates. In some embodiments, two or more cell aggregates can also be further artificially adhered or aggregated. Cell aggregates also include clusters in which cell populations are further adhered or aggregated to each other, and assembleoids. The shape of cell aggregates is not limited to spherical shapes, but can also be bispherical, bead-shaped, aggregates of spheres, string-like or branched (shapes described in Scientific reports, 11:21421 (2021), Japanese Patent Application No. 2021-078154), and the like.

The "uniformed cell aggregates" means that the size of each cell aggregate is constant when a plurality of cell aggregates are cultured, and that the variance in the length of the maximum diameter is small when the size of the cell aggregates are evaluated by the length of the maximum diameter. More specifically, it means that not less than 75% of a plurality of cell aggregates are within mean ± 100%, preferably mean ± 50%, more preferably mean ± 20%, of the mean maximum diameter in the plurality of cell aggregates.

A "cell population" refers to a cell group consisting of two or more cells. A cell population may be composed of one type of cell, or may be composed of multiple types of cells. The cells constituting the cell population may be suspended in a medium or may be attached to a culture vessel or the like. The cells constituting a cell population may be single cells, or the cells may adhere to each other to form a cell population in at least a portion of the cell population. As used herein, for example, the "single cell" refers to a cell almost free of mutual adhesion of cells (e.g., plane attachment). In some embodiments, being dispersed into single cells refers to a state almost free of cell-cell junction (e.g., adhesive junction). A cell population may contain cell aggregates.

The "tissue" refers to the structure of a cell population that has a structure in which multiple types of cells with different morphologies and properties are three-dimensionally configured in a certain pattern.

"Ectoderm" refers to the outermost germ layer among the three germ layers formed after fertilization of an egg during the early development process of an organism. As development progresses, the ectoderm divides into the neuroectoderm and superficial ectoderm, and the neuroectoderm further divides into the neural tube and neural crest. Various organs of the body are formed from these ectoderms, and organs formed from ectoderms are said to be derived from ectoderm. For example, organs or tissues of the central nervous system, such as the brain and spinal cord, are formed from the neural tube. For example, a part of central nervous system cells, facial bones and cartilage, sensory nerve cells, autonomic nerve cells, pigment cells, mesenchymal cells, and the like are formed from the neural crest. The epidermis, inner ear, anterior pituitary gland, and upper respiratory tissues including the olfactory epithelium are formed from the superficial ectoderm. Placodes and placode-derived tissues are derived from superficial ectoderm. Pluripotent stem cells express genes known as ectoderm markers, such as Pax3, Otx2, and Sox1, in the process of differentiating into ectoderm.

"Endoderm" refers to the innermost germ layer among the three germ layers formed after fertilization of an egg during the early development process of an organism. The endoderm forms, for example, the digestive organs, urinary tract, pharynx, trachea, bronchi, and lungs. In the process of differentiating into endoderm, pluripotent stem cells express genes known as endoderm markers, such as SOX17, HNF-3β/FoxA2, Klf5, GATA4, GATA6, and PDX-1.

"Mesoderm" refers to the germ layer formed between the ectoderm and endoderm. Mesoderm forms organs or tissues such as the circulatory system, skeleton, and muscle. In the process of differentiating into mesoderm, pluripotent stem cells express genes known as mesoderm markers, such as T/Brachury, SMA, ABCA4, Nkx2.5, and PDGFRα.

The "neural tissue" refers to a tissue constituted of neural cells including cerebrum, midbrain, cerebellum, spinal cord, retina, sensory nerve, peripheral nerve, and the like in the developing stage or adult stage. In the present specification, the "neuroepithelial tissue" refers to neural tissue that forms an epithelial structure with a layered structure, and the amount of neuroepithelial tissue in a neural tissue can be evaluated by bright field observation using an optical microscope and the like.

The "central nervous system" refers to the area where nervous tissue accumulates and plays a central role in information processing. In vertebrates, the brain and spinal cord are included in the central nervous system. The central nervous system is derived from the ectoderm.

The "neural cell" refers to a cell other than epidermal lineage cell in a tissue derived from ectoderm. That is, the neural cell includes cells such as neural precursor cell, neuron (neural cell), glial cell, neural stem cell, neuron precursor cell, glial precursor cell and the like. The neural cell also encompasses cell constituting the below-mentioned retinal tissue (retinal cell), retinal progenitor cell, retinal layer-specific neuron, neural retinal cell, and retinal pigment epithelial cell. The neural cell can be identified by using Nestin, βIII tubulin (TuJ1), PSA-NCAM, N-cadherin and the like as a marker.

Neuron is a functional cell that forms a neural circuit and contributes to signal transmission, and can be identified by using the expression of immature neuronal markers such as TuJ1, Dcx, and HuC/D and/or mature neural cell markers such as Map2, and NeuN as an index.

As glial cell, astrocyte, oligodendrocyte, and Müller glia can be mentioned. As an astrocyte marker, GFAP can be mentioned; as an oligodendrocyte marker, O4 can be mentioned; and as a Müller glia marker, CRALBP and the like can be mentioned.

The neural stem cell is a cell having differentiation potency (multipotency) into neuron and glial cell, and proliferative capacity (sometimes referred to as self-renewal competence) maintaining multipotency. As the neural stem cell marker, Nestin, Sox2, Musashi, Hes family, CD133 etc. can be mentioned; however, these markers are markers for progenitor/precursor cells in general and are not considered neural stem cell-specific markers. The number of neural stem cells can be evaluated by neurosphere assay, and clonal assay.

The neuronal precursor cell is a cell having proliferative capacity, which produces neuron and does not produce glial cell. As a neuronal precursor cell marker, Tbr2, Tα1 etc. can be mentioned. An immature neuronal marker (TuJ1, Dcx, HuC/D)-positive and growth marker (Ki67, pH3, MCM)-positive cell can also be identified as a neuronal precursor cell. The glial precursor cell is a cell having proliferative capacity, which produces glial cell and does not produce neuron.

The neural precursor cell is an assembly of precursor cells including neural stem cell, neuronal precursor cell and glial precursor cell, and has proliferative capacity and neuron- and glial-productivity. The neural precursor cell can be identified using Nestin, GLAST, Sox2, Sox1, Musashi, Pax6 and the like as markers. A neural cell marker-positive and growth marker (Ki67, pH3, MCM)-positive cell can also be identified as a neural precursor cell.

The "cerebral tissue" refers to a tissue in which one or more types of cells constituting the fetal or adult cerebrum (e.g., cortical neural precursor cell, dorsal cortical neural precursor cell, ventral cortical neural precursor cell, cerebral layer structure-specific neuron, layer 1 neuron, layer 2 neuron, layer 3 neuron, layer 4 neuron, layer 5 neuron, layer 6 neuron, glial cells (astrocyte and oligodendrocyte), progenitor cells of these, etc.) are arranged three-dimensionally in a layered manner. The fetal cerebrum is also called forebrain or telencephalon. The presence of each cell can be confirmed by known methods, for example, the presence or absence of cell marker expression and the level thereof, and the like.

As cerebral cell markers, FoxG1 (also known as Bf1) expressed in cerebral cells, Sox2 and Nestin expressed in cortical neural precursor cells, Pax6 and Emx2 expressed in dorsal cortical neural precursor cells, Dlx1, Dlx2 and Nkx2.1 expressed in ventral cortical neural precursor cells, Tbr2, Nex, Svet1 expressed in neuron precursor cells, Tbr1 expressed in layer 6 neuron, Ctip2 expressed in layer 5 neuron, RORβ expressed in layer 4 neuron, Cux1 or Brn2 expressed in layer 3 neuron or layer 2 neuron, Reelin expressed in layer 1 neuron, and the like can be mentioned.

In the present invention, the "ventricle" refers to a cavity formed by the central nervous tissue. In the living body, it is an acellular structure that is normally filled with a tissue fluid such as cerebrospinal fluid, and the apical surface of the neural tissue faces the ventricle. The ventricle periphery layer surrounding the ventricles is a region where neural stem cells are present and cell proliferation and neuron production occur in the developing stage. Whether the cell population and tissue produced by the production method of the present invention include ventricle can be detected by, for example, a method such as immunohistochemistry using a central nervous tissue marker (Bf1, Pax6, etc.) and an apical surface marker (PKC-zeta, etc.).

In the present invention, the "diencephalon" refers to the neural tissue of the central nervous system that is in contact with the third ventricle. The diencephalon includes tissues such as epithalamus, thalamus, hypothalamus, and pituitary gland.

In the present invention, the "hypothalamus" refers to one region of the diencephalon that is in contact with the pituitary gland. The hypothalamus is further segmented into the dorsal hypothalamus and ventral hypothalamus. The hypothalamus can be identified using markers such as Rx, Vax1, and Six3. The dorsal hypothalamus can be identified using markers such as Otp, Brn2 and vasopressin. The ventral hypothalamus can be identified using markers such as Nkx2.1 and SF1.

In the present invention, the "nonneural epithelial tissue" refers to a tissue other than the neuroepithelial tissues among the tissues having an epithelial structure. An epithelial tissue can also be formed from any germ layer of ectoderm, mesoderm, endoderm, or nutrition ectoderm. The epithelial tissue includes epithelium, mesothelium, and endothelium. Examples of the tissue included in the nonneural epithelial tissue include epidermis, corneal epithelium, nasal cavity epithelium, mouth cavity epithelium, trachea epithelium, bronchus epithelium, airway epithelium, kidney epithelium, renal cortex epithelium, placenta epithelium and the like.

Epithelial tissues are generally connected by various intercellular junctions, and form tissues having a monolayer or multilayer structure. Confirmation of the presence or absence of such epithelial tissues and quantification of the amount thereof can be performed by observation with an optical microscope or a method such as immunohistochemistry using antibodies against epithelial cell markers (anti-E-Cadherin antibody, anti-N-Cadherin antibody, anti-EpCAM antibody etc.).

In the present invention, the "epithelial polarity" shows spatially formed bias of the distribution of components and cellular functions in epithelial cells. For example, corneal epithelial cells are localized in the outermost layer of the eyeball, express apical-specific proteins such as membrane-bound mucins (MUC-1, 4, 16) on the apical side to retain tears, and express basal-specific proteins such as α6 integrin and β1 integrin on the basal side to adhere to the basement membrane.

Whether epithelial polarity is present in a tissue derived from a living body and in a cell population prepared by the production method of the present invention can be detected by, for example, a method such as immunohistochemistry using Phalloidin, an apical marker (anti-MUC-1 antibody, anti-PKC-zeta antibody etc.) and a basal marker (anti-α6 integrin antibody, anti-β1 integrin antibody etc.).

In the present invention, the "placode" refers to primordia of organs formed by thickening of a part of epidermal ectoderm mainly in the developmental process of vertebrate. As the tissue derived from placode, crystallin lens, nose, inner ear, trigeminal nerve, adenopituitary and the like can be mentioned. As a marker of placode or a precursor tissue thereof (preplacodal region), Six1, Six4, Dlx5, Eya2, Emx2, Bf1, and the like can be mentioned.

In the present invention, the "pituitary placode" is a thickened structure that is formed in the region of epidermis ectoderm in the process of embryonic development, and expresses a pituitary progenitor cell marker. As the pituitary progenitor cell marker, Lim3 (Lhx3), Pitx1/2, Islet1/2 and the like can be mentioned. The pituitary placode expresses at least one pituitary progenitor cell marker selected from the group consisting of Lim3, Pitx1/2, and Islet1/2, preferably all pituitary progenitor cell markers. The pituitary placode is invaginated and forms Rathke's pouch, which is a sac-like structure in the middle of development, and forms adenohypophysis when the development further progresses.

In the present invention, the "adenohypophysis" refers to a tissue containing at least one kind of anterior lobe or middle lobe pituitary cells. The pituitary cell includes pituitary hormone-producing cells that produce hormones that regulate physiological functions, and non-hormone-producing cells. Examples of the pituitary hormone-producing cell include cells constituting the anterior lobe such as growth hormone (GH)-producing cells, prolactin (PRL)-producing cells, adrenocorticotropic hormone (ACTH)-producing cells, thyroid-stimulating hormone (TSH)-producing cells, follicle-stimulating hormone (FSH)-producing cells, luteinizing hormone (LH)-producing cells; and cells constituting the middle lobe such as melanocyte-stimulating hormone (MSH)-producing cells. Examples of the non-hormone producing cell include vascular endothelial cells, pericytes, folliculostellate cells, pituitary stem cells and pituitary progenitor cells.

In one embodiment, the adenohypophysis contains at least one kind, preferably two kinds, more preferably three kinds, selected from the group consisting of growth hormone (GH)-producing cells, prolactin (PRL)-producing cells, and adrenocorticotropic hormone (ACTH)-producing cells. In another embodiment, adenohypophysis contains at least one kind, preferably two or more kinds (2, 3, 4, 5 or 6 kinds), of pituitary hormone-producing cells selected from the group consisting of growth hormone (GH)-producing cells, prolactin (PRL)-producing cells, adrenocorticotropic hormone (ACTH)-producing cells, thyroid-stimulating hormone (TSH)-producing cells, follicle-stimulating hormone (FSH)-producing cells, and luteinizing hormone (LH)-producing cells. Whether adenohypophysis or pituitary hormone-producing cell is contained in a tissue derived from the living body and a cell population generated by the production method of the present invention can be detected by a method such as immunohistochemistry using growth hormone (GH)-producing cell markers (anti-Pit1 antibody, anti-GH antibody etc.), prolactin (PRL)-producing cell marker (anti-Pit1 antibody, anti-PRL antibody etc.), adrenocorticotropic hormone (ACTH)-producing cell marker (anti-T-Pit antibody, anti-NeuroD1 antibody, anti-ACTH antibody etc.), thyroid-stimulating hormone (TSH)-producing cell marker (anti-GATA2 antibody, anti-ACTH antibody etc.), follicle-stimulating hormone (FSH)-producing cells, and luteinizing hormone (LH)-producing cell marker (anti-GATA2 antibody, anti-SF1 antibody, anti-FSH antibody, anti-LH antibody etc.), ELISA for secreted hormones and the like.

In the present invention, the "pituitary stem cell" refers to an undifferentiated multipotent stem cell or progenitor cell that is present in the pituitary gland and contributes to regeneration of pituitary tissue and supply of pituitary hormone-producing cells. Whether a pituitary stem cell is contained in the cell population or tissue produced by the production method of the present invention can be detected by a method such as immunohistochemistry using, for example, antibody to a pituitary stem cell markers such as Sox2, Sox9, E-Cadherin, Nestin, S100β, GFRα2, Prop1, CD133, β-Catenin, Klf4, Oct4, Pax6, Coxsackievirus and adenovirus common receptor (CXADR), PRRX1/2, Ephrin-B2, ACE and a cell proliferation marker such as Ki67, phosphorylated histone H3, MCM; proliferating cell labeling assay using nucleic acid analogue such as BrdU, EdU, IdU; uptake assay of fluorescence-labeled dipeptide (β-alanyl-lysyl-N-7-amino-4-methylcoumarin-3-acetic acid), pituitary sphere (pitusphere) formation assay.

In the present invention, the "mouth cavity epithelium" refers to epithelial tissue and cells thereof that form the oral cavity. Examples of the mouth cavity epithelium include oral mucosal epithelium, salivary gland epithelium, and odontogenic epithelium. The mouth cavity mucosal epithelium is generally a mucosal tissue consisting of stratified squamous epithelium, and includes basal cells, Merkel cells, melanin-producing cells, and the like on the basement membrane in contact with connective tissue, and thorn cells, granular cells, and stratum corneum layer are formed on the upper layer thereof. The mouth cavity mucosal epithelium can be detected as, for example, cytokeratin 7, 8, 13, 14, 19-positive tissue.

In the present invention, the "niche" or "stem cell niche" refers to a microenvironment involved in the proliferation, differentiation, maintenance of properties, and the like of stem cells. Examples of the niche in the living body include hematopoietic stem cell niche, hair follicle stem cell niche, intestinal epithelial stem cell niche, muscle stem cell niche, and pituitary niche. In these niches, stem cells unique to each tissue and supporting cells that provide the niche are present, and stem cells are maintained by cytokine, chemokine, extracellular matrix, cell adhesion factor, intercellular signal transduction factor and the like provided by the supporting cells.

In the present invention, the "pituitary niche" refers to a microenvironment involved in proliferation, differentiation, maintenance of properties, and the like of pituitary stem cells. Examples of the pituitary niche include Maginal Cell Layer (MCL) niche existing around the residual cavity (Rathke's cleft) that remains between the anterior pituitary and the middle lobe as a trace of the hollow part of the sac-like Rathke's pouch in the developing stage, and Parenchymal niche scattered in the anterior pituitary gland.

In the present invention, the "mesenchymal cell" is a non-epithelial cell that is mainly derived from mesoderm and neural crest and forms connective tissue. A part of these cells are multipotent somatic stem cells called mesenchymal stem cells. Whether a mesenchymal cell is contained in the cell population or tissue produced by the production method of the present invention can be detected by a method such as immunohistochemistry using an antibody to a mesenchymal cell marker such as Nestin, Vimentin, Cadherin-11, Laminin, and CD44. Whether a mesenchymal stem cell is included can be detected by a method such as immunohistochemistry using an antibody to a mesenchymal stem cell marker such as CD9, CD13, CD29, CD44, CD55, CD59, CD73, CD105, CD140b, CD166, VCAM-1, STRO-1, c-Kit, Sca-1, Nucleostemin, CDCP1, BMPR2, BMPR1A, and BPMR1B.

### 2. Production method of cell population containing pituitary tissue

The present invention provides a cell population containing pituitary tissue and a production method thereof. In the following, it is also to be referred to as the production method of the present invention.

One embodiment of the production method of the present invention is a method for producing a cell population containing pituitary tissue, including the following steps (1') - (2):
(1') a first step of culturing pluripotent stem cells in the presence of a JNK signal transduction pathway inhibiting substance to form a cell population,
(2) a second step of culturing the cell population obtained in the first step in the presence of a BMP signal transduction pathway activating substance and a Sonic hedgehog signal transduction pathway activating substance, thereby obtaining a cell population containing pituitary tissue.

In step (1'), preferably, a Wnt signal transduction pathway inhibiting substance is used in combination with a JNK signal transduction pathway inhibiting substance.

A more preferred embodiment of the production method of the present invention is a method for producing a cell population containing pituitary tissue, including the following step (a) and the following steps (1') and (2):
(a) step a of culturing pluripotent stem cells in the absence of feeder cells and in a medium comprising 1) a TGFβ family signal transduction pathway inhibiting substance and/or a Sonic hedgehog signal transduction pathway activating substance, and 2) a factor for maintaining an undifferentiated state,
(1') a first step of culturing (preferably suspension-culturing) the cells obtained in step a, in the presence of a JNK signal transduction pathway inhibiting substance,
(2) a second step of culturing (preferably suspension-culturing) the cell population obtained in the first step in the presence of a BMP signal transduction pathway activating substance and a Sonic hedgehog signal transduction pathway activating substance, thereby obtaining a cell population containing pituitary tissue.

Preferably, the first step is a step of forming a cell aggregate, and the cell population obtained in the first step and subjected to the second step may be a cell aggregate.

In step (1'), preferably, a Wnt signal transduction pathway inhibiting substance is used in combination with a JNK signal transduction pathway inhibiting substance.

A still more preferred embodiment of the production method of the present invention is a method for producing a cell population containing pituitary tissue, including the following step (a) and the following steps (1) to (3):
(a) step a of culturing pluripotent stem cells in the absence of feeder cells and in a medium comprising 1) a TGFβ family signal transduction pathway inhibiting substance and/or a Sonic hedgehog signal transduction pathway activating substance, and 2) a factor for maintaining an undifferentiated state,
   (1) a first step of culturing (preferably suspension-culturing) the cells obtained in step a, in the presence of a JNK signal transduction pathway inhibiting substance and a Wnt signal transduction pathway inhibiting substance,
   (2) a second step of culturing (preferably suspension-culturing) the cell population obtained in the first step in the presence of a BMP signal transduction pathway activating substance and a Sonic hedgehog signal transduction pathway activating substance,
   (3) a third step of culturing (preferably suspension-culturing) the cell population obtained in second step in the absence of a Sonic hedgehog signal transduction pathway activating substance to obtain a cell population containing pituitary tissue.

Preferably, the first step is a step of forming a cell aggregate, and the cell population obtained in the first step and subjected to the second step and the cell population obtained in the second step and subjected to the third step may be each a cell aggregate.

### <Step (a)>: step a

Step a of culturing pluripotent stem cells in the absence of feeder cells and in a medium comprising 1) a TGFβ family signal transduction pathway inhibiting substance and/or a Sonic hedgehog signal transduction pathway activating substance, and 2) a factor for maintaining an undifferentiated state is explained.

In step (a), the pluripotent stem cells are treated with a TGFβ family signal transduction pathway inhibiting substance and/or a Sonic hedgehog signal transduction pathway activating substance, and then subjected to culture (preferably suspension-culturing) in the first step. As a result, the state of pluripotent stem cells changes, the efficiency of forming nonneural epithelial tissue is improved, the quality of the resulting cell population (aggregate) is improved, differentiation becomes easier, cell death is less likely to occur, and the production efficiency of pituitary cells is improved.

Step (a) is performed in the absence of feeder cells.

The absence of feeder cells (feeder-free) in the present invention means a condition substantially free of feeder cells (e.g., the percentage of the number of feeder cells relative to the total number of cells is not more than 3%).

In the production method of the pituitary in the present invention, pluripotent stem cell is preferably an embryonic stem cell or an induced pluripotent stem cell. Induced pluripotent stem cell is available from given organizations, or a commercially available product can be purchased. For example, human induced pluripotent stem cell line 201B7 strain, 201B7-Ff cell, 253G1 cell, 253G4 cell, 1201C1 cell, 1205D1 cell, 1210B2 cell, and 1231A3 cell are available from Kyoto University and iPS Academia Japan, Inc. As induced pluripotent stem cell lines, for example, Ff-I01 cell, Ff-I14 cell, and QHJI01s04 cell, established in Kyoto University, are available from Kyoto University. Also, HC-6 #10 strain, 1231A3 strain, and 1383D2 strain are available from RIKEN.

The TGFβ family signal transduction pathway (i.e., TGFβ superfamily signal transduction pathway) is a signal transduction pathway intracellularly transduced by Smad family with transformation growth factor β (TGFβ), Nodal/Activin or BMP as a ligand.

The TGFβ family signal transduction pathway inhibiting substance is a substance that inhibits TGFβ family signal transduction pathway, that is, a signal transduction pathway transduced by the Smad family. Specifically, a TGFβ signal transduction pathway inhibiting substance, a Nodal/Activin signal transduction pathway inhibiting substance and a BMP signal transduction pathway inhibiting substance can be mentioned. As the TGFβ family signal transduction pathway inhibiting substance, a TGFβ signal transduction pathway inhibiting substance is preferable.

The TGFβ signal transduction pathway inhibiting substance is not particularly limited as long as it is a substance inhibiting a signal transduction pathway caused by TGFβ, and may be any of nucleic acid, protein and low-molecular organic compound. As the substance, for example, a substance directly acting on TGFβ (e.g., protein, antibody, and aptamer), a substance suppressing expression of gene encoding TGFβ (e.g., antisense oligonucleotide, and siRNA), a substance that inhibits the binding of TGFβ receptor and TGFβ, and a substance that inhibits physiological activity caused by signal transduction by the TGFβ receptor (e.g., TGFβ receptor inhibitor, and Smad inhibitor) can be mentioned. As a protein known as a TGFβ signal transduction pathway inhibiting substance, Lefty can be mentioned.

As a TGFβ signal transduction pathway inhibiting substance, compounds well known to those of ordinary skill in the art can be used. Specifically, Alk5/TGFβR1 inhibitors such as SB431542 (sometimes to be abbreviated as "SB431") (4-[4-(3,4-Methylenedioxyphenyl)-5-(2-pyridyl)-1H-imidazol-2-yl]benzamide), SB505124 (2-[4-(1,3-Benzodioxol-5-yl)-2-(1,1-dimethylethyl)-1H-imidazol-5-yl]-6-methylpyridine), SB525334 (6-[2-(1,1-Dimethylethyl)-5-(6-methyl-2-pyridinyl)-1H-imidazol-4-yl]quinoxaline), LY2157299 (4-[5,6-Dihydro-2-(6-methyl-2-pyridinyl)-4H-pyrrolo[1,2-b]pyrazol-3-yl]-6-quinolinecarboxamide), LY2109761 (4-[5,6-dihydro-2-(2-pyridinyl)-4H-pyrrolo[1,2-b]pyrazol-3-yl]-7-[2-(4-morpholinyl)ethoxy]-quinoline), GW788388 (4-{4-[3-(Pyridin-2-yl)-1H-pyrazol-4-yl]-pyridin-2-yl}-N-(tetrahydro-2H-pyran-4-yl)benzamide), LY364947 (4-[3-(2-Pyridinyl)-1H-pyrazol-4-yl]quinoline), SD-208 (2-[(5-Chloro-2-fluorophenyl)pteridin-4-yl]pyridin-4-yl-amine), EW-7197 (N-(2-fluorophenyl)-5-(6-methyl-2-pyridinyl)-4-[1,2,4]triazolo[1,5-a]pyridin-6-yl-1H-Imidazole-2-methanamine), A83-01 (3-(6-Methylpyridin-2-yl)-4-(4-quinolyl)-1-phenylthiocarbamoyl-1H-pyrazole), RepSox (2-[5-(6-Methylpyridin-2-yl)-1H-pyrazol-4-yl]-1,5-naphthyridine), SM16 (4-[4-(1,3-Benzodioxol-5-yl)-5-(6-methyl-2-pyridinyl)-1H-imidazol-2-yl]bicyclo[2.2.2]octane-1-carboxamide), R268712 (4-[2-Fluoro-5-[3-(6-methyl-2-pyridinyl)-1H-pyrazol-4-yl]phenyl]-1H-pyrazole-1-ethanol), IN1130 (3-[[5-(6-Methyl-2-pyridinyl)-4-(6-quinoxalinyl)-1H-imidazol-2-yl]methyl]benzamide), Galunisertib (4-[5,6-Dihydro-2-(6-methyl-2-pyridinyl)-4H-pyrrolo[1,2-b]pyrazol-3-yl]-6-quinolinecarboxamide), AZ12799734 (4-({4-[(2,6-dimethylpyridin-3-yl)oxy]pyridin-2-yl}amino)benzenesulfonamide), A77-01 (4-[3-(6-Methylpyridin-2-yl)-1H-pyrazol-4-yl]quinoline), KRCA 0008 (1,1-[(5-Chloro-2,4-pyrimidinediyl)bis[imino(3-methoxy-4,1-phenylene)-4,1-piperazinediyl]]bisethanone), GSK 1838705 (2-[[2-[[1-[(Dimethylamino)ethanoyl]-5-(methyloxy)-2,3-dihydro-1H-indol-6-yl]amino]-7H-pyrrolo[2,3-d]pyrimidin-4-yl]amino]-6-fluoro-N-methylbenzamide), Crizotinib (3-[(1R)-1-(2,6-Dichloro-3-fluorophenyl)ethoxy]-5-[1-(piperidin-4-yl)-1H-pyrazol-4-yl]-2-aMinopyridine), Ceritinib (5-Chloro-N2-[2-isopropoxy-5-Methyl-4-(4-piperidyl)phenyl]-N4-(2-isopropylsulfonylphenyl)pyriMidine-2,4-diaMine), ASP 3026(N2-[2-Methoxy-4-[4-(4-methyl-1-piperazinyl)-1-piperidinyl]phenyl]-N4-[2-[(1-methylethyl)sulfonyl]phenyl]-1,3,5-triazine-2,4-diamine), TAE684(5-Chloro-N2-[2-methoxy-4-[4-(4-methyl-1-piperazinyl)-1-piperidinyl]phenyl]-N4-[2-[(1-methylethyl)sulfonyl]phenyl]-2,4-pyrimidinediamine), AZD3463 (N-[4-(4-Amino-1-piperidinyl)-2-methoxyphenyl]-5-chloro-4-(1H-indol-3-yl)-2-pyrimidinamine), TP0427736 (6-[4-(4-methyl-1,3-thiazol-2-yl)-1H-imidazol-5-yl]-1,3-benzothiazole), TGFBR1-IN-1 (5-(1,3-benzothiazol-6-yl)-N-(4-hydroxyphenyl)-1-(6-methylpyridin-2-yl)pyrazole-3-carboxamide), TEW-7197 (2-fluoro-N-[[5-(6-methylpyridin-2-yl)-4-([1,2,4]triazolo[1,5-a]pyridin-6-yl)-1H-imidazol-2-yl]methyl]aniline), LY3200882 (2-[4-[[4-[1-cyclopropyl-3-(oxan-4-yl)pyrazol-4-yl]oxypyridin-2-yl]amino]pyridin-2-yl]propan-2-ol), BIBF-0775 ((3Z)-N-Ethyl-2,3-dihydro-N-methyl-2-oxo-3-[phenyl[[4-(1-piperidinylmethyl)phenyl]amino]methylene]-1H-indole-6-carboxamide), and the like, SMAD3 inhibitors such as SIS3 (1-(3,4-dihydro-6,7-dimethoxy-2(1H)-isoquinolinyl)-3-(1-methyl-2-phenyl-1H-pyrrolo[2,3-b]pyridin-3-yl)-2-propen-1-one) and the like, receptor degradation promoters such as ITD-1 (4-[1,1'-Biphenyl]-4-yl-1,4,5,6,7,8-hexahydro-2,7,7-trimethyl-5-oxo-3-quinolinecarboxylic acid ethyl ester) and the like, derivatives of these compounds, and the like can be mentioned. These substances may be used alone or in combination. SB431542 is a compound known as an inhibitor of TGFβ receptor (ALK5) and Activin receptor (ALK4/7) (i.e., TGFβR inhibitor). SIS3 is a TGFβ signal transduction pathway inhibiting substance that inhibits phosphorylation of SMAD3 which is an intracellular signal transduction factor under the control of TGFβ receptor. ITD-1 is a proteasomal degradation promoter of TGF-β type II receptor. It is known to those skilled in the art that the above-mentioned compounds and the like have activity as TGFβ signal transduction pathway inhibiting substances (e.g., described in Expert opinion on investigational drugs, 2010, 19.1: 77-91. and the like).

The TGFβ signal transduction pathway inhibiting substance preferably contains an Alk5/TGFβR1 inhibitor. The Alk5/TGFβR1 inhibitor preferably contains at least one selected from the group consisting of SB431542, SB505124, SB525334, LY2157299, GW788388, LY364947, SD-208, EW-7197, A83-01, RepSox, SM16, R268712, IN1130, Galunisertib, AZ12799734, A77-01, KRCA 0008, GSK 1838705, Crizotinib, Ceritinib, ASP 3026, TAE684, AZD3463, and TP0427736, further preferably SB431542 or A83-01.

The concentration of a TGFβ signal transduction pathway inhibiting substance in the medium can be appropriately determined according to the substances used within a range capable of achieving the aforementioned effects. When SB431542 is used as the TGFβ signal transduction pathway inhibiting substance in step (a), it is generally used at a concentration of about 1 nM to about 100 pM, preferably about 10 nM - about 100 pM, more preferably about 10 nM to about 50 pM, further preferably about 100 nM to about 50 pM, particularly preferably about 1 µM to about 10 µM. When a TGFβ signal transduction pathway inhibiting substance other than SB431542 is used, it is desirably used at a concentration that shows TGFβ signal transduction pathway inhibiting activity equivalent to that of SB431542 at the above-mentioned concentration. The TGFβ signal transduction pathway inhibitory activity of SB431542 and the like can be determined by methods well known to those skilled in the art, for example, by detecting Smad phosphorylation by Western blotting (Mol Cancer Ther.(2004) 3,737-45.).

The Shh signal pathway activating substance is a substance capable of enhancing signal transduction mediated by Shh. Examples of the Shh signal transduction pathway activating substance include proteins belonging to the Hedgehog family (e.g., Shh, Ihh), Shh receptor, Shh receptor agonist, Smo agonist, Purmorphamine (9-cyclohexyl-N-[4-(morpholinyl)phenyl]-2-(1-naphthalenyloxy)-9H-purin-6-amine), GSA-10 (Propyl 4-(1-hexyl-4-hydroxy-2-oxo-1,2-dihydroquinoline-3-carboxamido)benzoate), Hh-Ag1.5, 20(S)-Hydroxycholesterol, SAG (Smoothened Agonist: N-Methyl-N'-(3-pyridinylbenzyl)-N'-(3-chlorobenzo[b]thiophene-2-carbonyl)-1,4-diaminocyclohexane), 20(S)-hydroxy Cholesterol(3S,8S,9S,10R,13S,14S,17S)-17-[(2R)-2-hydroxy-6-methylheptan-2-yl]-10,13-dimethyl-2,3,4,7,8,9,11,12,14,15,16,17-dodecahydro-1H-cyclopenta[a]phenanthren-3-ol), and the like. These substances may be used alone or in combination. It is known to those skilled in the art that the above-mentioned compounds and the like have activity as Shh signal transduction pathway activating substances (e.g., described in Molecular BioSystems, 2010, 6.1: 44-54.).

The Shh signal transduction pathway activating substance preferably contains at least one selected from the group consisting of SAG, Purmorphamine, and GSA-10, more preferably SAG. The concentration of the Shh signal transduction pathway activating substance in the medium can be appropriately determined, according to the substances to be used, to fall within a range capable of achieving the aforementioned effects. In step (a), SAG is generally used at a concentration of about 1 nM to about 2000 nM, preferably about 10 nM to about 1000 nM, more preferably about 10 nM to about 700 nM, further preferably about 50 nM to about 700 nM, particularly preferably about 100 nM to about 600 nM, most preferably about 100 nM to about 500 nM. When a Shh signal transduction pathway activating substance other than SAG is used, it is desirably used at a concentration that shows Shh signal transduction promoting activity equivalent to that of SAG at the aforementioned concentration. Shh signal transduction promoting activity can be determined by a method well known to those of ordinary skill in the art, for example, reporter gene assay focusing on the expression of Gli1 gene (Oncogene (2007) 26, 5163-5168).

To enable culturing to maintain undifferentiated state, the medium used in step (a) contains a factor for maintaining an undifferentiated state. The factor for maintaining an undifferentiated state is not particularly limited as long as it is a substance having an action to suppress differentiation of pluripotent stem cells. Examples of the factor for maintaining an undifferentiated state widely used by those of ordinary skill in the art include an FGF signal transduction pathway activating substance, a TGFβ family signal transduction pathway activating substance, and insulin in the case of primed pluripotent stem cells (e.g., human ES cells, human iPS cells). As the FGF signal transduction pathway activating substance, fibroblast growth factors (e.g., bFGF, FGF4, FGF8) can be specifically mentioned. As the TGFβ family signal transduction pathway activating substance, a TGFβ signal transduction pathway activating substance, a Nodal/Activin signal transduction pathway activating substance can be mentioned. As the TGFβ signal transduction pathway activating substance, for example, TGFβ1, TGFβ2 can be mentioned. As the Nodal/Activin signal transduction pathway activating substance, for example, Nodal, Activin A, Activin B can be mentioned. These substances may be used alone or in combination. When human pluripotent stem cells (e.g., human ES cells, human iPS cells) are cultured, the medium in step (a) preferably contains bFGF as a factor for maintaining an undifferentiated state.

The factor for maintaining an undifferentiated state to be used is generally a factor for maintaining an undifferentiated state of mammals. Since the factor for maintaining an undifferentiated state may have cross-reactivity among mammal species, a factor for maintaining an undifferentiated state of any mammal may also be used as long as the undifferentiated state of the pluripotent stem cells to be cultured can be maintained. Preferably, the factor for maintaining an undifferentiated state is a factor for maintaining an undifferentiated state of a mammal of the same species as the cells to be cultured. For example, for the culturing of human pluripotent stem cells, human factor for maintaining an undifferentiated state (e.g., bFGF, FGF4, FGF8, EGF, Nodal, Activin A, Activin B, TGFβ 1, and TGFβ 2) are used. The factor for maintaining an undifferentiated state is preferably isolated.

The factor for maintaining an undifferentiated state can be produced by any host or artificially synthesized as long as it has the ability to maintain an undifferentiated state of the pluripotent stem cells to be cultured. The factor for maintaining an undifferentiated state used in the present invention is preferably one that has undergone the same modification as that produced in vivo, and further preferably one produced under conditions that do not contain foreign components, in cells of the same type as the pluripotent stem cells to be cultured.

One embodiment of the production method of the present invention includes a step of providing an isolated factor for maintaining an undifferentiated state. One embodiment of the production method of the present invention includes a step of extrinsically (exogenously) adding an isolated factor for maintaining an undifferentiated state to a medium used in step (a). A factor for maintaining an undifferentiated state may be added in advance to a medium to be used in step (a).

The concentration of the factor for maintaining an undifferentiated state in the medium to be used in step (a) is a concentration capable of maintaining the undifferentiated state of the pluripotent stem cells to be cultured, and can be appropriately determined by those of ordinary skill in the art. For example, when bFGF is used as a factor for maintaining an undifferentiated state in the absence of feeder cells, the concentration thereof is generally about 4 ng/mL - about 500 ng/mL, preferably about 10 ng/mL - about 200 ng/mL, more preferably about 30 ng/mL - about 150 ng/mL.

Step (a) is performed in the absence of feeder cells. In step (a), the pluripotent stem cells may be cultured under any conditions of suspension culturing and adhesion culturing, preferably adhesion culturing. For culturing pluripotent stem cells under feeder-free conditions, an appropriate matrix may be used as a scaffold to provide a scaffold in stead of the feeder cells to the pluripotent stem cell. The pluripotent stem cells are subjected to adhesion culturing in a culture vessel whose surface is coated with a matrix as a scaffold.

As a matrix available as a scaffold, laminin (Nat Biotechnol 28, 611-615 (2010)), laminin fragment (Nat Commun 3, 1236 (2012)), basement membrane preparation (Nat Biotechnol 19, 971-974 (2001)), gelatin, collagen, heparan sulfate proteoglycan, entactin, vitronectin and the like can be mentioned. Laminin 511 is preferably used as the matrix (Nat Biotechnol 28, 611-615 (2010)).

A laminin fragment is not particularly limited as long as it has adhesiveness to pluripotent stem cells and enables maintenance culturing of pluripotent stem cell under feeder-free conditions, and is preferably E8 fragment. Laminin E8 fragment was identified as a fragment with strong cell adhesion activity among the fragments obtained by digestion of laminin 511 with elastase (EMBO J., 3:1463-1468, 1984, J. Cell Biol., 105:589-598, 1987). E8 fragment of laminin 511 is preferably used (Nat Commun 3, 1236 (2012), Scientific Reports 4, 3549 (2014)). The laminin E8 fragment is not required to be an elastase digestion product of laminin and may be a recombinant. Alternatively, it may be produced by a gene recombinant animal (Bombyx mori, etc.). To avoid contamination of unidentified components, a recombinant laminin fragment is preferably used. An E8 fragment of laminin 511 is commercially available and can be purchased from, for example, Nippi, Inc., and the like.

To avoid contamination with unidentified components, the laminin or laminin fragment to be used in the present invention is preferably isolated. Preferably, in the culturing of pluripotent stem cells under feeder-free conditions in step (a), pluripotent stem cells are cultured in an adhered state in a culture vessel with surface coated with isolated laminin 511 or E8 fragment of laminin 511, more preferably E8 fragment of laminin 511.

The medium to be used in step (a) is not particularly limited as long as it is a medium enabling culturing of pluripotent stem cells to maintain undifferentiated state under feeder-free conditions (feeder-free medium).

The medium to be used in step (a) may be a serum-containing medium or serum-free medium. To avoid contamination with chemically undetermined components, the medium to be used in step (a) is preferably a serum-free medium. The medium may contain a serum replacement.

While the period for the culturing of pluripotent stem cells in step (a) is not particularly limited as long as the effect of improving the quality of the cell population (aggregate) formed in subsequent first step can be achieved, it is generally 0.5 to 144 hr, preferably 2 to 96 hr, more preferably 6 to 48 hr, further preferably 12 to 48 hr, particularly preferably 18 to 28 hr, for example, 24 hr. That is, step (a) is started 0.5 to 144 hr (preferably 18 to 28 hr) before the start of first step, and the first step is continuously performed on completion of step (a).

In one preferable embodiment of step (a), human pluripotent stem cells are cultured in an adhered state in the absence of feeder cells and in a serum-free medium containing bFGF. The adhesion culturing is preferably performed in a culture vessel with surface coated with laminin 511, E8 fragment of laminin 511 or vitronectin. The adhesion culturing is preferably performed using StemFit medium as a feeder-free medium. The StemFit medium contains bFGF as a component for maintaining an undifferentiated state (Scientific Reports (2014)4, 3594).

In one preferred embodiment of step (a), human pluripotent stem cells are cultured in suspension in the absence of feeder cells in a serum-free medium containing bFGF. In the suspension culturing, the human pluripotent stem cells may form aggregates of human pluripotent stem cells.

In step (a) and the below-mentioned steps, culture conditions such as culture temperature, CO₂ concentration and the like can be appropriately set. The culture temperature is, for example, about 30°C to 40°C, preferably about 37°C. When a bicarbonate-buffered medium is used, the CO₂ concentration is, for example, about 1% to 10%, preferably about 5%.

### <Step (1)>, <step (1')>: the first step

In step (1'), pluripotent stem cells are cultured in the presence of a c-Jun N-terminal kinase (JNK) signal transduction pathway inhibiting substance to obtain a cell population.

JNK is a kinase belonging to the MAPK family and is involved in intracellular signal transduction stimulated by various environmental stresses, inflammatory cytokines, growth factors, and GPCR agonists.

In the present invention, the JNK signal transduction pathway inhibiting substance is not limited as long as it can suppress the signal transduction transmitted by JNK. JNK signal transduction pathway inhibiting substance includes, for example, substances having the activity to inhibit signal transduction by mechanisms that inhibit enzymatic activity, multimerization, binding with other factors or nucleic acids, promote degradation, and the like, of factors upstream or downstream of the JNK signal transduction mechanism, or JNK itself. Examples of the JNK signal transduction pathway inhibiting substance include, but are not limited to, JNK inhibitor, Rac inhibitor, MKK inhibitor, MEK inhibitor, Src inhibitor, receptor tyrosine kinase (RTK) inhibitor, ASK inhibitor, and the like.

Examples of the c-Jun N-terminal kinase (JNK) inhibitor include JNK-IN-8 ((E)-3-(4-(dimethylamino)but-2-enamido)-N-(3-methyl-4-((4-(pyridin-3-yl)pyrimidin-2-yl)amino)phenyl)benzamide), SP600125 (Anthra[1-9-cd]pyrazol-6(2H)-one), DB07268 (2-[[2-[(3-Hydroxyphenyl)amino]-4-pyrimidinyl]amino]benzamide), Tanzisertib (trans-4-[[9-[(3S)-Tetrahydro-3-furanyl]-8-[(2,4,6-trifluorophenyl)amino]-9H-purin-2-yl]amino]cyclohexanol), Bentamapimod (1,3-Benzothiazol-2-yl)[2-[[4-[(morpholin-4-yl)methyl]benzyl]oxy]pyrimidin-4-yl]acetonitrile, TCS JNK 6o (N-(4-Amino-5-cyano-6-ethoxy-2-pyridinyl)-2,5-dimethoxybenzeneacetamide), SU3327 (5-[(5-Nitro-2-thiazolyl)thio]-1,3,4-thiadiazol-2-amine), CEP1347 ((9S,10R,12R)-5-16-Bis[(ethylthio)methyl]-2,3,9,10,11,12-hexahydro-10-hydroxy-9-methyl-1-oxo-9,12-epoxy-1H-diindolo[1,2,3-fg:3',2',1'-kl]pyrrolo[3,4-i][1,6]benzodiazocine-10-carboxylic acid methyl ester), c-JUN peptide, AEG3482 (6-Phenylimidazo[2,1-b]-1,3,4-thiadiazole-2-sulfonamide), TCS JNK 5a (N-(3-Cyano-4,5,6,7-tetrahydrobenzo[b]thienyl-2-yl)-1-naphthalenecarboxamide), BI-78D3 (4-(2,3-Dihydro-1,4-benzodioxin-6-yl)-2,4-dihydro-5-[(5-nitro-2-thiazolyl)thio]-3H-1,2,4-triazol-3-one), IQ-3 (11H-Indeno[1,2-b]quinoxalin-11-one O-(2-furanylcarbonyl)oxime), SR 3576 (3-[4-[[[(3-Methylphenyl)amino]carbonyl]amino]-1H-pyrazol-1-yl]-N-(3,4,5-trimethoxyphenyl)benzamide), IQ-1S (11H-Indeno[1,2-b]quinoxalin-11-one oxime sodium salt), JIP-1 (153-163), CC-401 (3-[3-[2-(1-Piperidinyl)ethoxy]phenyl]-5-(1H-1,2,4-triazol-5-yl)-1H-indazole dihydrochloride), BI-87G3 (2-(5-Nitrothiazol-2-ylthio)benzo[d]thiazole), AS601245(2-(1,3-benzothiazol-2-yl)-2-[2-(2-pyridin-3-ylethylamino)pyrimidin-4-yl]acetonitrile), CV-65 (3,7-Dimethyl-1,9-dihydropyrido[3,2-g]quinoline-2,5,8,10-tetrone), D-JNK1 (CAS No. 1445179-97-4), ER-358063, ER-409903, ER-417258, CC-359 ((4S)-4-(2,4-difluoro-5-pyrimidin-5-ylphenyl)-4-methyl-5,6-dihydro-1,3-thiazin-2-amine), CC-401 (3-[3-(2-piperidin-1-ylethoxy)phenyl]-5-(1H-1,2,4-triazol-5-yl)-1H-indazole), CC-930 (4-[[9-[(3S)-oxolan-3-yl]-8-(2,4,6-trifluoroanilino)purin-2-yl]amino]cyclohexan-1-ol), SB203580 (4-[4-(4-fluorophenyl)-2-(4-methylsulfinylphenyl)-1H-imidazol-5-yl]pyridine) and derivatives of these, and the like can be mentioned. These substances may be used alone or in combination. It is known to those skilled in the art that the above-mentioned compounds and the like have activity as JNK inhibitors (e.g., described in J Enzyme Inhib Med Chem. 2020; 35(1): 574-583.).

Examples of the Rac inhibitor include EHT1864 (5-(5-(7-(Trifluoromethyl)quinolin-4-ylthio)pentyloxy)-2-(morpholinomethyl)-4H-pyran-4-one dihydrochloride), NSC23766 (N6-[2-[[4-(Diethylamino)-1-methylbutyl]amino]-6-methyl-4-pyrimidinyl]-2-methyl-4,6-quinolinediamine trihydrochloride), EHop-016 (N4-(9-Ethyl-9H-carbazol-3-yl)-N2-[3-(4-morpholinyl)propyl]-2,4-pyrimidinediamine), 1A-116 (N-(3,5-Dimethylphenyl)-N'-[2-(trifluoromethyl)phenyl]guanidine), ZCL278 (2-(4-broMo-2-chlorophenoxy)-N-(4-(N-(4,6-diMethylpyriMidin-2-yl)sulfaMoyl)phenylcarbaMothioyl)acetaMide), MBQ-167 (9-ethyl-3-(5-phenyl-1H-1,2,3-triazol-1-yl)-9H-carbazole), KRpep-2d (actinium; [(2S)-1-[[(2S)-1-[[(2S)-1-[[(2S)-1-[[(3R,8R,11S,14S,20S,23S,26S,29S,32S,35S,38S)-8-[[(2S)-1-[[(2S)-1-[[(2S)-1-[[(2S)-1-amino-5-carbamimidamido-1-oxopentan-2-yl]amino]-5-carbamimidamido-1-oxopentan-2-yl]amino]-5-carbamimidamido-1-oxopentan-2-yl]amino]-5-carbamimidamido-1-oxopentan-2-yl]carbamoyl]-29-[(2R)-butan-2-yl]-20-(carboxymethyl)-26-(hydroxymethyl)-23,32-bis[(4-hydroxyphenyl)methyl]-35-(2-methylpropyl)-2,10,13,19,22,25,28,31,34,37-decaoxo-11-propan-2-yl-5,6-dithia-1,9,12,18,21,24,27,30,33,36-decazatricyclo[36.3.0.014,18]hentetracontan-3-yl]amino]-5-carbamimidamido-1-oxopentan-2-yl]amino]-5-carbamimidamido-1-oxopentan-2-yl]amino]-5-carbamimidamido-1-oxopentan-2-yl]amino]-5-carbamimidamido-1-oxopentan-2-yl]azanide), ARS-853 (1-[3-[4-[2-[[4-Chloro-2-hydroxy-5-(1-methylcyclopropyl)phenyl]amino]acetyl]-1-piperazinyl]-1-azetidinyl]-2-propen-1-one), Salirasib (2-(((2E,6E)-3,7,11-Trimethyldodeca-2,6,10-trien-1-yl)thio)benzoic acid), ML141 (4-(5-(4-methoxyphenyl)-3-phenyl-4,5-dihydropyrazol-1-yl)benzenesulfonamide) and derivatives of these, and the like can be mentioned. These substances may be used alone or in combination. It is known to those skilled in the art that the above-mentioned compounds and the like have activity as Rac inhibitors (e.g., described in Cancer research, 2018, 78.12: 3101-3111.).

The timing of addition of the JNK signal transduction pathway inhibiting substance in the present invention is not limited as long as the effect of improving the efficiency of producing pituitary tissue from human pluripotent stem cells is exerted. In the below-mentioned step (2), it is preferable that the BMP signal transduction pathway inhibiting substance should be added already when a BMP signal transduction pathway activating substance is added, which is within 72 hours from the start of differentiation induction. A more preferred timing for adding the JNK inhibitor is at the same time as the start of differentiation induction.

In the medium in step (1'), Wnt signal transduction pathway inhibiting substance is further preferably present. Such first step is step (1). That is, step (1) is the first step of culturing pluripotent stem cells in the presence of a JNK signal transduction pathway inhibiting substance and a Wnt signal transduction pathway inhibiting substance.

The Wnt signal transduction pathway is a signal transduction pathway that uses a Wnt family protein as a ligand and mainly uses Frizzled as a receptor. Examples of the signal transduction pathway include classical Wnt pathway (Canonical Wnt pathway), non-classical Wnt pathway (Non-Canonical Wnt pathway), and the like. The classical Wnt pathway is transmitted by β-Catenin. The Non-classical Wnt pathway includes Planar Cell Polarity (PCP) pathway, Wnt/JNK pathway, Wnt/Calcium pathway, Wnt-RAP1 pathway, Wnt-Ror2 pathway, Wnt-PKA pathway, Wnt-GSK3MT pathway, Wnt-aPKC pathway, Wnt-RYK pathway, and Wnt-mTOR pathway. In the Non-classical Wnt pathway, a common signal transduction factor which is also activated in other signaling pathways other than Wnt is present. In the present invention, such factors other than the aforementioned JNK pathway are also considered the constitution factors of the Wnt signal transduction pathway and inhibiting substances of the factors are also included in the Wnt signal transduction pathway inhibiting substance.

The Wnt signal transduction pathway inhibiting substance is not limited as long as it can suppress signal transduction induced by Wnt family proteins. The inhibiting substance may be any of nucleic acid, protein and low-molecular organic compound. Examples of the substance include a substance that inhibits Wnt processing and extracellular secretion, a substance that directly acts on Wnt (e.g., protein, antibody, and aptamer), a substance that suppresses expression of a gene encoding Wnt (e.g., antisense oligonucleotide, siRNA, CRISPRi, etc.), a substance that suppresses binding of Wnt receptor and Wnt, and a substance that suppresses physiological activity caused by signal transduction by Wnt receptor.

As a protein known as a Wnt signal transduction pathway inhibiting substance, proteins belonging to secreted Frizzled Related Protein (sFRP) class (sFRP1 to 5, Wnt inhibitory Factor-1 (WIF-1), Cerberus), proteins belonging to Dickkopf (Dkk) class (Dkk1 to 4, Kremen), APCDD1, APCDD1L, proteins belonging to Draxin family, IGFBP-4, Notum, proteins belonging to SOST/Sclerostin family, and the like can be mentioned.

As the Wnt signal transduction pathway inhibiting substance, a compound well known to those of ordinary skill in the art can be used. As inhibiting substance for the classical Wnt signal transduction pathway, for example, Frizzled inhibitor, Dishevelled (Dvl) inhibitor, Tankyrase inhibitor, casein kinase 1 inhibitor, catenin responsive transcription inhibitor, p300 inhibitor, CREB-binding protein (CBP) inhibitor, BCL-9 inhibitor, TCF degrader (Am J Cancer Res. 2015; 5(8): 2344-2360), and the like can be mentioned. As inhibiting substance for the non-classical Wnt signal transduction pathway, for example, Porcupine (PORCN) inhibitor, Calcium/calmodulin-dependent protein kinase II (CaMKII) inhibitor, TGF-β-activated kinase 1 (TAK1) inhibitor, Nemo-Like Kinase (NLK) inhibitor, LIM Kinase inhibitor, mammalian target of rapamycin (mTOR) inhibitor, Rac inhibitor, c-Jun NH 2-terminal kinase (JNK) inhibitor, protein kinase C (PKC) inhibitor, Methionine Aminopeptidase 2 (MetAP2) inhibitor, Calcineurin inhibitor, nuclear factor of activated T cells (NFAT) inhibitor, ROCK inhibitor, and the like can be mentioned. While the action mechanism has not been reported, KY-02111 (N-(6-Chloro-2-benzothiazolyl)-3,4-dimethoxybenzenepropanamide) and KY03-I (2-(4-(3,4-dimethoxyphenyl)butanamide)-6-Iodobenzothiazole) can be recited as the Wnt signal transduction pathway inhibiting substance. These substances may be used alone or in combination.

As the PORCN inhibitor for example, IWP-2(N-(6-Methyl-2-benzothiazolyl)-2-[(3,4,6,7-tetrahydro-4-oxo-3-phenylthieno[3,2-d]pyrimidin-2-yl)thio]acetamide), IWP-3(2-[[3-(4-fluorophenyl)-3,4,6,7-tetrahydro-4-oxothieno[3,2-d]pyrimidin-2-yl]thio]-N-(6-methyl-2-benzothiazolyl)acetamide), IWP-4(N-(6-methyl-2-benzothiazolyl)-2-[[3,4,6,7-tetrahydro-3-(2-methoxyphenyl)-4-oxothieno[3,2-d]pyrimidin-2-yl]thio]acetamide), IWP-L6(N-(5-phenyl-2-pyridinyl)-2-[(3,4,6,7-tetrahydro-4-oxo-3-phenylthieno[3,2-d]pyrimidin-2-yl)thio]acetamide), IWP-12(N-(6-Methyl-2-benzothiazolyl)-2-[(3,4,6,7-tetrahydro-3,6-dimethyl-4-oxothieno[3,2-d]pyrimidin-2-yl)thio]acetamide), IWP-O1(1H-1,2,3-Triazole-1-acetamide,5-phenyl-N-(5-phenyl-2-pyridinyl)-4-(4-pyridinyl)-), LGK-974(2-(2',3-Dimethyl-2,4'-bipyridin-5-yl)-N-(5-(pyrazin-2-yl)pyridin-2-yl)acetamide), Wnt-C59(2-[4-(2-Methylpyridin-4-yl)phenyl]-N-[4-(pyridin-3-yl)phenyl]acetamide), ETC-131, ETC-159(1,2,3,6-Tetrahydro-1,3-dimethyl-2,6-dioxo-N-(6-phenyl-3-pyridazinyl)-7H-purine-7-acetamide), GNF-1331(N-(6-methoxy-1,3-benzothiazol-2-yl)-2-[(4-propyl-5-pyridin-4-yl-1,2,4-triazol-3-yl)sulfanyl]acetamide), GNF-6231(N-[5-(4-Acetyl-1-piperazinyl)-2-pyridinyl]-2'-fluoro-3-methyl[2,4'-bipyridine]-5-acetamide), Porcn-IN-1(N-[[5-fluoro-6-(2-methylpyridin-4-yl)pyridin-3-yl]methyl]-9H-carbazole-2-carboxamide), RXC004, CGX1321, and derivatives of these, and the like can be mentioned. These substances may be used alone or in combination.

The Wnt signal transduction pathway inhibiting substance preferably contains at least one selected from the group consisting of PORCN inhibitor, KY02111, and KY03-I, more preferably PORCN inhibitor. The Wnt signal transduction pathway inhibiting substance also preferably contains a substance having inhibitory activity against non-classical Wnt pathway of Wnt. The Wnt signal transduction pathway inhibiting substance more preferably contains a substance having inhibitory activity against Wnt/Planar Cell Polarity (PCP) pathway. The PORCN inhibitor used in the present invention preferably contains at least one selected from the group consisting of IWP-2, IWP-3, IWP-4, IWP-L6, IWP-12, LGK-974, Wnt-C59, ETC-159, and GNF-6231, more preferably IWP-2 or Wnt-C59, further preferably IWP-2.

The concentration of a Wnt signal transduction pathway inhibiting substance in the medium can be appropriately determined according to the substances used within a range capable of achieving the aforementioned effects. From the aspects of improving the production efficiency of the cells constituting the pituitary, for example, when IWP-2 which is one kind of PORCN inhibitor is used as the Wnt signal transduction pathway inhibiting substance, the concentration thereof is generally about 10 nM to about 50 µM, preferably about 10 nM to about 30 µM, further preferably about 100 nM to about 10 µM, most preferably about 0.5 µM. When Wnt-C59 which is one kind of PORCN inhibitor is used, the concentration thereof is generally about 10 pM to about 1 µM, preferably about 100 pM to about 500 nM, more preferably about 50 nM. When KY02111 is used, the concentration thereof is generally about 10 nM to about 50 µM, preferably about 10 nM to about 30 µM, more preferably about 100 nM to about 10 µM, further preferably about 5 µM. When a Wnt signal transduction pathway inhibiting substance other than the above is used, it is desirably used at a concentration that shows Wnt signal transduction pathway inhibiting activity equivalent to that of the above-mentioned concentration.

The medium in the first step (step (1) or step (1')) preferably further contains a TGFβ signal transduction pathway inhibiting substance. As a TGFβ signal transduction pathway inhibiting substance to be used in the first step, those similar to the ones exemplified in step (a) can be used. The TGFβ signal transduction pathway inhibiting substances in step (a) and the first step may be the same or different, preferably the same.

The concentration of a TGFβ signal transduction pathway inhibiting substance in the medium can be appropriately determined according to the substances used within a range capable of achieving the aforementioned effects. When SB431542 is used as the TGFβ signal transduction pathway inhibiting substance, it is generally used at a concentration of about 1 nM to about 100 µM, preferably about 10 nM - about 100 µM, more preferably about 100 nM to about 50 µM, further preferably about 500 nM to about 10 µM. When a TGFβ signal transduction pathway inhibiting substance other than SB431542 is used, it is desirably used at a concentration that shows TGFβ signal transduction pathway inhibiting activity equivalent to that of SB431542 at the above-mentioned concentration.

In the first step and the steps thereafter, from the aspects of suppressing differentiation into mesendoderm and improving the production efficiency of ectoderm ·placode-like tissues, it is also preferable to add an inhibiting substance against Transforming growth factor-β-activated kinase 1 (TAK1). TAK1 is a serine-threonine protein kinase of the MAP kinase kinase kinase (MAPKKK) family that mediates signal transduction activated by TGFβ, bone morphogenetic protein (BMP), interleukin 1 (IL-1), TNF-α, and the like.

The TAK1 inhibiting substance is not limited as long as it can suppress signal transduction mediated by TAK1. It may be a nucleic acid, a protein, or a low-molecular organic compound. Such substances include, for example, substances that inhibit the binding of TAK1 to a substrate, substances that inhibit phosphorylation of TAK1, substances that promote dephosphorylation of TAK1, substances that inhibit transcription or translation of TAK1, substances that promote degradation of TAK1, and the like.

As the TAK1 inhibiting substance, for example, (5Z)-7-Oxozeaenol((3S,5Z,8S,9S,11E)-3,4,9,10-tetrahydro-8,9,16-trihydroxy-14-methoxy-3-methyl-1H-2-benzoxacyclotetradecine-1,7(8H)-dione), N-Des(aminocarbonyl)AZ-TAK1 inhibitor(3-Amino-5-[4-(4-morpholinylmethyl)phenyl]-2-thiophenecarboxamide), Takinib (N1-(1-Propyl-1H-benzimidazol-2-yl)-1,3-benzenedicarboxamide), NG25 (N-[4-[(4-Ethyl-1-piperazinyl)methyl]-3-(trifluoromethyl)phenyl]-4-methyl-3-(1H-pyrrolo[2,3-b]pyridin-4-yloxy)-benzamide trihydrochloride), Sarsasapogenin, and derivatives and analogs of these can be mentioned. These substances may be used alone or in combination.

The TAK1 inhibiting substance is preferably (5Z)-7-Oxozeaenol. When (5Z)-7-Oxozeaenol is used as the TAK1 inhibiting substance in the first step, it is generally used at a concentration of about 1 nM to about 100 µM, preferably about 10 nM to about 50 µM, more preferably about 100 nM to about 25 µM, further preferably about 500 nM to about 10 pM. When a TAK1 inhibiting substance other than (5Z)-7-Oxozeaenol is used, it is preferably used at a concentration that exhibits TAK1 inhibitory activity equivalent to that of (5Z)-7-Oxozeaenol at the above-mentioned concentration. The TAK1 inhibitory activity can be determined, for example, by methods such as kinase assay and the like described in Cell chemical biology 24.8(2017) :1029-1039. From the aspect of controlling the proportion of cells contained in pituitary tissue, the above-mentioned TAK1 inhibiting substance can be added at any stage of the first step and the steps thereafter, and then removed. In one preferred embodiment, the above-mentioned TAK1 inhibiting substance is added at the beginning of the below-mentioned step (b) .

The medium used in the first step is not particularly limited as long as it is as described in the above-mentioned definition. The medium to be used in the first step may be a serum-containing medium or serum-free medium. In order to avoid contamination with chemically undetermined components, a serum-free medium is preferably used in the present invention. In order to avoid complicated preparation, for example, a serum-free medium supplemented with an appropriate amount of a commercially available serum replacement such as KSR is preferably used. The amount of KSR to be added to a serum-free medium in the case of human ES cell is generally about 1% to about 30%, preferably about 2% to about 20%. Examples of the serum-free medium include a 1:1 mixture medium of IMDM and F-12 which is supplemented with 5% KSR, 450 µM 1-monothioglycerol, and 1xChemically Defined Lipid Concentrate, or GMEM medium supplemented with 5% to 20% KSR, NEAA, pyruvic acid, and 2-mercaptoethanol.

At the start of the first step, cells may be in either an adherent state or a floating state. In a preferred embodiment, pluripotent stem cells are dispersed into single cells and then reaggregated to form cell aggregates in a floating state. For this purpose, it is preferable to perform an operation to disperse the pluripotent stem cells, for example the pluripotent stem cells obtained in step (a), into single cells before the start of the first step. The "dispersed cells" obtained by the dispersing operation are preferably single cells, but may also include masses of cells consisting of a small number of cells, for example, not less than 2 and not more than 100, and may contain masses of cells consisting of not less than 2 and not more than 50 cells. The "dispersed cells" may contain, for example, 70% or more of single cells and 30% or less of cell masses, preferably 80% or more of single cells and 20% or less of cell masses.

As a method for dispersing pluripotent stem cells, mechanical dispersion treatment, cell dissociation solution treatment, and cell protectant addition treatment can be mentioned, and these treatments may be performed in combination. As a method for dispersing the cells, preferably, a cell dissociation solution is performed simultaneously with a cell protectant addition treatment, and then a mechanical dispersion treatment is performed.

As a cell protectant to be used for a cell protectant addition treatment, FGF signal transduction pathway activating substance, heparin, Rho-associated protein kinase (ROCK) inhibiting substance, myosin inhibiting substance, polyamines, integrated stress response (ISR) inhibitor, caspase inhibitor, cell adhesion-promoting substance, serum, or serum replacement can be mentioned. As a preferred cell protectant, ROCK inhibiting substances can be mentioned. In order to suppress cell death of pluripotent stem cells (particularly, human pluripotent stem cells) induced by dispersing, it is preferable to add a ROCK inhibiting substance from the start of culture in the first step. Examples of the ROCK inhibiting substance include Y-27632 ((R)-(+)-trans-4-(1-Aminoethyl)-N-(4-pyridyl)cyclohexanecarboxamide, dihydrochloride), Fasudil (HA1077) (1-(5-Isoquinolinylsulfonyl)homopiperazine, hydrochloride), H-1152 (5-[[(2S)-hexahydro-2-methyl-1H-1,4-diazepin-1-yl]sulfonyl]-4-methyl-isoquinoline, dihydrochloride), HA-1100 (Hydroxyfasudil) (1-(1-Hydroxy-5-isoquinolinesulfonyl)homopiperazine, hydrochloride), Chroman 1 ((3S)-N-[2-[2-(dimethylamino)ethoxy]-4-(1H-pyrazol-4-yl)phenyl]-6-methoxy-3,4-dihydro-2H-chromene-3-carboxamide), Belumosudil (KD025, 2-[3-[4-[(1H-Indazol-5-yl)amino]quinazolin-2-yl]phenoxy]-N-isopropylacetamide), HSD1590 ([2-Methoxy-3-(4,5,10-triazatetracyclo[7.7.0.02,6.012,16]hexadeca-1(9),2(6),3,7,10,12(16)-hexaen-11-yl)phenyl]boronic acid), CRT0066854 ((S)-3-phenyl-N1-(2-pyridin-4-yl-5,6,7,8-tetrahydrobenzo[4,5]thieno[2,3-d]pyrimidin-4-yl)propane-1,2-diamine), RKI1447 (1-(3-hydroxybenzyl)-3-(4-(pyridin-4-yl)thiazol-2-yl)urea), Ripasudil (4-Fluoro-5-[[(2S)-hexahydro-2-methyl-1H-1,4-diazepin-1-yl]sulfonyl]isoquinoline), GSK269962A (N-[3-[2-(4-amino-1,2,5-oxadiazol-3-yl)-1-ethylimidazo[4,5-c]pyridin-6-yl]oxyphenyl]-4-(2-morpholin-4-ylethoxy)benzamide), GSK429286A (N-(6-fluoro-1H-indazol-5-yl)-2-methyl-6-oxo-4-(4-(trifluoromethyl)phenyl)-1,4,5,6-tetrahydropyridine-3-carboxamide), Y-33075 ((R)-4-(1-Aminoethyl)-N-1H-pyrrolo[2,3-b]pyridin-4-ylbenzamide), LX7101 (N,N-Dimethylcarbamic acid 3-[[[4-(aminomethyl)-1-(5-methyl-7H-pyrrolo[2,3-d]pyrimidin-4-yl)-4-piperidinyl]carbonyl]amino]phenyl ester), AT13148 ((alphaS)-alpha-(Aminomethyl)-alpha-(4-chlorophenyl)-4-(1H-pyrazol-4-yl)benzenemethanol), SAR407899 (6-(piperidin-4-yloxy)isoquinolin-1(2H)-one hydrochloride), GSK180736A (4-(4-fluorophenyl)-N-(1H-indazol-5-yl)-6-methyl-2-oxo-1,2,3,4-tetrahydropyrimidine-5-carboxamide), Hydroxyfasudil (1-(1-hydroxy-5-isoquinolinesulfonyl)homopiperazine, HCl), bdp5290 (4-Chloro-1-(4-piperidinyl)-N-[3-(2-pyridinyl)-1H-pyrazol-4-yl]-1H-pyrazole-3-carboxamide), sr-3677 (N-[2-[2-(Dimethylamino)ethoxy]-4-(1H-pyrazol-4-yl)phenyl]-2,3-dihydro-1,4-benzodioxin-2-carboxamidehydrochloride), CCG-222740 (N-(4-Chlorophenyl)-5,5-difluoro-1-(3-(furan-2-yl)benzoyl)piperidine-3-carboxamide), ROCK inhibitor-2 (N-[(1R)-1-(3-methoxyphenyl)ethyl]-4-pyridin-4-ylbenzamide), Rho-Kinase-IN-1 (N-[1-[(4-methylsulfanylphenyl)methyl]piperidin-3-yl]-1H-indazol-5-amine), ZINC00881524 (N-(4,5-dihydronaphtho[1,2-d]thiazol-2-yl)-2-(3,4-dimethoxyphenyl)acetamide), SB772077B ((3S)-1-[[2-(4-Amino-1,2,5-oxadiazol-3-yl)-1-ethyl-1H-imidazo[4,5-c]pyridin-7-yl]carbonyl]-3-pyrrolidinamine dihydrochloride), Verosudil (N-(1,2-Dihydro-1-oxo-6-isoquinolinyl)-alpha-(dimethylamino)-3-thiopheneacetamide), GSK-25 (4-(4-chloro-2-fluorophenyl)-2-(2-chloropyridin-4-yl)-1-(6-fluoro-1H-indazol-5-yl)-6-methyl-4H-pyrimidine-5-carboxamide) and derivatives of these and the like. As the cell adhesion-promoting substance, for example, adhesamine and adhesamine-RGDS derivatives (manufactured by Nagase Sangyo Co., Ltd.) and the like can be mentioned. As the cell protectant, a cell protectant after preparation can also be used. Examples of the cell protectant after preparation include RevitaCell Supplement (manufactured by Thermo Fisher Scientific), and CloneR, CloneR2 (manufactured by Stemcell Technologies). These substances may be used alone or in combination. In the first step, when ROCK inhibiting substance Y-27632 is added as a cell protectant, it is added into the culture environment at a concentration of generally about 10 nM to about 10 mM, preferably about 100 nM to about 1 mM, more preferably about 1 pM to about 100 pM. In the first step, when ROCK inhibiting substance Chroman 1 is added as a cell protectant, it is added into the culture environment at a concentration of generally about 10 pM to about 1 mM, preferably about 100 pM to about 100 µM, more preferably about 1 nM to about 10 µM.

As a cell dissociation solution to be used for the cell dissociation solution treatment, a solution containing an enzyme such as trypsin, collagenase, hyaluronidase, elastase, pronase, DNase, papain and so on, and at least one chelating agent such as ethylenediaminetetraacetic acid and so on can be mentioned. A commercially available cell dissociation solution such as TripLE Select (manufactured by Thermo Fisher Scientific), TripLE Express (manufactured by Thermo Fisher Scientific), Accumax (manufactured by Innovative Cell Technologies) can also be used. A cell dissociation solution preferred for the treatment of pluripotent stem cells obtained after step (a) is phosphate buffer (PBS) added with 5 mM EDTA, but it is not limited thereto.

As a method of mechanical dispersion treatment, a pipetting treatment or scraping by a scraper can be mentioned. The dispersed cells are suspended in the above-mentioned medium.

A method for dispersing pluripotent stem cells includes, for example, a method involving treating a colony of pluripotent stem cells with ethylenediaminetetraacetic acid, or Accumax in the presence of a ROCK inhibiting substance, and further dispersing them by pipetting.

When suspension culturing is performed in the first step, a suspension of the dispersed pluripotent stem cells is seeded in a cell non-adhesive culture vessel. When the culture vessel is non-adhesive, the cells are suspension cultured and a plurality of pluripotent stem cells gather and form cell aggregates.

In suspension culturing, plural cell aggregates may be simultaneously formed in one culture vessel by seeding the dispersed pluripotent stem cells in a comparatively large culture vessel such as a 10 cm dish. To prevent easy occurrence of size dispersion of each cell aggregate, for example, a given amount of the dispersed pluripotent stem cells are preferably seeded in each well of a multiwell plate (U-bottom, V-bottom) such as a non-cell adhesive 96-well microplate, and static culturing is performed, whereby the cells rapidly aggregate to form one cell aggregate in each well. For example, a culture vessel can be made non-cell-adhesive by processing such as coating the surface of the culture vessel with a superhydrophilic polymer. Examples of the non-cell-adhesive muliwell plate include PrimeSurface 96V bottom plate (MS-9096V, manufactured by SUMITOMO BAKELITE CO., LTD.). Centrifugation may be performed to form cell aggregates more quickly. By collecting cell aggregates formed in each well from a plurality of wells, a uniform population of cell aggregates can be obtained. When the cell aggregates are uniform, the production efficiency for each well and each repeated experiment can be more stabilized in the subsequent steps, and cells constituting the pituitary can be produced with higher reproducibility.

In another embodiment for forming cell aggregates from dispersed pluripotent stem cells, a culture vessel in which one well is divided into a plurality of microwells and two or more cell masses are formed can be used. In other words, in one aspect of the present embodiment, the suspension culturing of any one or more of the aforementioned first step, second step, step b, and third step is performed in a culture vessel in which at least one well is formed, the well is divided into a plurality of microwells, and suspension culturing is performed so that one cell mass is formed in each microwell, whereby cell masses in the number corresponding to the divided microwells in one well may be prepared. As the above-mentioned microwell, a culture vessel where the cells settle in one place on the bottom and the formation of aggregates is promoted, such as mortar, downward facing square pyramid, concave shape processing, grids, ridges, and the like formed in plurality, a culture vessel that has been treated to allow cells to adhere to only a part of the bottom surface thereof to facilitate the formation of aggregates, and the like can also be used. The culture area per well of the culture vessel having microwells is not particularly limited, but from the aspect of efficiently producing cell masses, the bottom area is preferably larger than 1 cm² (equivalent to a 48-well plate), more preferably larger than 2 cm² (equivalent to a 24-well plate), further preferably larger than 4 cm² (equivalent to a 12-well plate). Examples of the above-mentioned culture vessel include, but are not limited to, embryoid formation plate AggreWell (manufactured by STEMCELL Technologies), PAMCELL (manufactured by ANK), spheroid microplate (manufactured by Corning), NanoCulture Plate/Dish (manufactured by Organogenix), Cell-able (manufactured by Toyo Gosei Co., Ltd.), EZSPHERE (manufactured by AGC TECHNO GLASS CO., LTD.), SPHERICALPLATE 5D (manufactured by Mitokogyo Corporation), TASCL (manufactured by Cymss-bio Co., Ltd.), microwell bags (e.g., those described in Scientific reports, 2022, 12.1: 1-11.), and the like.

As the culture vessel, a three-dimensional cell culture container permitting exchange of the medium of the whole plate while cell aggregates are contained in each well is also preferred. Examples of such three-dimensional cell culture container include PrimeSurface 96 Slit well plate (manufactured by SUMITOMO BAKELITE CO., LTD.) and the like. This plate has narrow openings (slits) that allow the medium to enter and exit at the top of each of the 96 wells. The slits are set to have a width that makes it difficult for cell aggregates to pass through, which makes it possible to replace the medium in the entire plate at once while preventing cell aggregates from adhering to each other, and improve operation efficiency and quality of cell aggregates.

The concentration of the pluripotent stem cells in the first step can be appropriately set so that cell aggregates can be more uniformly and efficiently formed. For example, when human pluripotent stem cells (e.g., human iPS cell obtained in step (a)) are suspension cultured using a 96-well microwell plate, a liquid prepared to achieve generally about 1 × 10³ to about 1 × 10⁵ cells, preferably about 3 x 10³ to about 5 × 10⁴ cells, more preferably about 4 × 10³ to about 2 × 10⁴ cells, further preferably about 4 × 10³ to about 1.6 × 10⁴ cells, particularly preferably about 8 × 10³ to about 1.2 × 10⁴ cells, per well is added to each well, and the plate is left to stand to form cell aggregates. For example, when human pluripotent stem cells (e.g., human iPS cell obtained in step (a)) are suspension cultured using EZSPHERE SP dish 35 mm Type905 which is a culture vessel having about 260 microwells per dish, a liquid prepared to achieve generally about 1 × 10⁴ to about 1 × 10⁸ cells, preferably about 3 × 10⁴ to about 5 × 10⁷ cells, more preferably about 4 × 10⁴ to about 2 × 10⁷ cells, further preferably about 4 × 10⁴ to about 1.6 × 10⁷ cells, particularly preferably about 8 × 10⁴ to about 1.2 × 10⁷ cells, per dish is added to the dish, and the dish is left to stand to form cell aggregates. For example, when human pluripotent stem cells (e.g., human iPS cell obtained in step (a)) are suspension cultured using AggreWell 800, 6-well plate, which is a culture vessel with about 1800 microwells per well, a liquid prepared to achieve generally about 1 × 10⁴ to about 1 × 10⁸ cells, preferably about 3 × 10⁴ to about 5 × 10⁷ cells, more preferably about 4 × 10⁴ to about 2 × 10⁷ cells, further preferably about 4 × 10⁴ to about 1.6 × 10⁷ cells, particularly preferably about 8 × 10⁴ to about 1.2 × 10⁷ cells, per well is added to each well, and the plate is centrifuged to form cell aggregates. The number of cells can be determined by counting with a hemocytometer.

The period for suspension culturing necessary for forming a cell aggregate can be determined as appropriate according to the pluripotent stem cell to be used. To form uniformed cell aggregates, it is desirably as short as possible. The steps for the dispersed cells to form cell aggregates can be divided into a step for gathering cells, and a step for forming aggregates from the gathered cells. From seeding the dispersed cells (i.e., at the time of the start of suspension culturing) to allowing for gathering of the cells in case of human pluripotent stem cell (human iPS cell, etc.) is, for example, preferably within about 24 hr, more preferably within about 12 hr. In the step of seeding the dispersed cells (i.e., at the time of the start of suspension culturing) to allow for forming a cell aggregate in the case of human pluripotent stem cells (e.g., human iPS cells), it is, for example, preferably within about 72 hr, more preferably within about 48 hr. The time up to cell aggregate formation can be appropriately adjusted by adjusting tools for causing aggregation of cells, centrifugation conditions, and the like.

When a cell aggregate is formed by rapidly gathering pluripotent stem cells, an epithelium-like structure can be formed with good reproducibility in the cells induced and differentiated from the formed aggregate. Examples of the experimental operation to form a cell aggregate include a method involving keeping cells in a small space by using a plate with small wells (e.g., plate with wells having a base area of about 0.1 to 2.0 cm² when calculated in terms of flat bottom), micropore and so on, a method involving aggregating cells by centrifugation for a short time using a small centrifugation tube. As a plate with small wells, for example, 24 well plate (area of about 1.88 cm² when calculated in terms of flat bottom), 48 well plate (area of about 1.0 cm² when calculated in terms of flat bottom), 96 well plate (area of about 0.35 cm² when calculated in terms of flat bottom, inner diameter about 6 to 8 mm), and 384 well plate can be mentioned. Preferred is 96 well plate. As a shape of the plate with small wells, the shape of the bottom surface when the well is seen from above is, for example, polygon, rectangle, ellipse, true circle, preferably true circle. As a shape of the plate with small wells when the well is seen from the side, the shape of the bottom surface is preferably a structure having high outer circumference and low concave inner part, which is, for example, U-bottom, V-bottom or M-bottom, preferably U-bottom or V-bottom, most preferably V-bottom. As a plate with small wells, a cell culture dish (e.g., 60 mm to 150 mm dish, culture flask) with a concave convex, or dent on the bottom surface may also be used. The bottom surface of a plate with small wells is preferably a non-cell-adhesive bottom surface, preferably a non-cell-adhesive-coated bottom surface.

As another method for forming cell aggregates, it is also preferable to use a three-dimensional printing machine or a 3D printer. A cell population with a desired morphology can be prepared by a method in which spheroids consisting of a single dispersed cell or multiple cells are suspended in biocompatible ink (bioink) and output using a bio 3D printer (e.g., BIO X manufactured by Celllink, etc.), or a cell population is pricked with a needle and stack it up (Spike manufactured by Cyfuse Biomedical K.K., etc.).

Formation of cell aggregates can be determined based on the size and cell number of the cell aggregate, macroscopic morphology of the aggregate, microscopic morphology by tissue staining analysis and uniformity thereof, expression of differentiation and undifferentiation markers and uniformity thereof, control of expression of differentiation marker and synchronism thereof, reproducibility of differentiation efficiency between aggregates, and so on.

At the beginning of the first step, in a preferred embodiment, adhesion culturing is performed. The pluripotent stem cells on the culture vessel after step (a) may be used as they are in the first step, or the pluripotent stem cells may be dispersed into single cells and then seeded again on the adhesive culture vessel. During re-seeding after dispersing pluripotent stem cells into single cells, appropriate extracellular matrix or synthetic cell adhesion molecules may be used as a scaffold. With the scaffold, pluripotent stem cells can be adhesion cultured in a culture vessel with a coated surface. The extracellular matrix is preferably Matrigel or laminin. As the synthetic cell adhesion molecule, synthetic peptides containing cell adhesive domain, such as poly-D-lysine, RGD sequence, and the like can be mentioned. The number of cells seeded is not particularly limited as long as they differentiate into the pituitary. From the aspect of reproducing adhesion and interaction between cells, a density that allows the cell density to reach semi-confluence, which is equivalent to 60% or more of the culture space of the culture vessel, within 72 hours after seeding into the culture vessel is also preferred.

It is also preferable to use a micropatterned culture vessel as the adhesive culture vessel. The micropattern on the culture vessel can be composed of a cell-adhesive region and a cell-non-adhesive region, and cells are preferably adhesion cultured in the cell-adhesive region. The shapes of the cell adhesive region and the cell non-adhesive region are not limited as long as they can be developed on the culture vessel. Only one region of a cell adhesive region and a cell non-adhesive region may be formed or a plurality thereof may be formed on one culture vessel. The cell adhesive region is preferably artificially treated for the purpose of improving adhesiveness. Examples of culture vessel with micropatterns include CYTOOchip (manufactured by CYTOO), ibidi Micropatterning (manufactured by ibidi), and the like. Culture vessels can also be prepared using PDMS mold, matrix, and the like. Alternatively, culture vessels coated with an extracellular matrix, a substrate that promotes cell adhesion, and the like are processed with a laser or the like using a cell processing device (Model: CPD-017, manufactured by Kataoka Seisakusho Co., Ltd.) to form cell adhesive region and cell-non-adhesive region in any shape. When culturing the pluripotent stem cells obtained in step (a) on a micropatterned culture vessel, it can be performed, for example, with reference to previously reported methods (Nature protocols, 11(11), 2223-2232.).

It is also preferable that the culture vessel has a flow path (micro flow path) for perfusing the culture medium, and cells may be cultured in a perfusion environment in the first step and the steps thereafter. Such culture vessel is also referred to as a microfluid chip. The culture vessel (e.g., microfluid chip) may be connected by a flow path to another culture vessel (for example, microfluid chip) for culturing cells or tissues other than the cells cultured in the production method of the present invention. This reproduces the interaction between the pituitary and other cells or tissues. Other cells or tissues to be co-cultured with the pituitary include, but are not limited to, tissues that are regulated by hormones secreted by the pituitary, tissues that promote growth, differentiation, maturation, and survival of the pituitary, such as cells or tissues of the brain, blood vessel, bone, muscle, fat, thyroid, liver, adrenal gland, testes, ovary, and breast. Examples of methods for perfusing the medium include, but are not limited to, use of a magnetic stirrer, a peristaltic pump, and the like.

Culture vessel may have a membrane that is permeable to oxygen or a medium. Culture vessel may be capable of forming a concentration gradient of compounds, growth factors, and the like. The membrane is, for example, a porous membrane. In a culture vessel having such a membrane, cells can be cultured by the production method of the present invention on one side separated by the membrane, and other cells or tissues, feeder cells, and the like can be cultured on the other side. This makes it possible to culture cells constituting the pituitary, or progenitor cells thereof, and cell populations containing them without contaminating other cells or tissues.

In the first step and the steps thereafter, when a medium change operation is performed, for example, it can be performed by an operation to add a fresh medium without discarding the existing medium (medium addition operation), an operation to discard about a half amount of the existing medium (about 30 - 90%, for example, about 40 - 60% of the volume of the existing medium) and add about a half amount of a fresh medium (about 30 - 90%, for example, about 40 - 60% of the volume of the existing medium) (half-medium change operation), and an operation to discard about the whole amount of the existing medium (not less than 90% of the volume of the existing medium) and add about the whole amount of a fresh medium (not less than 90% of the volume of the existing medium) (full-medium change operation) and the like.

When a particular component is added at a certain time point, for example, an operation to calculate the final concentration, to discard about a half amount of the existing medium, and to add about a half amount of a fresh medium containing a particular component at a concentration higher than the final concentration (half amount medium change operation) may be performed. When the concentration of a component contained in the existing medium is to be decreased by dilution at a certain time point, for example, the medium change operation may be performed plural times per day, preferably plural times (e.g., 2 - 3 times) within 1 hr. Also, when the concentration of a component contained in the existing medium is to be decreased by dilution at a certain time point, the cell or cell aggregate may be transferred to another culture container. While the tool used for the medium change operation is not particularly limited, for example, pipetter, pipetteman (registered trademark), multichannel pipetteman, and continuous dispenser, can be mentioned. For example, when a 96 well plate is used as a culture vessel, a multichannel pipetter may be used.

The period for culturing in the first step is generally about 8 hr to 6 days, preferably about 12 hr to 60 hr.

In the first step and the steps thereafter, from the aspect of improving the production efficiency of pituitary, it is also preferable to add a compound for promoting differentiation into a placodal region. As a compound having the above-mentioned action, for example, BRL-54443, Phenanthroline and Parthenolide described in US20160326491A1, and the like can be mentioned. When BRL-54443 is used as a compound for promoting differentiation into a placodal region, it is used at a concentration of generally about 10 nM to about 100 µM; when Phenanthroline is used, it is used at a concentration of generally about 10 nM to about 100 µM; and when Parthenolide is used, it is used at a concentration of generally about 10 nM to about 100 µM.

In the first step, from the aspect of improving the efficiency of differentiation induction into pituitary, culturing may also be performed in the presence of a Sonic hedgehog signal transduction pathway activating substance. As the Sonic hedgehog signal transduction pathway activating substance used in the first step, one similar to those exemplified in step (a) can be used. The Shh signal transduction pathway activating substances in step (a) and step (1) may be the same or different, preferably the same, and is preferably SAG.

The concentration of Shh signal transduction pathway activating substance in the medium can be appropriately determined according to the substances used within a range capable of achieving the aforementioned effects. When SAG is used as the Shh signal transduction pathway activating substance in the first step, it is generally used at a concentration of generally about 1 nM to about 3 µM, preferably about 10 nM to about 2 µM, more preferably about 30 nM to about 1 µM, further preferably about 50 nM to about 500 nM.

### <Step (2)>: second step

In step (2), the cell population obtained in the first step is cultured in the presence of a BMP signal transduction pathway activating substance and a Sonic hedgehog signal transduction pathway activating substance. When cells are suspension cultured in the first step, the cell aggregates formed may be suspension cultured also in step (2). When cells are adhesion cultured in the first step, the cells may be continuously adhesion cultured also in step (2). After the cells are suspension cultured in the first step, they may be adhesion cultured in step (2).

The BMP signal transduction pathway activating substance is a substance capable of enhancing signal transduction mediated by BMP. Examples of the substance capable of enhancing signal transduction mediated by BMP include a substance that stabilizes BMP ligand in the culture environment and improves the titer, a substance that binds to type I BMP receptors ALK-1, ALK-2, ALK-3, and ALK-6 and activates and induces intracellular signal transduction downstream of the receptors, a substance that induces phosphorylation of Smad-1, Smad-5, Smad-8, and Smad-9 involved in intracellular BMP signal transduction, a substance that induces or enhances functions such as activation and suppression of gene transcription by Smad-1/5/8/9, and the like. Examples of the BMP signal transduction pathway activating substance include BMP proteins such as BMP2, BMP4, and BMP7, GDF proteins such as GDF5, 6 and 7, anti-BMP receptor antibody, BMP partial peptide, and the like. These substances may be used alone or in combination. As a definition of a BMP signal transduction pathway activating substance from the viewpoint of biological activity, for example, substances that have the ability to induce differentiation of cells such as the mouse pre-chondrogenic cell line ATDC5, mouse cranial vault-derived cell line MC3T3-E1, mouse striated muscle-derived cell line C2C12, and the like into osteoblast-like cells, and alkali phosphatase production induction potency. Examples of the substance with the above-mentioned activity include BMP2, BMP4, BMP5, BMP6, BMP7, BMP9, BMP10, BMP13/GDF6, BMP14/GDF5, GDF7 and the like.

BMP2 protein and BMP4 protein are available from, for example, R&D Systems, BMP7 protein is available from, for example, Biolegend, GDF5 protein is available from, for example, PeproTech, GDF6 protein is available from, for example, PrimeGene, and GDF7 protein is available from, for example, FUJIFILM Wako Pure Chemical Corporation. The BMP signal transduction pathway activating substance preferably contains at least one protein selected from the group consisting of BMP2, BMP4, BMP7, BMP13, and GDF7, more preferably BMP4.

The concentration of a BMP signal transduction pathway activating substance in the medium can be appropriately determined according to the substances used within a range capable of achieving the aforementioned effects. From the aspect of improving the production efficiency of cells constituting the pituitary, when BMP4 is used as a BMP signal transduction pathway activating substance, it is generally used at a concentration of about 1 pM to about 100 nM, preferably about 10 pM to about 50 nM, more preferably about 25 pM to about 25 nM, further preferably about 25 pM to about 5 nM, particularly preferably about 100 pM to about 5 nM, most preferably about 500 pM to about 2 nM. When a BMP signal transduction pathway activating substance other than BMP4 is used, it is desirably used at a concentration that shows BMP signal transduction pathway promoting activity equivalent to that of BMP4 at the aforementioned concentration. When using, for example, a commercially available recombinant BMP protein and the like as a BMP signal transduction pathway activating substance, those of ordinary skill in the art can easily determine the concentration of the BMP signal transduction pathway activating substance to be added by comparing the activity described in the product attachment, for example, the ED50 value of the ability to induce alkaline phosphatase production against the mouse pre-chondrogenic cell line ATDC5, and the concentration and activity of the aforementioned BMP4.

As the BMP signal transduction pathway activating substance, a compound well known to those of ordinary skill in the art can also be used. Examples of the BMP signal transduction pathway activating substance include Smurf1 inhibiting substance, Chk1 inhibiting substance, phosphorylation Smad stabilizing substance, and the like. Examples of the compound having the above-mentioned activity include A-01 ([4-[[4-Chloro-3-(trifluoromethyl)phenyl]sulfonyl]-1-piperazinyl][4-(5-methyl-1H-pyrazol-1-yl)phenyl]methanone), PD407824 (9-Hydroxy-4-phenyl-pyrrolo[3,4-c]carbazole-1,3(2H,6H)-dione), SB4 (2-[[(4-Bromophenyl)methyl]thio]benzoxazole), SJ000291942 (2-(4-Ethylphenoxy)-N-(4-fluoro-3-nitrophenyl)-acetamide) and derivatives of these, and the like.

As the Shh signal transduction pathway activating substance used in step (2), one similar to those exemplified in step (a) can be used. The Shh signal transduction pathway activating substances in step (a) and step (2), and the Shh signal transduction pathway activating substance in step (1) in some cases, may be the same or different, preferably the same, and is preferably SAG.

The concentration of Shh signal transduction pathway activating substance in the medium can be appropriately determined according to the substances used within a range capable of achieving the aforementioned effects. When SAG is used as the Shh signal transduction pathway activating substance in step (2), it is generally used at a concentration of about 1 nM to about 5 µM, preferably about 10 nM to about 4.5 µM, more preferably about 50 nM to about 4 µM, further preferably about 100 nM to about 3 µM.

The medium used in step (2) is not particularly limited as long as it contains a Shh signal transduction pathway activating substance and a BMP signal transduction pathway activating substance. The medium to be used in step (2) includes, for example, the media listed in the first step.

From the aspect of improving the production efficiency of the pituitary, the timing of starting step (2) is preferably from 0.5 hr to 6 days, more preferably 0.5 hr to 72 hr, further preferably 24 hr to 60 hr, after the start of culture in the first step. When performing suspension culturing, when step (2) is started during the above-mentioned period in the presence of a Wnt signal pathway inhibiting substance, a nonneural epithelial-like tissue is formed on the surface of the cell aggregate, and the pituitary can be formed extremely efficiently.

When suspension culturing is performed in the first step, the timing of starting step (2) from the aspect of improving the production efficiency of the cells constituting the pituitary is a period when 100 or more, more preferably 30% or more, further preferably 50% or more, of cells in the surface layer of the cell aggregate formed in the first step form tight junctions with each other. Those of ordinary skill in the art can easily determine whether tight junctions are formed in cell aggregates by, for example, microscopic observation or methods such as immunostaining using an anti-ZO-1 antibody, and the like.

To start the culture in the presence of a BMP signal transduction pathway activating substance in step (2), the above-mentioned medium exchange operation (e.g., medium addition operation, half-volume medium exchange operation, full-volume medium exchange operation, etc.) may be performed using the culture vessel in which the first step has been performed, or the cells may be transferred to another culture vessel.

The period of culturing in the medium containing the BMP signal transduction pathway activating substance in step (2) can be set as appropriate. The culture period in step (2) is generally not less than 8 hr, preferably not less than 10 hr, more preferably not less than 12 hr, further preferably not less than 14 hr, most preferably not less than 16 hr.

The period of culturing in the medium containing the Shh signal transduction pathway activating substance in step (2) can be set as appropriate. When the suspension culturing in step (1) is performed further in the presence of a Shh signal transduction pathway activating substance, the culture period in the presence of the Shh signal transduction pathway activating substance in step (1) and step (2) is preferably 30 days, from the aspect of improving the pituitary hormone (particularly ACTH) secretory capacity.

In step (2) and the steps thereafter, it is also preferable to add an FGF signal transduction pathway activating substance to the culture environment from the aspect of promoting differentiation into pituitary placode. The FGF signal transduction pathway activating substance is not particularly limited as long as it is a substance capable of enhancing the signal transduction pathway mediated by FGF (fibroblast growth factor). Examples of the FGF signal transduction pathway activating substance include FGF proteins such as FGF1, FGF2 (sometimes referred to as bFGF), FGF3, FGF8, FGF10, and the like, anti-FGF receptor antibody, FGF partial peptide and the like. These substances may be used alone or in combination.

FGF2 protein and FGF8 protein are available from, for example, FUJIFILM Wako Pure Chemical Corporation. The FGF signal transduction pathway activating substance preferably contains at least one selected from the group consisting of FGF2, FGF3, FGF8 and FGF10, and variant thereof, more preferably contains FGF2, further preferably contains recombinant human FGF2.

The concentration of FGF signal transduction pathway activating substance in the medium can be appropriately determined according to the substances used within a range capable of achieving the aforementioned effects. From the aspects of differentiation into cells constituting the pituitary, and promotion of differentiation and survival and proliferation of the cell, when FGF2 is used as the FGF signal transduction pathway activating substance, it is used at a concentration of generally about 1 pg/ml to about 100 µg/ml, preferably about 10 pg/ml to about 50 µg/ml, more preferably about 100 pg/ml to about 10 µg/ml, further preferably about 500 pg/ml to about 1 µg/ml, most preferably about 1 ng/ml to about 200 ng/ml. When an FGF signal transduction pathway activating substance other than FGF2 is used, it is desirably used at a concentration showing FGF signal transduction pathway promoting activity equivalent to that of FGF2 at the above-mentioned concentration. The FGF signal transduction pathway promoting activity of a substance to be added can be measured by, for example, a method such as cell proliferation test using 3T3 cell and the like.

To retain the activity of FGF protein in a medium, it is also preferable to add heparin, heparan sulfate to the medium containing the FGF protein. Heparin is available as a sodium salt from, for example, FUJIFILM Wako Pure Chemical Corporation. The concentration of heparin or heparan sulfate in the medium can be appropriately determined to fall within a range capable of achieving the aforementioned effects. The concentration of heparin sodium in the medium is generally about 1 ng/ml to about 100 mg/ml, preferably about 10 ng/ml to about 50 mg/ml, more preferably about 100 ng/ml to about 10 mg/ml, further preferably about 500 ng/ml to about 1 mg/ml, most preferably about 1 µg/ml to about 200 ug/ml. When heparan sulfate is used, it is preferably a concentration showing FGF protein protecting activity equivalent to that of heparin at the above-mentioned concentration. To retain the activity of FGF protein under a cell culture environment such as 37°C, for example, it is also preferable to use modified FGF such as Thermostable FGF2 described in US8772460B2 or FGF2 sustained-release beads such as StemBeads FGF2 in which FGF2 is bound to a biodegradable polymer. Thermostable FGF2 is available from, for example, HumanZyme, inc. StemBeads FGF2 is available from, for example, StemCulture.

The timing of adding the FGF signal transduction pathway activating substance is added in step (2) and the steps thereafter can be set as appropriate. In preferred one embodiment, an FGF signal transduction pathway activating substance is added 6 hr, more preferably 12 hr, further preferably 18 hr, after the addition of the BMP signal transduction pathway activating substance in step (2).

It is also preferable to continuously add in step (2) the additives used in step (a) or the first step, such as JNK signal transduction pathway inhibiting substance, Wnt signal transduction pathway inhibiting substance, TGFβ signal transduction pathway inhibiting substance, TAK1 inhibiting substance, and the like. The JNK signal transduction pathway inhibiting substance, Wnt signal transduction pathway inhibiting substance, or TGFβ signal transduction pathway inhibiting substance to be added in step (2) may be different from the substance used in the previous steps but preferably the same. The concentration and kind of the additive can be appropriately adjusted. These substances may be added at the same time as the start of step (2) or at different times.

### <Step (b)>: b step

In step (b), the cell population obtained in step (2) is cultured under addition conditions of a BMP signal transduction pathway inhibiting substance. When cells are suspension cultured in step (2), the cell aggregates formed may be continuously suspension cultured also in step (b). When cells are adhesion cultured in step (2), the cells may be continuously adhesion cultured also in step (b).

The BMP signal transduction pathway inhibiting substance is not limited as long as it can suppress signal transduction induced by BMP family proteins. It may be any of nucleic acid, protein and low-molecular organic compound. Examples of the substance include a substance that inhibits BMP processing and extracellular secretion, a substance that directly acts on BMP (e.g., protein, antibody, and aptamer), a substance that suppresses expression of a gene encoding BMP (e.g., antisense oligonucleotide, and siRNA), a substance that suppresses binding of BMP receptor and BMP, and a substance that suppresses physiological activity caused by signal transduction by BMP receptor. The BMP receptor includes type I BMP receptor and type II BMP receptor. As the type I BMP receptor, BMPR1A, BMPR1B, and ACVR are known; as the type II BMP receptor, TGF-beta R-II, ActR-II, ActR-IIB, BMPR2, and MISR-II are known.

As a protein known as a BMP signal transduction pathway inhibiting substance, for example, Noggin, Chordin, Follistatin, Gremlin, Inhibin, Twisted Gastrulation, Coco, secretory protein belonging to the DAN family can be mentioned. In the above-mentioned step (2), a BMP signal transduction pathway activating substance is added to the culture medium. To more effectively inhibit the BMP signal transduction pathway thereafter, the BMP signal transduction pathway inhibiting substance in step (b) preferably contains a substance that inhibits a signal transduction pathway after the extracellular secretion of BMP, for example, a substance that inhibits the binding between BMP receptor and BMP, a substance that inhibits physiological activity caused by signal transduction by BMP receptors, more preferably an inhibitor of type I BMP receptor.

As the BMP signal transduction pathway inhibiting substance, a compound well known to those of ordinary skill in the art can also be used. Examples of the BMP signal transduction pathway inhibiting substance include an inhibiting substance of BMP type I receptor. Examples of the compound having the above-mentioned activity include K02288 (3-[(6-Amino-5-(3,4,5-trimethoxyphenyl)-3-pyridinyl]phenol), Dorsomorphin (6-[4-[2-(1-Piperidinyl)ethoxy]phenyl]-3-(4-pyridinyl)pyrazolo[1,5-a]pyrimidine), LDN-193189 (4-[6-[4-(1-Piperazinyl)phenyl]pyrazolo[1,5-a]pyrimidin-3-yl]quinoline dihydrochloride), LDN-212854 (5-[6-[4-(1-Piperazinyl)phenyl]pyrazolo[1,5-a]pyriMidin-3-yl]quinoline), LDN-214117 (1-(4-(6-methyl-5-(3,4,5-trimethoxyphenyl)pyridin-3-yl)phenyl)piperazine), ML347(5-[6-(4-Methoxyphenyl)pyrazolo[1,5-a]pyrimidin-3-yl]quinoline)), DMH1 (4-(6-(4-Isopropoxyphenyl)pyrazolo[1,5-a]pyrimidin-3-yl)quinoline), DMH2 (4-[6-[4-[2-(4-Morpholinyl)ethoxy]phenyl]pyrazolo[1,5-a]pyrimidin-3-yl]-quinoline), compound 1 (3-(1,2,3-benzothiadiazol-6-yl)-1-[2-(cyclohex-1-en-1-yl)ethyl]urea), VU0465350 (7-(4-isopropoxyphenyl)-3-(1H-pyrazol-4-yl)imidazo[1,2-a]pyridine), VU0469381 (5-(6-(4-methoxyphenyl)pyrazolo[1,5-a]pyrimidin-3-yl)quinolone), OD36 (4-chloro-7,10-dioxa-13,17,18,21-tetrazatetracyclo[12.5.2.12,6.017,20]docosa-1(20),2(22),3,5,14(21),15,18-heptaene), OD52, E6201 ((3S,4R,5Z,8S,9S,11E)-14-(ethylamino)-8,9,16-trihydroxy-3,4-dimethyl-3,4,9,10-tetrahydro-1H-benzo[c][1]oxacyclotetradecine-1,7(8H)-dione), Saracatinib (N-(5-chloro-1,3-benzodioxol-4-yl)-7-[2-(4-methylpiperazin-1-yl)ethoxy]-5-(oxan-4-yloxy)quinazolin-4-amine), BYL719 ((2S)-1-N-[4-methyl-5-[2-(1,1,1-trifluoro-2-methylpropan-2-yl)pyridin-4-yl]-1,3-thiazol-2-yl]pyrrolidine-1,2-dicarboxamide), and the like. These substances may be used alone or in combination.

The BMP signal transduction pathway inhibiting substance is preferably BMP type I receptor inhibitor, more preferably contains at least one selected from the group consisting of K02288, Dorsomorphin, LDN-193189, LDN-212854, LDN-214117, ML347, DMH1, and DMH2, further preferably contains K02288 to LDN-193189.

The concentration of the BMP signal transduction pathway inhibiting substance in the medium can be appropriately determined to fall within a range capable of achieving the aforementioned effects and according to the substances to be used. From the aspect of pituitary tissue formation efficiency, when K02288 is used as a BMP signal transduction pathway inhibiting substance in step (b), it is used at a concentration of generally about 1 nM to about 100 µM, preferably about 10 nM to about 50 µM, more preferably about 100 nM to about 50 µM, further preferably about 500 nM to about 25 µM. When LDN-193189 is used as a BMP signal transduction pathway inhibiting substance, it is used at a concentration of generally about 1 nM to about 100 µM, preferably about 10 nM to about 10 µM, more preferably about 25 nM to about 1 µM, further preferably about 100 nM to about 500 nM. When LDN-212854 is used as a BMP signal transduction pathway inhibiting substance, it is used at a concentration of generally about 1 nM to about 100 µM, preferably about 10 nM to about 10 µM, more preferably about 25 nM to about 5 µM, further preferably about 250 nM to about 3 µM. When ML347 is used as a BMP signal transduction pathway inhibiting substance, it is used at a concentration of generally about 1 nM to about 100 µM, preferably about 10 nM to about 50 µM, more preferably about 100 nM to about 50 µM, further preferably about 1 µM to about 25 µM. When DMH2 is used as a BMP signal transduction pathway inhibiting substance, it is used at a concentration of generally about 1 nM to about 100 µM, preferably about 10 nM to about 10 µM, more preferably about 25 nM to about 5 µM, further preferably about 250 nM to about 3 µM. When a BMP signal transduction pathway inhibiting substance other than K02288 is used, it is desirably used at a concentration showing BMP signal transduction pathway inhibitory activity equivalent to that of K02288 at the above-mentioned concentration.

The timing of starting step (b) after step (2) can be set as appropriate. The timing of starting step (b) is generally not less than 8 hr and within 15 days, preferably not less than 10 hr and within 12 days, more preferably not less than 12 hr and within 9 days, further preferably not less than 14 hr and within 8 days, most preferably not less than 16 hr and within 7 days, from the start of step (2).

In step (2) and the steps thereafter, the cell population may be treated with corticosteroids by adding corticosteroids to the medium. The treatment with corticosteroids promotes differentiation of pituitary placode and/or Rathke's pouch into pituitary hormone-producing cells other than ACTH-producing cells (i.e., GH-producing cells, PRL-producing cells, TSH-producing cells, LH-producing cells, FSH-producing cells, etc.). Examples of the corticosteroids include, but are not limited to, natural glucocorticoids such as hydrocortisone, cortisone acetate, fludrocortisone acetate, and the like; artificially-synthesized glucocorticoids such as dexamethasone, betamethasone, predonisolone, methylprednisolone, triamcinolone, and the like, and the like.

The concentration of corticosteroids in the medium is not particularly limited as long as it can promote differentiation of pituitary placode and/or Rathke's pouch into pituitary hormone-producing cells (excluding ACTH-producing cells). It can be appropriately determined according to the kind of the corticosteroids. For example, in the case of hydrocortisone, it is generally 100 ng/ml or more, preferably 1 µg/ml or more. The upper limit of the hydrocortisone concentration is not particularly set as long as differentiation into pituitary hormone-producing cells (excluding ACTH-producing cells) is not adversely affected. From the aspect of culture cost, it is generally 1000 µg/ml or less, preferably 100 µg/ml or less. In one embodiment, the concentration of hydrocortisone in the medium is generally about 100 ng/ml to about 1000 µg/ml, preferably about 1 to about 100 µg/ml. When dexamethasone is used as the corticosteroids, the concentration thereof in the medium can be set to about 1/25 that of hydrocortisone.

In step (2) and the steps thereafter, the timing of adding corticosteroids to the medium is not particularly limited as long as it can promote differentiation of pituitary placode and/or Rathke's pouch into pituitary hormone-producing cells (excluding ACTH-producing cells). Corticosteroids may be added to the medium from the start of the second step, or corticosteroids may be added to the medium after culturing for a certain period of time in a medium free of addition of corticosteroids after the start of the second step. Preferably, after the start of the second step, corticosteroids are added to the medium when the emergence of ACTH-producing cells is confirmed in the cell population. That is, the cell aggregates are cultured in a medium free of addition of corticosteroids until the emergence of ACTH-producing cells is confirmed in the cell aggregates, and the step (2) and the steps thereafter are continued in a medium containing corticosteroids, after the emergence of ACTH-producing cells is confirmed. The emergence of ACTH-producing cells can be confirmed by immunohistological staining using an antibody against ACTH. When human pluripotent stem cells are used, the emergence of ACTH-producing cells can be generally expected after 30 days from the start of the first step. In one embodiment, therefore, corticosteroids are added to the medium after 30 days from the start of the first step.

The period of treatment of cell aggregates with corticosteroids is not particularly limited as long as it can promote differentiation of pituitary placode and/or Rathke's pouch into pituitary hormone-producing cells (excluding ACTH-producing cells). In general, cell aggregates are treated with corticosteroids until promotion of differentiation into pituitary hormone-producing cells (excluding ACTH-producing cells) is confirmed in the corticosteroid treatment group as compared with a corticosteroid non-treatment group. The treatment period is generally 7 days or more, preferably 12 days or more. The upper limit of the treatment period is not particularly set and the corticosteroids may be removed from the medium at the stage when promotion of differentiation into pituitary hormone-producing cells (excluding ACTH-producing cells) is confirmed in the corticosteroid treatment group as compared with the corticosteroid non-treatment group.

Step (2) and the steps thereafter are also preferably performed in the presence of a retinoic acid signal transduction pathway activating substance, from the aspect of promoting differentiation into pituitary and differentiation into growth hormone-producing cells. Examples of the retinoic acid transduction pathway activating substance include substances that bind to retinoic acid receptor (RAR) or retinoid X receptor (RXR) and activate transcription in the downstream and the like. Examples of the compound having the above-mentioned action include all-trans-retinoic acid, isotretinoin, 9-cis retinoic acid, TTNPB (4-[(E)-2-[(5,5,8,8-Tetramethyl-5,6,7,8-tetrahydronaphthalene)-2-yl]-1-propenyl]benzoic acid), Ch55 (4-[(E)-3-(3,5-di-tert-butylphenyl)-3-oxo-1-propenyl]benzoic acid), EC19 (3-[2-(5,6,7,8-Tetrahydro-5,5,8,8-tetramethyl-2-naphthalenyl)ethynyl]benzoic acid), EC23 (4-[2-(5,6,7,8-Tetrahydro-5,5,8,8-tetramethyl-2-naphthalenyl)ethynyl]benzoic acid), Fenretinide (4-hydroxyphenylretinamide), Acitretin ((all-e)-9-(4-methoxy-2,3,6-trimethylphenyl)-3,7-dimethyl-2,4,6,8-nonatetraen), Trifarotene, Adapalene, AC 261066 (4-[4-(2-Butoxyethoxy-)-5-methyl-2-thiazolyl]-2-fluorobenzoic acid), AC 55649 (4-N-Octylbiphenyl-4-carboxylic acid), AM 580 (4-[(5,6,7,8-Tetrahydro-5,5,8,8-tetramethyl-2-naphthalenyl)carboxamido]benzoic acid), AM80 (4-[(5,5,8,8-Tetramethyl-6,7-dihydronaphthalen-2-yl)carbamoyl]benzoic acid), BMS 753 (4-[[(2,3-Dihydro-1,1,3,3-tetramethyl-2-oxo-1H-inden-5-yl)carbonyl]amino]benzoic acid), BMS 961 (3-Fluoro-4-[(r)-2-hydroxy-2-(5,5,8,8-tetramethyl-5,6,7,8-tetrahydro-naphthalen-2-yl)-acetylamino]-benzoic acid), CD1530 (4-(6-Hydroxy-7-tricyclo[3.3.1.13,7]dec-1-yl-2-naphthalenyl)benzoic acid), CD2314 (5-(5,6,7,8-Tetrahydro-5,5,8,8-tetramethyl-2-anthracenyl)-3-thiophenecarboxylic acid), CD437 (2-naphthalenecarboxylic acid,6-(4-hydroxy-3-tricyclo(3.3.1.1(3,7))dec-1-ylphen), CD271 (6-[3-(1-Adamantyl)-4-methoxyphenyl]-2-naphthalene carboxylic acid) and derivatives of these, and the like. These substances may be used alone or in combination.

The retinoic acid transduction pathway activating substance in step (2) and the steps thereafter preferably contains all-trans-retinoic acid or EC23. The concentration of retinoic acid transduction pathway activating substance in the medium is not particularly limited as long as the aforementioned effects can be achieved. When EC23 is used as the retinoic acid transduction pathway activating substance, for example, the concentration of EC23 is about 10 pM to about 30 µM, preferably about 100 pM to about 20 µM, more preferably about 10 nM to about 10 µM, further preferably about 100 nM to about 5 µM. When a retinoic acid transduction pathway activating substance other than EC23 is used, it is desirably used at a concentration showing retinoic acid transduction pathway action activity equivalent to that of EC23 at the above-mentioned concentration.

From the aspect of regulating the differentiation tendency, it is also preferable to perform step (2) and the steps thereafter in the presence of a Notch signal transduction pathway inhibiting substance. In the present invention, the Notch signal transduction pathway shows a signal transduction pathway activated by direct interaction between Notch protein, which is a receptor expressed on a cell membrane, and Notch ligand (Delta, Jagged, etc.) expressed on the membrane of adjacent cells. In cells in which a Notch signal is transmitted, the Notch protein is stepwisely processed and the intracellular domain excised on the membrane is transported into the nucleus and controls the expression of downstream genes.

The Notch signal transduction pathway inhibiting substance is not particularly limited as long as it can suppress signal transduction mediated by Notch. It may be any of nucleic acid, protein and low-molecular organic compound. Examples of the substance include functionally deficient Notch receptor and ligand, substances that inhibit Notch processing (S1 cleavage), substances that inhibit sugar chain modification of Notch and Notch ligand, substances that inhibit cell membrane transfer, substances (γ-secretase inhibitors) that inhibit processing (S2 cleavage, S3 cleavage) of Notch intracellular domain (NICD), substances that decompose NICD, substances that inhibit NICD-dependent transcription, and the like.

As the Notch signal transduction pathway inhibiting substance, a compound well known to those of ordinary skill in the art can also be used. As a compound having the activity as a Notch signal transduction pathway inhibiting substance, for example, DAPT (N-[N-(3,5-difluorophenacetyl)-1-alanyl]-S-phenylglycine t-butyl ester), DBZ ((2S)-2-[[2-(3,5-difluorophenyl)acetyl]amino]-N-[(7S)-5-methyl-6-oxo-7H-benzo[d][1]benzazepin-7-yl]propanamide), MDL28170 (benzyl N-[(2S)-3-methyl-1-oxo-1-[[(2S)-1-oxo-3-phenylpropan-2-yl]amino]butan-2-yl]carbamate), FLI-06 (cyclohexyl 2,7,7-trimethyl-4-(4-nitrophenyl)-5-oxo-1,4,6,8-tetrahydroquinoline-3-carboxylate), L-685,458 (tert-butyl N-[6-[[1-[(1-amino-1-oxo-3-phenylpropan-2-yl)amino]-4-methyl-1-oxopentan-2-yl]amino]-5-benzyl-3-hydroxy-6-oxo-1-phenylhexan-2-yl]carbamate), CB-103 (6-(4-tert-butylphenoxy)pyridin-3-amine) and derivatives of these, the substance described in Onco Targets Ther.2013; 6:943-955, and the like can be mentioned. The Notch signal transduction pathway inhibiting substance preferably contains DAPT.

The concentration of the Notch signal transduction pathway inhibiting substance in the medium is not particularly limited as long as it is within a range capable of achieving the aforementioned effects. For example, when DAPT is used as the Notch signal transduction pathway inhibiting substance, for example, the concentration of DAPT is about 100 pM to about 50 µM, preferably about 1 nM to about 30 µM, more preferably about 100 nM to about 20 µM, further preferably about 1 pM to about 10 pM. When a Notch signal transduction pathway inhibiting substance other than DAPT is used, it is desirably used at a concentration showing Notch signal transduction pathway inhibitory activity equivalent to that of DAPT at the above-mentioned concentration.

### <Step (3)>, <step (3')>: the third step

In the third step, the cell population cultured in step (2) or step (b) is cultured under non-addition conditions of a Sonic hedgehog signal (Shh) transduction pathway activating substance. The third step in which the cell population cultured in the second step is cultured in the absence of a Shh signal transduction pathway activating substance to obtain a cell population containing pituitary tissue is step (3), and the third step in which the cell population cultured in step (b) is cultured in the absence of a Shh signal transduction pathway activating substance to obtain a cell population containing pituitary tissue is step (3'). When cells are suspension cultured in step (2) or step (b), the cell aggregates formed may be suspension cultured in the third step. When cells are adhesion cultured in step (2) or step (b), the cells may be continuously adhesion cultured also in the third step. After the cells are suspension cultured in step (2) or step (b), they may be adhesion cultured in the third step.

The medium used in the third step is not particularly limited as long as it does not contain a Shh signal transduction pathway activating substance. The non-addition condition of a Shh signal transduction pathway activating substance in this step refers to a condition in which a Shh signal transduction pathway activating substance is not intentionally added to the culture environment of the cell population, and the non-addition condition of a Shh signal transduction pathway activating substance includes even when a Shh signal transduction pathway activating substance is unintentionally included in the culture environment due to autocrine secretion by a cell population. Examples of the medium used in the third step include the medium listed in the first step, the gfCDM medium containing 10% to 20% KSR, and the like.

The third step and the steps thereafter may include a step of embedding and culturing the cell aggregate in a gel, from the aspect of promoting survival and differentiation maturation of cells constituting the pituitary. Examples of the gel include gels using agarose, methylcellulose, collagen, Matrigel, and the like, and it is preferable to use Matrigel.

In the step of embedding and culturing the cell in a gel in the third step and the steps thereafter, the cell aggregate may be embedded as it is or the cells after dispersion and isolation may be seeded in the gel. The cells may be seeded after sorting out specific cell tumors such as basal cell by using a cell sorter or the like. As a further embodiment of the culture method of embedding in a gel, co-culture with cells other than the pituitary such as fibroblast, mesenchymal cell, vascular cell, and the like can also be performed. Gel-embedding culture as described above can be performed with reference to, for example, Nature 501, 373-379 (2013), Nature, 499, 481-484 (2013), Nat Protoc 14, 518-540 (2019), Genes 2020, 11, 603, and the like.

In the third step and the steps thereafter, it is also preferable to perform a culture method in which the cells are physically shaken, for the purpose of improving nutrition and oxygen supply to the cells and improving substance exchange. Examples of such culture method include methods other than static culture, such as shaking culture, rotation culture, stirring culture, and the like. The means for performing shaking culture, rotation culture, stirring culture, and the like are not particularly limited. For example, they can be performed by placing the culture vessel, in which the cells are cultured, on a rotator, shaker, or the like, or by placing the cells in an environment where a stirrer or the like is rotating. Those skilled in the art can appropriately set the parameters such as the speed and the like of shaking culture, rotation culture, and stirring culture within a range that does not cause damage to cells. For example, when shaking culture is performed using a waveform fluctuation 3D shaker (e.g., Mini-Shaker 3D, manufactured by Biosan), for example, the shaking speed range can be set within the range of 5 to 60 rpm, preferably 5 to 40 rpm, more preferably 5 to 20 rpm. When shaking culture is performed using a reciprocating type shaker (e.g., NS-LR, manufactured by AS ONE Corporation), for example, the shaking speed range can be set within the range of 15 to 60 rpm, preferably 15 to 50 rpm, more preferably 15 to 45 rpm. When shaking culture is performed using a seesaw type shaker (e.g., NS-S, manufactured by AS ONE Corporation), for example, the shaking speed range can be set within the range of 5 to 50 rpm, preferably 5 to 40 rpm, more preferably 5 to 30 rpm. When cell aggregates are cultured by shaking culture or rotation culture, for example, a spinner flask (e.g., 3152, manufactured by Corning Incorporated) is set on a magnetic stirrer, and culture can also be performed at a rotation speed at which cell aggregates do not form sediment visually. Culture can also be performed using a three-dimensional rotating suspension culture device (e.g., CellPet CUBE, manufactured by JTEC Corporation; Clinostar, manufactured by Celvivo). From the aspect of suppressing physical damage such as friction to cells and the like, it is also preferable to culture the aforementioned cell aggregates embedded in the gel by shaking culture, rotation culture, stirring culture.

In the third step and the steps thereafter, it is also preferable to perform culturing under a high oxygen atmosphere from the aspect of suppression of cell death and promotion cell proliferation. The high oxygen conditions in the culturing process can be realized, for example, by connecting an oxygen cylinder to an incubator for culturing cells and artificially supplying oxygen. The oxygen concentration for such purpose is generally 25% to 80%, more preferably 30% to 60%.

In the third step and the steps thereafter, it is possible to use a culture vessel with high gas exchange efficiency from the aspect of increasing the amount of oxygen supply to the medium for culturing cell aggregates. Examples of such culture vessel include cell culture dish, Lumoxdish with a gas permeable film as bottom surface of the plate (manufactured by Sarstedt K.K.), VECELL 96 well plate (manufactured by Vessel CO. LTD.) and the like. It is also preferable to use same in combination with culture under the aforementioned high oxygen concentration conditions.

In the third step and the steps thereafter, from the aspect of maintaining the structure of epithelial tissue in the cell aggregate, a cell protectant can also be added to the medium. As the cell protectant to be used in the third step and the steps thereafter, the aforementioned FGF signal transduction pathway activating substance, heparin, ROCK inhibiting substance, basement membrane preparation, myosin inhibiting substance, polyamines, ISR inhibitor, caspase inhibitor, serum, serum replacement, and the like can be mentioned. As the myosin inhibiting substance, for example, Blebbistatin as an inhibiting substance of nonmuscle myosin II ATPase, ML-7, ML-9, and W-7 as inhibiting substance of myosin light chain kinase (MLCK), MLCK inhibitor peptide 18, derivatives of these, and the like can be mentioned. The cell protectant to be added may be different from the one added in the first step, but is preferably the same. As a preferred cell protectant, ROCK inhibiting substance can be mentioned. In the third step and the steps thereafter, when Y-27632, which is a ROCK inhibiting substance, is added as a cell protectant, it is added into the culture environment to achieve a concentration of generally about 10 nM to about 10 mM, preferably about 100 nM to about 1 mM, more preferably about 1 pM to about 100 pM. When Chroman 1, which is a ROCK inhibiting substance, is added, it is added into the culture environment to achieve a concentration of generally about 10 pM to about 1 mM, preferably about 100 pM to about 100 µM, more preferably about 1 nM to about 10 pM. When Blebbistatin, which is an inhibiting substance of nonmuscle myosin II ATPase, is added as a cell protectant, it is added into the culture environment to achieve a concentration of generally about 10 nM to about 10 mM, preferably about 100 nM to about 1 mM, more preferably about 1 pM to about 100 pM.

In the third step and the steps thereafter, a substance other than cell protectant that has the action of maintaining the structure of nonneural epithelial tissue can also be added. Examples of the above-mentioned substance include substances that promote cell adhesion, substances that promote the synthesis of base membrane components, substances that inhibit the decomposition of base membrane components, and the like. The substances that promote cell adhesion may be those that promote any of cell-to-cell adhesion, cell-to-base membrane adhesion, cell-to-culture vessel adhesion, and the like or the production of factors involved in cell adhesion. For example, as the substances that promote cell adhesion, Adhesamine, Adhesamine-RGDS derivative, Pyrintegrin, Biotin tripeptide-1, Acetyl Tetrapeptide-3, RGDS Peptide and derivatives of these, and the like can be mentioned. For example, as the substances that promote synthesis of base membrane components, ascorbic acid derivative and the like can be mentioned. Examples of the ascorbic acid derivative include sodium ascorbate phosphate, magnesium ascorbate phosphate, ascorbic acid 2-glucoside, 3-O-ethyl ascorbic acid, tetrahexyldecanic acid ascorbyl, ascorbyl palmitate, ascorbyl stearate, ascorbyl 2-phosphate 6-palmitate, glyceryl octyl ascorbic acid, and the like. As the substances that inhibit decomposition of the base membrane components, for example, inhibitors of matrix metalloprotease and serine protease and the like can be mentioned. When ascorbic acid 2-phosphate, which is one kind of ascorbic acid derivative which is a substance that promotes synthesis of base membrane components, is added, it is added into the culture environment to achieve a concentration of generally not less than 10 µg/ml and not more than 1000 µg/ml, preferably not less than 30 µg/ml and not more than 500 µg/ml, further preferably not less than 50 µg/ml and not more than 300 ug/ml. When other ascorbic acid, ascorbic acid derivatives, and the like are added, they may be added so that their molar equivalents are approximately the same as the above-mentioned concentrations.

In the third step and the steps thereafter, from the aspect of promoting the survival of pituitary cells, it is also preferable to add a substance having an action of reducing oxidative stress. As substances with the above-mentioned activity, for example, antioxidants, substances with free radical scavenging action, NADPH oxidase inhibiting substances, cyclooxygenase inhibiting substance, lipoxygenase (LOX) inhibiting substance, superoxide dismutase (SOD)-like substances, Nrf2 activator, and the like can be mentioned. Examples of the substance having the above-mentioned activity include, but are not limited to, ascorbic acid, N-acetyl-L-cysteine, (±)-α-tocopherol acetate, Apocynin (4'-Hydroxy-3'-methoxyacetophenone), nicotine amide, taurine (2-aminoethanesulfonic acid), IM-93 (1-Isopropyl-3-(1-methyl-1H-Indol-3-yl)-4-(N,N-dimethyl-1,3-propanediamine)-1H-Pyrrole-2, 5H-dione), Caffeic Acid (3,4-Dihydroxycinnamic Acid), Celastrol (3-Hydroxy-24-nor-2-oxo-1(10),3,5,7-friedelatetraen-29-oic Acid; Tripterin), Ebselen (2-Phenyl-1,2-benzisoselenazol-3(2H)-one), (-)-Epigallocatechin Gallate((2R,3R)-2-(3,4,5-Trihydroxyphenyl)-3,4-dihydro-1[2H]-benzopyran-3,5,7-triol-3-(3,4,5-trihydroxybenzoate)), EUK-8 (N,N'-Bis(salicylideneamino)ethane-manganese(II)), Edaravone (3-Methyl-1-phenyl-2-pyrazolin-5-one), MnTBAP (Mn(III)tetrakis(4-benzoic acid)porphyrin Chloride), Nordihydroguaiaretic Acid, Resveratrol (trans-3,4,5-Trihydroxystilbene) and derivatives of these, and the like. Reagents prepared for cell culture (e.g., antioxidant supplement, manufactured by Sigma Aldrich, A1345) can also be used. The substance having an action of reducing oxidative stress to be used in the present invention preferably contains at least one selected from the group consisting of ascorbic acid, N-acetyl-L-cysteine, and derivatives of these. Ascorbic acid can be added to the medium at a concentration of, for example, as ascorbic acid 2 phosphate (derivative thereof), about 1 nM to about 1 M, preferably about 10 nM to about 100 mM, more preferably about 100 nM to about 10 mM, further preferably about 1 pM to about 3 mM, and N-acetyl-L-cysteine can be added to the medium at a concentration of, for example, about 1 nM to about 1 M, preferably about 10 nM to about 100 mM, more preferably about 100 nM to about 10 mM, further preferably about 1 µM to about 5 mM.

In the third step and the steps thereafter, from the aspect of promoting survival of the pituitary cell, it is also preferable to add a substance that inhibits stress-response signal transduction pathway (substance that inhibits intracellular signal transduction mechanism for the stress). The stress-responsive MAP kinase pathway (stress-activated protein kinase: SAPK) is one of the major intracellular signal transduction mechanisms against stress. As inhibitors of stress-responsive MAP kinase pathway, for example, MAP3K inhibitor, MAP2K inhibitor, ASK inhibitor, MEK inhibitor, Akt inhibitor, Rho family kinase inhibitor, JNK inhibitor, p38 inhibitor, MSK inhibitor, STAT inhibitor, NF-κB inhibitor, CAMK inhibitor and the like can be mentioned.

Examples of the MEK inhibitor include Selumetinib (AZD6244, 6-(4-bromo-2-chloroanilino)-7-fluoro-N-(2-hydroxyethoxy)-3-methylbenzimidazole-5-carboxamide), Mirdametinib (PD0325901, N-[(2R)-2,3-dihydroxypropoxy]-3,4-difluoro-2-(2-fluoro-4-iodoanilino)benzamide), Trametinib (GSK1120212, N-[3-[3-cyclopropyl-5-(2-fluoro-4-iodoanilino)-6,8-dimethyl-2,4,7-trioxopyrido[4,3-d]pyrimidin-1-yl]phenyl]acetamide), U0126 (1,4-diamino-2,3-dicyano-1,4-bis(2-aminophenylthio)butadiene), PD184352 (CI-1040, 2-(2-chloro-4-iodoanilino)-N-(cyclopropylmethoxy)-3,4-difluorobenzamide), PD98059 (2-(2-amino-3-methoxyphenyl)chromen-4-one), BIX 02189 (3-[N-[3-[(dimethylamino)methyl]phenyl]-C-phenylcarbonimidoyl]-2-hydroxy-N,N-dimethyl-1H-indole-6-carboxamide), Pimasertib (AS-703026, N-[(2S)-2,3-dihydroxypropyl]-3-(2-fluoro-4-iodoanilino)pyridine-4-carboxamide), Pelitinib (EKB-569, (E)-N-[4-(3-chloro-4-fluoroanilino)-3-cyano-7-ethoxyquinolin-6-yl]-4-(dimethylamino)but-2-enamide), BIX 02188 (3-[N-[3-[(dimethylamino)methyl]phenyl]-C-phenylcarbonimidoyl]-2-hydroxy-1H-indole-6-carboxamide), TAK-733 (3-[(2R)-2,3-dihydroxypropyl]-6-fluoro-5-(2-fluoro-4-iodoanilino)-8-methylpyrido[2,3-d]pyrimidine-4,7-dione), AZD8330 (2-(2-fluoro-4-iodoanilino)-N-(2-hydroxyethoxy)-1,5-dimethyl-6-oxopyridine-3-carboxamide), Binimetinib (MEK162, 6-(4-bromo-2-fluoroanilino)-7-fluoro-N-(2-hydroxyethoxy)-3-methylbenzimidazole-5-carboxamide), SL-327 ((Z)-3-amino-3-(4-aminophenyl)sulfanyl-2-[2-(trifluoromethyl)phenyl]prop-2-enenitrile), Refametinib (RDEA119, N-[3,4-difluoro-2-(2-fluoro-4-iodoanilino)-6-methoxyphenyl]-1-[(2S)-2,3-dihydroxypropyl]cyclopropane-1-sulfonamide), GDC-0623 (5-(2-fluoro-4-iodoanilino)-N-(2-hydroxyethoxy)imidazo[1,5-a]pyridine-6-carboxamide), BI-847325 (3-[3-[N-[4-[(dimethylamino)methyl]phenyl]-C-phenylcarbonimidoyl]-2-hydroxy-1H-indol-6-yl]-N-ethylprop-2-ynamide), RO5126766 (CH5126766, 3-[[3-fluoro-2-(methylsulfamoylamino)pyridin-4-yl]methyl]-4-methyl-7-pyrimidin-2-yloxychromen-2-one), Cobimetinib (GDC-0973, [3,4-difluoro-2-(2-fluoro-4-iodoanilino)phenyl]-[3-hydroxy-3-[(2S)-piperidin-2-yl]azetidin-1-yl]methanone) and derivatives of these, and the like.

Examples of the p38 inhibitor include SB203580 (4-[4-(4-fluorophenyl)-2-(4-methylsulfinylphenyl)-1H-imidazol-5-yl]pyridine), Doramapimod (BIRB 796, 1-[5-tert-butyl-2-(4-methylphenyl)pyrazol-3-yl]-3-[4-(2-morpholin-4-ylethoxy)naphthalen-1-yl]urea), SB202190 (FHPI, 4-[4-(4-fluorophenyl)-5-pyridin-4-yl-1H-imidazol-2-yl]phenol), Ralimetinib dimesylate (5-[2-tert-butyl-4-(4-fluorophenyl)-1H-imidazol-5-yl]-3-(2,2-dimethylpropyl)imidazo[4,5-b]pyridin-2-amine; methanesulfonic acid), VX-702 (6-(N-carbamoyl-2,6-difluoroanilino)-2-(2,4-difluorophenyl)pyridine-3-carboxamide), PH-797804 (3-[3-bromo-4-[(2,4-difluorophenyl)methoxy]-6-methyl-2-oxopyridin-1-yl]-N,4-dimethylbenzamide), Neflamapimod (VX-745, 5-(2,6-dichlorophenyl)-2-(2,4-difluorophenyl)sulfanylpyrimido[1,6-b]pyridazin-6-one), TAK-715 (N-[4-[2-ethyl-4-(3-methylphenyl)-1,3-thiazol-5-yl]pyridin-2-yl]benzamide), PD 169316 (4-[4-(4-fluorophenyl)-2-(4-nitrophenyl)-1H-imidazol-5-yl]pyridine), TA-02 (4-[2-(2-fluorophenyl)-4-(4-fluorophenyl)-1H-imidazol-5-yl]pyridine), SD 0006 (1-[4-[3-(4-chlorophenyl)-4-pyrimidin-4-yl-1H-pyrazol-5-yl]piperidin-1-yl]-2-hydroxyethanone), Pamapimod (6-(2,4-difluorophenoxy)-2-(1,5-dihydroxypentan-3-ylamino)-8-methylpyrido[2,3-d]pyrimidin-7-one), BMS-582949 (4-[5-(cyclopropylcarbamoyl)-2-methylanilino]-5-methyl-N-propylpyrrolo[2,1-f][1,2,4]triazine-6-carboxamide), SB239063 (4-[4-(4-fluorophenyl)-5-(2-methoxypyrimidin-4-yl)imidazol-1-yl]cyclohexan-1-ol), Skepinone-L (13-(2,4-difluoroanilino)-5-[(2R)-2,3-dihydroxypropoxy]tricyclo[9.4.0.03,8]pentadeca-1(11),3(8),4,6,12,14-hexaen-2-one), DBM 1285 (N-cyclopropyl-4-[4-(4-fluorophenyl)-2-piperidin-4-yl-1,3-thiazol-5-yl]pyrimidin-2-amine; dihydrochloride), SB 706504 (1-cyano-2-[2-[[8-(2,6-difluorophenyl)-4-(4-fluoro-2-methylphenyl)-7-oxopyrido[2,3-d]pyrimidin-2-yl]amino]ethyl]guanidine), SCIO 469 (2-[6-chloro-5-[(2R,5S)-4-[(4-fluorophenyl)methyl]-2,5-dimethylpiperazine-1-carbonyl]-1-methylindol-3-yl]-N,N-dimethyl-2-oxoacetamide), Pexmetinib (1-[5-tert-butyl-2-(4-methylphenyl)pyrazol-3-yl]-3-[[5-fluoro-2-[1-(2-hydroxyethyl)indazol-5-yl]oxyphenyl]methyl]urea), UM-164 (2-[[6-[4-(2-hydroxyethyl)piperazin-1-yl]-2-methylpyrimidin-4-yl]amino]-N-[2-methyl-5-[[3-(trifluoromethyl)benzoyl]amino]phenyl]-1,3-thiazole-5-carboxamide), p38 MAPK Inhibitor (4-(2,4-difluorophenyl)-8-(2-methylphenyl)-7-oxido-1,7-naphthyridin-7-ium), p38 MAP Kinase Inhibitor III (4-[5-(4-fluorophenyl)-2-methylsulfanyl-1H-imidazol-4-yl]-N-(1-phenylethyl)pyridin-2-amine), p38 MAP Kinase Inhibitor IV (3,4,6-trichloro-2-(2,3,5-trichloro-6-hydroxyphenyl)sulfonylphenol), CAY105571 (4-[5-(4-fluorophenyl)-2-[4-(methylsulfonyl)phenyl]-1H-imidazol-4-yl]-pyridine) and derivatives of these, and the like.

As the JNK inhibitor, for example, those similar to the ones described in the first step can be mentioned. The substance that inhibits the intracellular signal transduction mechanism against stress to be used in the present invention is preferably one or more selected from the group consisting of MEK inhibitor, p38 inhibitor, and JNK inhibitor. When SB203580 is used as the p38 inhibitor, it can be added to the medium at a concentration of generally about 1 nM to about 1 mM, preferably about 10 nM to about 100 µM, more preferably about 100 nM to about 10 µM, further preferably about 500 nM to about 5 µM. When PD0325901 is used as the MEK inhibitor, it can be added to the medium at a concentration of generally about 1 nM to about 1 mM, preferably about 10 nM to about 100 µM, more preferably about 100 nM to about 10 µM, further preferably about 500 nM to about 5 µM. When JNK-IN-8 is used as the JNK inhibitor, it can be added to the medium at a concentration similar to that described in the first step. When other MEK inhibitor, p38 inhibitor, or JNK inhibitor is used, it is preferably used at a concentration showing inhibitory activity equivalent to that of the addition concentration of the above-mentioned inhibitor.

### 3. Cell population containing pituitary tissue

The present invention provides a cell population containing pituitary tissue, particularly a cell population containing 1) neural cells or neural tissue, 2) pituitary tissue, and 3) mesenchymal cells. It is hereinafter to be also referred to as the cell population of the present invention. The cell population of the present invention can be preferably produced by the above-mentioned production method of the present invention.

In the cell population of the present invention, 1) neural cells or neural tissue are(is) preferably cells or tissue of the central nervous system, or precursor tissue thereof and, as the cell or tissue of the central nervous system, retina, cerebral cortex, diencephalon (e.g., hypothalamus) and the cells derived from such tissues can be mentioned. It is more preferably diencephalon (e.g., hypothalamus) or precursor tissue thereof, further preferably diencephalon having a ventricle-like structure in the tissue or a precursor tissue thereof. The 1) neural cells or neural tissue is, for example, N-Cadherin positive neuroepithelial tissue.

The 2) pituitary tissue in the cell population of the present invention is preferably formed continuously from nonneural epithelial tissue. More preferably, nonneural epithelial tissue and pituitary tissue cover at least one of 1) neural cells or neural tissue and 3) mesenchymal cells.

It is also preferable that the aforementioned nonneural epithelial tissue is mouth cavity epithelium or precursor tissue thereof. The pituitary tissue preferably contains pituitary hormone-producing cells or progenitor cells thereof (pituitary progenitor cells), preferably contains pituitary stem cells, preferably contains folliculostellate cells, more preferably contains all of pituitary hormone-producing cells, pituitary progenitor cells, pituitary stem cells, and folliculostellate cells. As the pituitary hormone-producing cell, at least one kind selected from the group consisting of growth hormone (GH)-producing cell, prolactin (PRL)-producing cell and adrenocorticotropic hormone (ACTH)-producing cell can be mentioned.

It is also preferable that the pituitary niche is formed in the pituitary tissue, it is also preferable that the pituitary niche is an MCL niche-like structure around the residual cavity remaining between the anterior pituitary and the middle pituitary, it is also preferable that the pituitary niche is a parenchymal layer niche-like structure, and it is more preferable that it contains both an MCL niche-like structure and a parenchymal layer niche-like structure.

In the cell population of the present invention, 3) mesenchymal cells are preferably cranial mesenchymal cells.

The 3) mesenchymal cells are preferably present between nonneural epithelial tissue coating a surface of the cell population and 1) neural cells or neural tissue present inside the cell population.

In the cell population of the present invention, for example, ventricle-like vacuoles are formed inside the 1) neuroepithelial tissue, and the surface of the 1) neuroepithelial tissue in contact with the vacuoles is Ezrin, PKC-zeta positive apical surface.

The 3) mesenchymal cells contained in the cell population of the present invention expresses, for example, at least one kind of mesenchymal cell marker selected from the group consisting of Nestin, Vimentin, Cadherin-11, Laminin, CD44, CD90, and CD105.

The nonneural epithelial tissue that can be contained in the present invention expresses, for example, at least one kind of nonneural epithelial tissue marker selected from the group consisting of cytokeratin, E-Cadherin, and EpCAM.

The pituitary stem cell that can be contained in the cell population of the present invention expresses, for example, at least one kind of pituitary stem cell marker selected from the group consisting of Sox2, Sox9, E-Cadherin, Nestin, S100β, GFRα2, Prop1, CD133, β-Catenin, Klf4, Oct4, Pax6, Coxsackievirus and adenovirus common receptor (CXADR), PRRX1/2, Ephrin-B2, and ACE. A preferred embodiment of the cell population of the present invention includes a pituitary stem cell positive to a pituitary stem cell marker (e.g., CXADR). The proportion of the number of pituitary stem cells in the cell population (proportion of the number of pituitary stem cells) may be not less than 1%, preferably not less than 3%, or not less than 5%.

### 4. Production method of pituitary tissue

The present invention provides a production method of pituitary tissue, and the method is characterized by collection of pituitary tissue from a cell population containing pituitary tissue obtained by the above-mentioned "2. Production method of cell population containing pituitary tissue". One embodiment includes the following steps (1), (2), and (4).
(1) a first step of culturing pluripotent stem cells in the presence of a JNK signal transduction pathway inhibiting substance and a Wnt signal transduction pathway inhibiting substance to form a cell population,
(2) a second step of culturing (preferably suspension culturing) the cell population obtained in the first step in the presence of a BMP signal transduction pathway activating substance and a Sonic hedgehog signal transduction pathway activating substance, thereby obtaining a cell population containing pituitary tissue,
(4) a fourth step of collecting pituitary tissue from the cell population obtained in the second step.

The first step and the second step can be performed in the same manner as in the first step and second step of the above-mentioned "2. Production method of cell population containing pituitary tissue". When desired, step a may be performed before the first step. When desired, the third step may be performed between the second step and the fourth step.

In the fourth step of collecting pituitary tissue from a cell population containing pituitary tissue, when the formed cell population is a flat tissue obtained by adhesion culture, or the like, the pituitary tissue can be recovered by a method including physically peeling off the tissue by using a needle or the like under microscopic observation. When the formed cell population is a three-dimensional tissue such as cell mass and the like, it is generally performed by detaching and collecting pituitary tissue formed outside the cell mass (Rathke's pouch part) by using tweezers and the like under microscopic observation. The pituitary tissue can be distinguished as, for example, as described in Nature communications, 2016, 7., a semitransparent thin epithelium on the surface layer of the obtained cell mass. As a method for collecting the pituitary tissue from the cell population (cell mass) in the fourth step, a freeze-thawing method, preferably a slow freezing method, can also be used. According to the method, a cell mass having pituitary tissue on the outside and mesenchymal nerve and neuroepithelial tissue in the inside is subjected to freeze-thawing whereby the pituitary tissue on the outside is detached from the cell mass without a physical treatment.

### 5. Reagent for evaluation of toxicity and efficacy, and toxicity and efficacy evaluation method

The cell population of the present invention, a cell population produced by the production method of the present invention, or tissue collected from the cell population may be pituitary tissue. Therefore, a reagent for evaluating the toxicity and efficacy of a test substance containing the cell population of the present invention, a cell population produced by the production method of the present invention, or pituitary tissue collected from the cell population can be provided.

Also, the present invention can provide a method for evaluating toxicity or efficacy by using the aforementioned cell population or pituitary tissue recovered from the cell population.

For example, a method for evaluating toxicity or efficacy of a test substance, including a step of bringing a cell population or pituitary tissue collected from the cell population into contact with a test substance, and a step of detecting an influence of the test substance on the cell population or pituitary tissue can be mentioned.

Alternatively, a method for evaluating toxicity and drug efficacy when a vector is used in gene therapy and other uses, including a step of contacting a cell population or pituitary tissue recovered from the cell population with the vector used for introducing a nucleic acid/gene of a specific sequence into cells; and a step of testing the effect of the vector or transgene/nucleic acid on the cell population or pituitary tissue, such as the effect of the transgene, the deliverability of the transgene, the degree of cell damage, and the like can be mentioned.

The cell population of the present invention, a cell population produced by the production method of the present invention, or pituitary tissue recovered from the cell population and used as a reagent for evaluating the toxicity and drug efficacy of a test substance is also preferably produced using, as a raw material, pluripotent stem cells whose genes have been manipulated by genome editing. The gene edited in the pluripotent stem cells as a raw material is preferably a disease-related gene, more preferably a pituitary disease-related gene. Examples of the aforementioned pituitary diseases include acromegaly, Cushing's disease, prolactin-producing pituitary adenoma, TSH (thyroid-stimulating hormone)-producing pituitary adenoma, craniopharyngioma, Rathke's cyst, hypophysitis, hypopituitarism, childhood growth hormone secretion deficiency, male hypogonadotropin (LH, FSH) hypogonadism, hypothalamic/pituitary amenorrhea, multiple endocrine neoplasia, and the like. These causative genes and disease-related genes are preferred targets for genome editing in pluripotent stem cells, but are not limited thereto. Another embodiment of the disease-related gene is a gene involved in cancer and tumor development in the pituitary or hypothalamus. Such disease-related genes include, but are not limited to, for example, AIP, GPR101, MEN1, MEN4, CDKN1B, PRKAR1A, PRKACB 2q16, SDHA/B/C/D, SDHAF2, NF1, DICER1, GNAS, USP8, PIK3CA, MTND1,2,4,5, MTTL2, MTTM, MTCYB, MTRNR2 and the like. Alternatively, cells may be collected from normal and diseased areas of the pituitary gland and other tissues of a healthy person or patient, iPS cells are established, and the pituitary tissue described in the present application can also be prepared.

### 6. Pharmaceutical composition, therapeutic drug, and method for treating diseases

One embodiment of the present invention is a pharmaceutical composition (composition for transplantation, tissue for transplantation, or Transplant) containing the cell population of the present invention, a cell population produced by the production method of the present invention, or pituitary tissue collected from the cell population. The pharmaceutical composition preferably further contains, in addition to the cell population of the present invention, a cell population produced by the production method of the present invention, or pituitary tissue collected from the cell population, a pharmaceutically acceptable carrier.

As a pharmaceutically acceptable carrier, a physiological aqueous solvent (physiological saline, buffer, serum-free medium, etc.) can be used. Where necessary, the pharmaceutical composition may contain preservative, stabilizer, reducing agent, tonicity agent, and the like that are generally used in medicines containing tissues or cells to be transplanted in transplantation therapy.

As one embodiment of the present invention, a therapeutic drug for a disease due to a disorder of pituitary gland, which contains the cell population of the present invention, or a cell population produced by the production method of the present invention, or pituitary tissue collected from the cell population can be provided.

Examples of the therapeutic drug for a disease due to a disorder of pituitary gland include a graft containing a suspension containing the cell population of the present invention or a cell population produced by the production method of the present invention.

Examples of the suspension include a liquid obtained by suspending a cell population in a medium, an artificial lacrimal fluid or physiological saline. The suspension may contain nonneural epithelial cells isolated from a cell population, and may also contain a factor that promotes adhesion of the cells, such as extracellular matrix, and hyaluronic acid.

Pituitary tissue collected from the cell population may also be used in place of cell masses.

Furthermore, a method for treating a disease due to a disorder of pituitary gland, including a step of transplanting an effective amount of pituitary tissue from the cell population of the present invention, or a cell population produced by the production method of the present invention, to a target in need of the transplantation can be provided.

The aforementioned disease due to a disorder of pituitary gland may be an animal disease due to a disorder of pituitary gland, or a disease due to a disorder of pituitary gland in a non-human animal. As the disease due to a disorder of pituitary gland, panhypopituitarism, pituitary dwarfism, hypoadrenocorticism, partial hypopituitarism, pituitary anterior hormone isolated deficiency, insufficient pituitary function/hormone secretion after surgery for pituitary adenoma and the like, craniopharyngioma, and the like can be specifically mentioned.

The cell population of the present invention, a cell population produced by the production method of the present invention, or pituitary tissue recovered from the cell population and used as a therapeutic drug for a disease due to a disorder of pituitary gland is also preferably produced using, as a raw material, pluripotent stem cells whose genes have been manipulated by genome editing. Examples of the genes targeted for genome editing include, but are not limited to, genes involved in the differentiation of pituitary tissue by the production method of the present invention, genes involved in differentiation into unintended cells other than the pituitary by-produced by the production method of the present invention, hormone-related genes secreted from the pituitary, genes involved in disease infection, and the like.

### [Example]

The present invention is explained in more detail in the following by referring to Examples, which are not to be construed as limiting the scope of the present invention. Unless particularly limited, the reagents and materials to be used are commercially available.

### [Example 1: Study of effect of JNK inhibitor on cell differentiation in production of cell population containing pituitary tissue (pituitary organoid) from human iPS cell]

Human iPS cells (HC-6#10 strain, obtained from RIKEN) were cultured under feeder-free conditions according to the method described in Scientific Reports, 4, 3594 (2014). As the feeder-free medium, StemFit medium (manufactured by Ajinomoto Co., Inc.) was used and, as the feeder-free scaffold, Laminin 511-E8 (manufactured by Nippi, Inc.) was used.

As specific maintenance culturing operation, subconfluent human iPS cells (HC-6#10 strain) were first washed twice with 0.5 mM EDTA/PBS and further treated with 5 mM EDTA/PBS at 37°C for 10 min. After pipetting, the cells were scraped from the surface of the culture dish and dispersed into single cells. Thereafter, the human iPS cells dispersed into single cells were seeded in a plastic culture dish coated with Laminin 511-E8, and cultured under feeder-free conditions in StemFit medium in the presence of Y-27632 (ROCK inhibiting substance, manufactured by FUJIFILM Wako Pure Chemical Corporation, 10 µM). When a 6-well plate (manufactured by Corning, for cell culturing, culture area 9.5 cm²) was used as the plastic culture dish, the number of plated cells of the human iPS cells dispersed into single cells was adjusted to 7 × 10³. One day after seeding, the entire amount of the medium was changed with StemFit medium free of Y-27632. Thereafter, once in 1 - 2 days, the entire amount of the medium was changed with StemFit medium free of Y-27632. Thereafter, the cells were cultured until 7 days after seeding when they reached subconfluence (60% of culture area is covered with cells).

When the cultured pluripotent stem cells were used for differentiation induction, SB-431542 (TGFβ signal transduction pathway inhibiting substance, manufactured by FUJIFILM Wako Pure Chemical Corporation, final concentration 5 µM) and SAG (Shh signal pathway activating substance, manufactured by Enzo Life Sciences, final concentration 300 nM) were added at 6 days after seeding and simultaneously with the medium change with Stemfit medium, and the cells were cultured for 24 hr (start of step (a)).

The prepared subconfluent human iPS cells were treated with 0.5 mM EDTA/PBS in the same manner as in the above-mentioned passaging. A serum-free medium for differentiation induction was added, pipetting was performed, and the cells were scraped from the surface of the culture dish and dispersed into single cells.

Thereafter, the human ES cells dispersed into single cells were suspended in 100 µl of a serum-free medium at 9 × 10³ cells per well of a non-cell-adhesive 96-well culture plate (PrimeSurface 96V-bottom plate, MS-9096V, manufactured by SUMITOMO BAKELITE), and suspension cultured under the conditions of 37°C, 5% CO₂. As the serum-free medium therefor, a serum-free medium which is a 1:1 (volume ratio) mixture of F-12+Glutamax medium (manufactured by Thermo Fisher Scientific) and IMDM+Glutamax medium (manufactured by Thermo Fisher Scientific) supplemented with 5% Knockout Serum Replacement (manufactured by Thermo Fisher Scientific), 450 µM 1-monothioglycerol (manufactured by FUJIFILM Wako Pure Chemical Corporation), 1x Chemically defined lipid concentrate (manufactured by Thermo Fisher Scientific), 50 unit/ml penicillin-50 µg/ml streptomycin (manufactured by Nacalai Tesque) was used. Hereinafter this serum-free medium is also to be referred to as 5% KSR gfCDM. At the time of the start of suspension culturing (day 0 after the start of suspension culturing, start of step (1)), Y-27632 (final concentration 10 µM), IWP-2 (Wnt signal transduction pathway inhibiting substance, manufactured by Tocris Bioscience, 0.5 µM), and SB431542 (TGF signal transduction pathway inhibiting substance, manufactured by Wako Pure Chemical Industries, Ltd., 1 µM) were added to the aforementioned serum-free medium. Furthermore, in order to verify the effect of inhibiting the JNK signal transduction pathway in inducing pituitary differentiation from pluripotent stem cells, conditions with the addition of a c-Jun N-terminal kinase (JNK) inhibitor, JNK-IN-8 (manufactured by Merck, 1 µM) were compared with those without the addition.

On day 2 after the start of the suspension culturing, a serum-free medium not containing Y-27632 and containing IWP-2, SB431542, BMP4 (BMP signal transduction pathway activating substance), and SAG was added at 100 µl per well. BMP4 was added at 1 nM to the medium so that the final concentration in the well would be 0.5 nM, and SAG was added at 1.4 µM to the medium so that the final concentration in the well would be 700 nM. Thereafter, a half amount of the medium was changed with a serum-free medium not containing both Y-27632 and BMP4 and containing IWP-2, SB-431542, and SAG was added at 6, 10, 13, 17, 21, and 24 days after the start of suspension culturing.

On day 28 after the start of suspension culture, phase contrast observation was performed using an inverted microscope (manufactured by KEYENCE CORPORATION, BIOREVO BZ-9000) (Fig. 1 bottom diagram). Spherical cell aggregates with a diameter of about 1000 µm were formed by the above-mentioned differentiation induction method. By comparison of the conditions with and without the addition of JNK inhibitor, it was found that, under the condition with the addition of JNK inhibitor, the size of the cell mass was about 100 um smaller in diameter than that under the condition without addition of JNK inhibitor, a large proportion of the surface of the cell mass is covered with nonneural epithelial tissue/placode-like tissue, which is the basis of pituitary tissue, the proliferation of unintended cells other than nonneural epithelial tissues is suppressed, and the differentiation efficiency was improved. The above results indicate that the production efficiency is improved by adding a JNK inhibitor in pituitary tissue production from human pluripotent stem cells.

### [Example 2: Study of timing of addition JNK inhibitor in production of cell population containing pituitary tissue (pituitary organoid) from human iPS cell]

In Example 2, according to step shown in Fig. 2A, a cell population including cells that constitute pituitary tissue was produced. Specifically, human iPS cells (1231A3 strain, obtained from RIKEN) were cultured under feeder-free conditions according to the method described in Scientific Reports, 4, 3594 (2014). As the feeder-free medium, StemFit medium was used and, as the feeder-free scaffold, Laminin 511-E8 was used.

Differentiation induction was performed under the same conditions as in Example 1 except for the condition of adding a JNK inhibitor, compared to the conditions of Example 1. As the condition for adding JNK inhibitor, conditions including addition of JNK-IN-8 (manufactured by Merck, 1 µM), which is a c-Jun N-terminal kinase (JNK) inhibitor, on days 0, 2, 6, 10, and 13 after the start of differentiation induction were compared with conditions without addition of JNK inhibitor. When the JNK inhibitor is added on days 2, 6, 10, and 13 after the start of differentiation induction, 2 µM was added to the medium when adding or replacing the medium, so that the final concentration in the well would be 1 µM.

On day 28 after the start of suspension culture, bright field observation by polarized illumination was performed using an inverted microscope (manufactured by KEYENCE CORPORATION, BIOREVO BZ-X800) (Fig. 2B). As a result, the nonneural epithelium/pituitary was formed more efficiently under conditions in which JNK inhibitor was added on days 0, 2, and 6 after the start of differentiation induction than under conditions in which it was not added, but the conditions including addition on days 10 and 13 after the start of differentiation showed no difference from the no addition conditions. The above results indicate that JNK inhibitor is preferably added until day 10 after the start of differentiation induction, in pituitary tissue production from human pluripotent stem cells.

### [Example 3: Study of effect of JNK inhibitor on pituitary hormone secretory capacity in production of cell population containing pituitary tissue (pituitary organoid) from human iPS cell]

In Example 3, according to step shown in Fig. 3A, a cell population including cells that constitute pituitary tissue was produced. Specifically, human iPS cells (201B7 strain, obtained from Kyoto University) were cultured under feeder-free conditions according to the method described in Scientific Reports, 4, 3594 (2014). As the feeder-free medium, StemFit medium was used and, as the feeder-free scaffold, Laminin 511-E8 was used.

The aforementioned subconfluent human iPS cells were subjected to cell dispersion treatment using TrypLE Select, and further dispersed to single cells by pipetting operation. Thereafter, the aforementioned human ES cells dispersed into single cells were suspended in 100 µl of a serum-free medium at 9000 cells per well of a non-cell-adhesive 96-well culture plate, and suspension cultured under the conditions of 37°C, 5% CO₂. As the serum-free medium (gfCDM+KSR) therefor, a serum-free medium which is a 1:1 mixture of F-12 medium and IMDM medium supplemented with 5% KSR, 450 µM 1-monothioglycerol, 1x Chemically defined lipid concentrate was used. At the time of the start of suspension culturing (day 0 after the start of differentiation induction), Y-27632 (final concentration 10 µM), IWP-2 (0.5 µM), SB431542 (1 µM), and SAG (100 nM) were added to the aforementioned serum-free medium.

On day 2 after the start of the differentiation induction, a serum-free medium not containing Y27632 and containing IWP-2, SB431542, BMP4 (0.5 nM), and SAG (700 nM) was added at 100 µl per well. Thereafter, a half amount of the medium was changed with a serum-free medium not containing both Y27632 and BMP4 and containing IWP-2, SB431542, and SAG was added at 6, 9, 12, 15, 19, 22, and 26 days after the start of differentiation induction. After day 19, the oxygen partial pressure during culture was set to 40%.

The aforementioned cell aggregates on the 29th day after the start of differentiation induction were each fixed with 4% para-formaldehyde, and cryosections were prepared. These cryosections were subjected to immunostaining using pituitary precursor markers Pitx1 (anti-Pitx1 antibody, homemade (Nature Communications, 7: 10351, 2016, Cell Reports, 30, 18-24, January 7, 2020)) and Lhx3 (anti-Lhx3 antibody, homemade (Nature Communications, 7: 10351, 2016, Cell Reports, 30, 18-24, January 7, 2020)), and antibody against epithelial cell marker E-cadherin (manufactured by Takara Bio Inc.). After the primary antibody reaction, detection was performed using a secondary antibody (manufactured by Life Technologies) fluorescence-labeled with Alexa488, 555, and 647. As a result, in the cell aggregates induced by the above differentiation induction method and on day 29 after the start of differentiation induction, under conditions in which 1 µM JNK-IN-8 was added from day 0 of differentiation induction, a higher proportion of the surface of the cell mass was covered with E-Cadherin-positive epithelial tissue, and the number of cells positive for Pitx1 and Lhx3, which are pituitary precursor markers, was also higher as compared with the non-addition control (Fig. 3B). The above results show that addition of a JNK inhibitor promotes induction of pituitary differentiation from pluripotent stem cells.

### [Example 4: Study of effect of JNK inhibitor on pituitary hormone secretory capacity in production of cell population containing pituitary tissue (pituitary organoid) from human iPS cell]

In Example 4, according to step shown in Fig. 4A, a cell population including cells that constitute pituitary tissue was produced. Specifically, the culture supernatant of a cell mass derived from human iPS cell line 201B7 prepared by the method described in Example 3 was collected over time, and the amount of ACTH secreted per cell mass was measured. As a specific measurement method, cell masses containing pituitary tissue were prepared under four conditions: from day 0 to day 30 of differentiation induction, conditions in which the JNK inhibitor JNK-IN-8 was added at 1 µM, 3 µM, or 10 µM, and no addition control. Among these, collapse of cell mass was confirmed under the conditions in which 10 µM of JNK-IN-8 was added. Thereafter, the cell masses prepared by adding 1 µM or 3 µM of JNK-IN-8 and the non-addition control were suspension cultured in a 10 cm dish with 20 ml of medium, and half of the medium was replaced every 3 to 4 days. The culture supernatant was collected before replacement of a half-volume of the medium on days 61, 103, 152, and 201 of differentiation induction, and frozen at -150°C. When all samples were collected, the ACTH concentration in the recovered medium was measured by the ELISA method used in clinical testing (testing outsourced to SRL, Ltd.). Based on the obtained ACTH concentration data (pg/mL), the total number of cell masses at the time of sampling and the total amount of culture medium were corrected to the ACTH concentration under the conditions where 20 cell masses were cultured in 20 ml of medium, and shown in a graph. A similar experiment was performed twice.

As a result, the ACTH concentration in the medium was about 5 times as high on day 61 of differentiation induction, about 2 times as high on days 103 and 152 of differentiation induction, and about 1.3 times as high on day 201 of differentiation induction under the conditions in which 1 µM JNK-IN-8 was added from day 0 of differentiation induction, compared with the JNK inhibitor non-addition conditions. Under the conditions in which 3 µM JNK-IN-8 was added, the ACTH concentration in the medium was higher on days 61 and 103 of differentiation induction than that in the non-addition condition, but lower on days 152 and 201 of differentiation induction than that in the non-addition condition. From the above results, it was shown that the addition of a JNK inhibitor improves the ACTH secretory capacity per cell mass in differentiation induction of pituitary tissue from pluripotent stem cells, and that the concentration of the JNK inhibitor to be added is preferably not more than 3 µM (Fig. 4B).

### [Example 5: Study of effect of shaking culture in production of cell population containing pituitary tissue (pituitary organoid) from human iPS cell]

In Example 5, according to step shown in Fig. 5, a cell population including cells that constitute pituitary tissue was produced. Specifically, a cell mass on day 30 of differentiation induction and produced from human iPS cell line 1231A3 by the method described in Example 2 was transferred into a non-cell-adhesive T75 tissue culture flask (manufactured by Corning Incorporated) and subsequent suspension culturing was performed. As the conditions for suspension culturing, a serum-free medium which is a 1:1 mixture of F-12 medium and IMDM medium supplemented with 10% KSR, 450 µM 1-monothioglycerol, 1x Chemically defined lipid concentrate was used and 1 µM SB431542 and 700 nM SAG were added. 48 cell masses were suspension cultured in 25 ml of a medium, and half of the medium was replaced every 3 to 4 days. The flask containing the above-mentioned cell masses was subjected to shaking culture at 30 rpm using a reciprocating shaker (NS-LR, manufactured by As One), and compared with the conditions of static culture in the same flask. On day 43 after the start of suspension culture, bright field observation by polarized illumination was performed using an inverted microscope (manufactured by KEYENCE CORPORATION, BIOREVO BZ-X800) (Fig. 5 bottom diagram).

As a result, in the cell mass subjected to shaking culture, the epithelial structure on the surface of the cell masses developed, the growth of pituitary tissue was improved, and it was shown that shaking culture is useful for the production of pituitary tissue from pluripotent stem cells.

### [Example 6: Study of effect of substance having the action of reducing oxidative stress in production of cell population containing pituitary tissue (pituitary organoid) from human iPS cell]

In Example 6, according to step shown in Fig. 6 top diagram, a cell population including cells that constitute pituitary tissue was produced. Specifically, cell masses containing pituitary tissue were prepared from human iPS cell line 1231A3 by the method described in Example 5. On day 30 of differentiation induction, the cell masses were transferred to a T75 flask, and the culture conditions were changed. As a substance having the action of reducing oxidative stress, N-acetylcysteine (NAC) was added to the medium at a concentration of 1 mM from day 10 of differentiation induction, and compared with the non-addition conditions. On days 30 and 51 after suspension culturing, bright field observation by polarized? illumination was performed using an inverted microscope (manufactured by KEYENCE CORPORATION, BIOREVO BZ-X800) (Fig. 6 bottom diagram).

As a result, under the conditions in which NAC was added, the cell masses were about 50 um smaller in diameter than in the non-addition conditions at the time point of day 30 of differentiation induction, and the proliferation of unintended cells was suppressed. Furthermore, by performing suspension culturing, a cell mass covered with a high proportion of epithelial tissue including the pituitary was formed on day 51 of differentiation induction. From the above-mentioned results, it was shown that addition of a substance having the action of reducing oxidative stress is useful for the production of pituitary tissue from pluripotent stem cells.

### [Reference Example 1: Study of effect of conducting step a in production of cell population containing pituitary tissue (pituitary organoid) from human ES cell]

Using human ES cells (RAX::Venus knock-in KhES-1 strain, RIKEN), a group to perform step a and a group to not perform step a were established according to the steps shown in Fig. 7A, and a cell population including cells that constitute pituitary tissue was produced.

Specifically, subconfluent human ES cells were seeded in a plastic culture dish coated with Laminin 511-E8 in the same manner as in Example 1, and cultured under feeder-free in StemFit medium containing 300 nM SAG and 5 µM SB431542 (Step (a)). One day after seeding, the aforementioned human ES cells were subjected to cell dispersion treatment using TrypLE Select, and further dispersed to single cells by pipetting operation. Thereafter, the aforementioned human ES cells dispersed into single cells were suspended in 100 µl of a serum-free medium at 1.0×10⁴ cells per well of a non-cell-adhesive 96-well culture plate, and suspension cultured under the conditions of 37°C, 5% CO₂. As the serum-free medium (gfCDM+KSR) therefor, a serum-free medium which is a 1:1 mixture of F-12 medium and IMDM medium supplemented with 5% KSR, 450 µM 1-monothioglycerol, 1x Chemically defined lipid concentrate was used. At the time of the start of suspension culturing (day 0 after the start of differentiation induction), Y-27632 (final concentration 20 µM) and SAG (100 nM) were added to the aforementioned serum-free medium (step (1)).

On day 2 after the start of the differentiation induction, a serum-free medium not containing Y-27632 and containing BMP4 (5 nM) and SAG (2 µM) was added at 100 µl per well. Thereafter, a half amount of the medium was changed with a serum-free medium not containing Y-27632 and containing BMP4 and SAG at 6, 9, 12, and 15 days after the start of differentiation induction (start of step (2)). Thereafter, a half amount of the medium was changed with a serum-free medium not containing both Y-27632 and BMP4 and containing SAG at 19, 22, and 26 days after the start of differentiation induction. After day 19, the oxygen partial pressure during culture was set to 40%. Using cell aggregates on day 32 after the start of suspension culturing, cryosections were prepared in the same manner as in Example 3. These cryosections were subjected to immunostaining using pituitary precursor marker PITX1 (anti-Pitx1 antibody, homemade) and antibody against epithelial cell marker E-cadherin (manufactured by Takara Bio Inc.), and cell nuclei were stained with DAPI. As a result, in the group in which step a was not performed, aggregates were not formed well and collapsed (data not shown). On the other hand, in the group in which step a was performed, it was confirmed that cell aggregates were formed that included two types of layers of a tissue with PITX1-positive and E-cadherin-positive cells and a tissue with RAX::Venus-positive cells (Fig. 7B). However, as a result of staining with an antibody against LHX3 (rabbit; 1:3,000, manufactured by Takara Bio Inc.), which is a pituitary progenitor cell marker, not so many LHX3-positive cells were detected. This result suggests that a cell aggregate having a two-layer structure can be obtained by performing step a. The scale bar at the bottom right of Fig. 7B indicates 200 µm.

### [Reference Example 2: Study of effect of TGFβ/Nodal/Activin/BMP and Wnt signal regulation in production of cell population containing pituitary tissue (pituitary organoid) from human iPS cell]

Using human ES cells (RAX::Venus knock-in KhES-1 strain, RIKEN), step a was performed according to the steps shown in Fig. 8A. After performing step a, suspension culturing was performed in a medium containing a Wnt signal inhibitor IWP2 (2 µM) and a TGFβ signal inhibitor SB431542 (1 µM) in addition to BMP4 (5 nM) and SAG (100 nM (step (1)) or 2 µM (step (2)). IWP2 and SB431542 were added during the period from day 0 to day 6 after the start of suspension culturing (Fig. 8B-1), from day 0 to day 12 after the start of suspension culturing (Fig. 8B-2), or from day 0 to day 29 after the start of suspension culturing (Fig. 8B-3). BMP4 was added during the period from day 2 to day 6 after the start of suspension culturing (upper panel of each of Figs. 8B-1 to -3) or from day 2 to day 18 after the start of suspension culturing (lower panel of each of Figs. 8B-1 to -3). Using cell aggregates on day 29 after the start of suspension culturing, cryosections were prepared in the same manner as in Example 3. These cryosections were subjected to immunostaining using PITX1 (anti-Pitx1 antibody, homemade) and antibody against epithelial cell marker E-cadherin (manufactured by Takara Bio Inc.). As a result, it was confirmed that the outside of the aggregate was a nonneural epithelial tissue that was positive for PITX1 and E-cadherin, and the inside was a neural epithelial tissue that was positive for RAX::Venus (Figs. 8B-1 to -3).

It was also found that LHX3-positive cells were present in the outer cell layer containing nonneural epithelial tissue that was PITX1-positive and E-cadherin-positive (Figs. 8B-1 to -3). The conditions under which PITX1-positive and E-cadherin-positive nonneural epithelial tissue could be formed with the highest probability were the case where IWP2 and SB431542 were added during the period of from day 0 to day 29 after the start of suspension culturing, and BMP4 was added during the period of from day 2 to day 6 after the start of suspension culturing (Fig. 8B-3). From these results, it was shown that outer nonneural epithelial tissue is formed with high efficiency from human iPS cells under differentiation-inducing conditions with the addition of Wnt signal inhibitor and TGFβ signal inhibitor, and a cell mass in which part of the outer nonneural epithelium is an LHX3-positive pituitary placode can be produced. The scale bar at the bottom right of Figs. 8B-1 to -3 indicates 200 µm.

### [Reference Example 3: Study of concentration of each signal regulator in production of cell population containing pituitary tissue (pituitary organoid) from human ES cell]

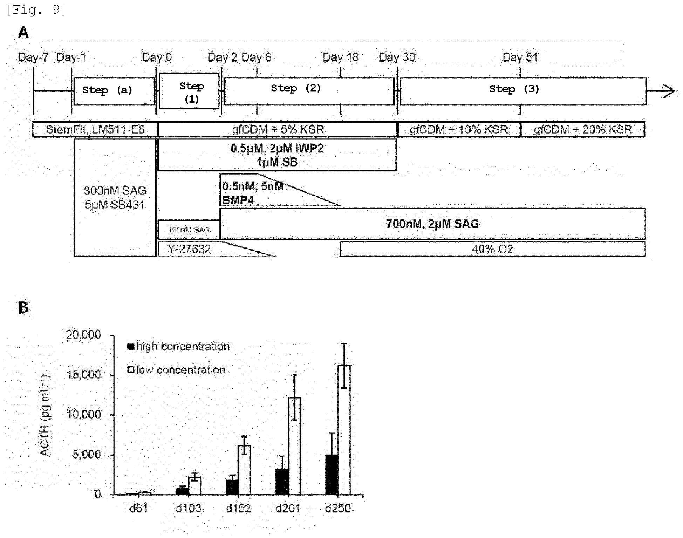

Then, for the purpose of examining the optimal concentrations of IWP2, BMP4, and SAG to be added in each step, two groups of a high concentration group (IWP2; 2 µM, BMP4; 5 nM, SAG; 2 µM) and a low concentration group (IWP2; 0.5 µM, BMP4; 0.5 nM, SAG; 700 nM) were established, and a comparative study was conducted using the ACTH concentration in the culture medium on days 61, 103, 152, 201, and 250 of differentiation induction as an indicator. Using human ES cells (KhES-1 strain) and according to step shown in Fig. 9A, a cell population including cells that constitute pituitary tissue was produced. Using the method described in Example 4, the concentration of ACTH in the medium was measured. As the result of the study, the low concentration group showed a higher ACTH concentration in the culture medium (Fig. 9B). This result suggests that the low concentration group (IWP2; 0.5 µM, BMP4; 0.5 nM, SAG; 700 nM) is suitable for inducing differentiation into a tissue with superior ACTH secretory capacity.

### [Example 7: Study of effect of JNK inhibitor on induction of pituitary epithelial tissue and pituitary hormone secretory cell in production of cell population containing pituitary tissue (pituitary organoid) from human iPS cell]

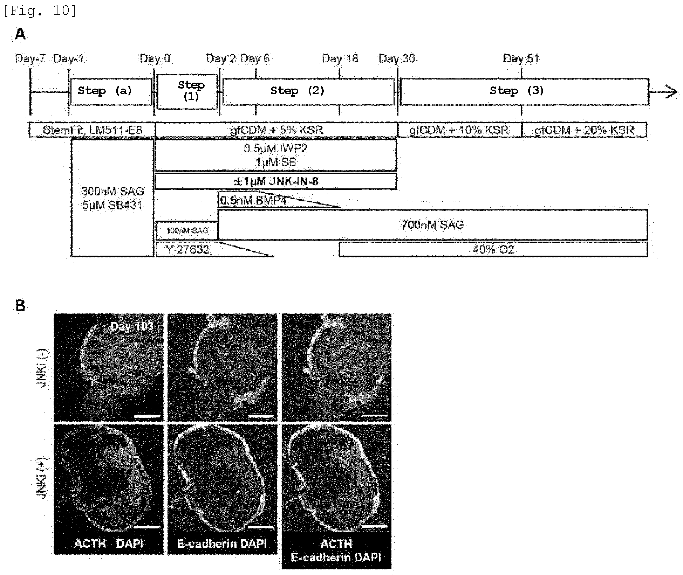

According to the steps shown in Fig. 10A, suspension culturing was performed using human iPS cells (201B7 strain) in the same manner as in Example 4, and continued until day 103. Using the obtained cell aggregates, cryosections were prepared in the same manner as in Example 3. The obtained sections were subjected to immunostaining using anti-ACTH antibody (manufactured by Fitzgerald Industries) and anti-E-cadherin antibody (manufactured by Takara Bio Inc.), and the nucleus was stained with DAPI. As a result, it was found that the proportion of E-cadherin-positive cells in the layer covering the surface of cell aggregates was higher and the proportion of ACTH-positive cells in the entire cell aggregate was also high under the conditions in which JNK-IN-8 was added, as compared with the non-addition control (JNKi(+); 42.2±4.4%, JNKi(-); 28.8±4.0% of total cells, mean±SEM, n=8-12) (Fig. 10B). The scale bar at the bottom right of Fig. 10B indicates 200 µm.

### [Example 8: Confirmation of presence of pituitary hormone-secreting cell in production of cell population containing pituitary tissue (pituitary organoid) from human iPS cell]

Human iPS cells (1231A3 strain) were suspension cultured by a method similar to that in Example 6. Using cell aggregates on day 59 of culture, cryosections were prepared in the same manner as in Example 3. The obtained sections were subjected to immunostaining using anti-ACTH antibody (manufactured by Lab Vision) and anti-SOX2 antibody (manufactured by Santa Cruz), and cell nuclei were stained with DAPI. As a result, it was confirmed that pituitary epithelial tissue was formed on the surface layer of the cell aggregates and ACTH-positive cells were present in the whole cell aggregates (Fig. 11). The scale bar at the bottom right of Fig. 11 indicates 200 µm.

### [Example 9: Study of SAG addition period in production of cell population containing pituitary tissue (pituitary organoid) from human iPS cell]

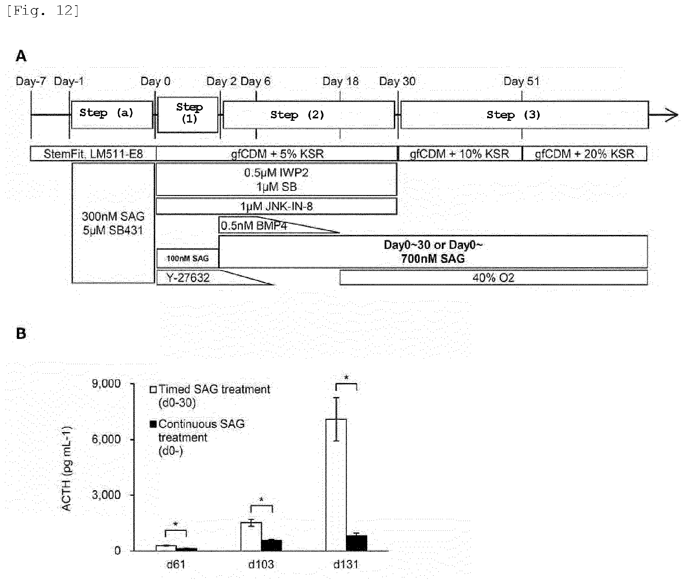

Using human iPS cells (201B7 strain) and according to the steps shown in Fig. 12A, cell aggregates were produced. Regarding the SAG treatment period, two groups of a group in which SAG is added from immediately after the start of differentiation induction until day 30, and a group in which SAG is added from immediately after the start of differentiation induction and after day 30 (until day 61, until day 103, and until day 131) were set, and the ACTH secretion capacity was compared. ACTH was measured by a method similar to that in Example 4.

As a result, it was found that ACTH secretion capacity was improved by stopping SAG treatment 30 days after the start of differentiation induction (Fig. 12B).

### [Example 10: Study of gene expression variation in production of cell population containing pituitary tissue (pituitary organoid) from human ES cell]

Using human ES cells (KhES-1 strain) and according to the steps shown in Fig. 13A, cell aggregates were produced. In order to confirm the degree of differentiation at each culture day after the start of differentiation induction, changes in the expression of pituitary markers (PITX1, LHX3, POMC (ACTH progenitor cells)) and hypothalamic markers (RAX, NKX2.1 (TTF1)) were examined by quantitative PCR. Specifically, it was performed as follows.

RNA was extracted from six cell aggregates per sample using the RNeasy Micro Kit (Qiagen). Quantitative PCR was performed using Biomark HD (Fluidigm). As the probes for each gene, GAPDH (Hs02758991_g1), PITX1 (Hs00267528-m1), LHX3 (Hs01033412_m1), POMC (Hs01596743_m1), RAX (Hs00429459-m1), and TTF1 (Hs00968940-m1) (TaqMan Probes; Thermo Fisher Scientific) were used. The obtained data were normalized using GAPDH as an endogenous control, and quantitative results were obtained using the comparative Ct method (ΔΔCt method).

As a result, the expression of LHX3 increased from day 6 to day 60 after the start of differentiation induction, the expression of LHX3 increased from day 19 to day 30 after the start of differentiation induction, and the expression of POMC increased after day 30 from the start of differentiation induction (Fig. 13B). These results show that differentiation into the pituitary was induced in a stepwise manner.

On the other hand, the expression of RAX reached a plateau around day 3 to day 6 after the start of differentiation induction, and thereafter decreased (Fig. 13C). The expression of TTF1 increased from day 6 to day 19 after the start of differentiation induction, and thereafter decreased (Fig. 13C). These results suggest that in the initial stages of differentiation, differentiation into hypothalamus (ventral hypothalamus) proceeds along with the differentiation into pituitary precursor tissue.

### [Example 11: Confirmation of expression of various hormone secretory cell in production of cell population containing pituitary tissue (pituitary organoid) from human ES cell]

Using human ES cells (KhES-1 strain) and a differentiation induction method similar to that in Example 10, cell aggregates were produced. Using cell aggregates on day 103 after the start of suspension culturing and in the same manner as in Example 3, cryosections were prepared. The sections were subjected to immunostaining using anti-prolactin (PRL) antibody (manufactured by Dako), anti-POU1F1 antibody (homemade), anti-thyroid-stimulating hormone (TSH) antibody (manufactured by Dako), anti-luteinizing hormone (LH) antibody (manufactured by Dako), and anti-follicle-stimulating hormone (FSH) antibody (manufactured by Dako), and cell nuclei were stained with DAPI. In addition, cell aggregates on day 152 after the start of suspension culturing were subjected to immunostaining using anti-growth hormone (GH) antibody (manufactured by Santa Cruz), and cell nucleus was stained with DAPI. As a result, the presence of cells that reacted with all the antibodies used was confirmed. This result shows that cell aggregates containing pituitary progenitor cells have the ability to secrete multiple types of hormones (Fig. 14). The scale bar at the bottom right of Fig. 14 indicates 50 µm.

### [Example 12: Study of structure of cell aggregates containing pituitary tissue (pituitary organoids) using electron microscope]

Cell aggregates containing pituitary tissue were produced using the same differentiation induction method as in Example 10, and the culture after day 51 from the start of suspension culturing in Fig. 13A was continued until day 201. The cell aggregates were fixed using 4% paraformaldehyde, 1% glutaraldehyde, and 2% sucrose at 4°C for 3 days. After dehydration with an alcohol solution and polymerization and embedding with LR-WH1TE resin (Nissin EM) according to a conventional method, ultrathin sections were prepared and observed with an electron microscope (Hitachi H-7500).

As a result, many cells in the pituitary tissue contained hormone-secreting granules (Figs. 15B, C, D). Cell death and fibrosis were observed in the inner layer of the cell aggregates (hypothalamic tissue), particularly in the deeper layer (Fig. 15A: areas indicated by "*"). Endocrine cells with secretory granules inside the cells, which correspond to the anterior pituitary, were present within the organoid wall (Fig. 15A, B, C). In many areas, the pituitary cell layer formed a thin cystic wall and was composed of endocrine cells in a false multi-row columnar epithelium. The lowest layer of the pituitary cell layer formed a basement membrane-like structure (Fig. 15A: arrow head), and the outermost layer was thinly covered with ciliated cells (Fig. 15A, B, C: region indicated with "→"). Endocrine cells containing many secretory granules were scattered, but these cells maintained a certain immature state, and the type of endocrine cells was not clear. The endocrine cells seemed to exhibit strong polarity in the direction of the basement membrane and the boundary with the outside, and for example, desmosome was unevenly distributed in the outside layer (Fig. 15C: arrow head). In addition, the presence of folliculo-stellate cells (FSCs) was also observed within the organoid wall (Fig. 15D: broken line). The scale bar at the bottom right of Fig. 15A indicates 8 pm, the scale bar at the lower right of Fig. 15B, C indicates 2 µm, and the scale bar at the lower right of Fig. 15D indicates 500 nm.

### [Example 13: Study of presence of pituitary stem cell in cell aggregate containing pituitary tissue (pituitary organoid) by immunostaining]

The follicular stellate cells in the pituitary confirmed in Example 12 have been reported to be a type of adult pituitary stem cells, and it was suggested that the pituitary-hypothalamic organoid produced by the present production method contain not only hormone-producing cells but also pituitary stem cells. Therefore, cell aggregates containing pituitary tissue were produced in the same manner as in Example 10, and the cell aggregates on day 103 after the start of suspension culturing (differentiation induction) were subjected to immunostaining with CXADR, a marker for pituitary stem cell. Specifically, cryosections were prepared as in Example 3. These cryosections were subjected to immunostaining with anti-ACTH antibody (mouse, 1:200; Fitzgerald) and anti-CXADR antibody (rabbit; 1:100; Atlas antibodies), and cell nuclei were stained with DAPI. These stained sections were observed using confocal laser scanning microscopy (manufactured by OLYMPUS CORPORATION) and immunostained images were obtained.

As a result, in the region where the pituitary tissue is present, ACTH-positive cells were present inside the cell aggregate, and CXADR-positive and ACTH-negative cells were present on the opposite side (outside the cell aggregate) (Fig. 16). Therefore, it was suggested that hormone-producing cells and pituitary stem cells exhibit polarity. The presence of pituitary stem cell in pituitary-hypothalamic organoids induced from pluripotent stem cells has not been reported so far, and was confirmed for the first time in this Example. The scale bar at the bottom right of Fig. 16 indicates 50 µm.

### [Example 14: Study of ACTH secretory capacity of cell aggregates containing pituitary tissue (pituitary organoids)]

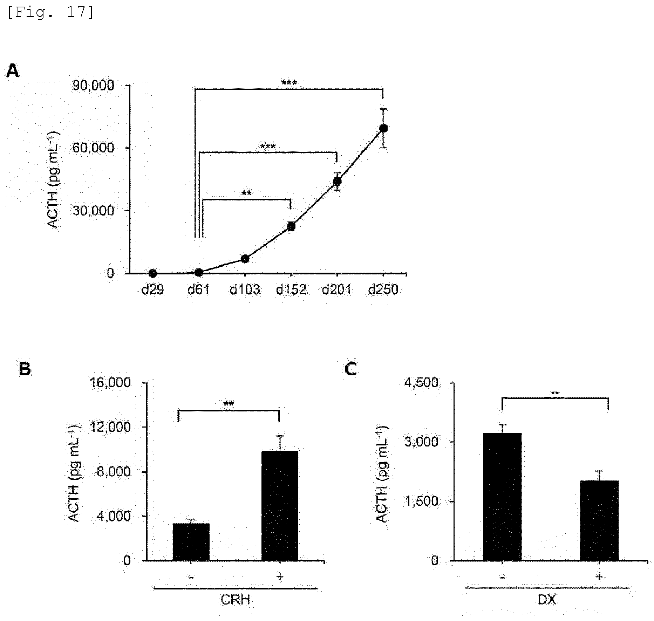

Cell aggregates containing pituitary tissue were produced in the same manner as in Example 10, and ACTH secretory capacity was studied. 20 cell aggregates with different numbers of culture days after the start of suspension culturing were transferred to a 10 cm suspension culture dish containing 10 mL of serum-free medium supplemented with 20% KSR, and after culturing for 3 - 4 days at 37°C, the culture supernatant was recovered. The ACTH concentration in the recovered culture supernatant was measured by the ELISA method used in clinical testing (testing outsourced to SRL, Ltd.). As a result, ACTH secretion was observed from day 29 after the start of suspension culturing (23 pg/mL), and the amount of ACTH secretion increased remarkably as the number of days of culture increased (Fig. 17A).

Then, it was studied whether an increase in ACTH secretion by CRH and suppression of ACTH secretion by glucocorticoid were observed. Specifically, 20 cell aggregates on day 103 after the start of suspension culturing were transferred to a 10 cm suspension culture dish containing 10 mL of serum-free medium supplemented with 20% KSR, and after culturing for 24 hr under conditions of 37°C, 40% O₂, the culture supernatant was recovered. After washing the cell aggregates with serum-free medium supplemented with 20% KSR, the cell aggregates were transferred to a 10 cm suspension culture dish containing 10 mL of fresh serum-free medium supplemented with 20% KSR, and 5 ug/mL CRH (manufactured by Nipro ES Pharma) or 500 ng/mL dexamethasone (DX, manufactured by Aspen Japan) was added. In each case, a no addition group was set as a control. After culturing for 24 hr under conditions of 37°C, 40% O₂, the culture supernatant was recovered. The ACTH concentration in the recovered culture supernatant was measured as described above. As a result, CRH stimulation increased the ACTH secretion amount by about 3 times (Fig. 17B).

On the other hand, DX treatment reduced the amount of ACTH secretion by about 40% (Fig. 17C). These results show that homeostasis due to hormones was maintained in the pituitary-hypothalamic organoids produced by the present production method.

### [Example 15: Identification of unintended cells emerged in pituitary organoid produced from human ES cells (gene analysis/immunostaining)]

In order to identify unintended cells, genes expressed in suspension cultured cell aggregates were identified using a gene expression analysis method. For analysis, human ES cells (KhES-1 strain) free of differentiation induction, and cell aggregates produced according to the steps shown in Fig. 13A and on days 30, 60, and 100 after the start of suspension culturing (5 or 6 cell aggregates were prepared for each), and samples for gene analysis were prepared using RLT buffer and RNeasy Micro Kit (both manufactured by Qiagen) for each cell aggregate (17 samples in total). The prepared RNA samples were analyzed using Biomark HD (Fluidigm). As the probe, TaqMan probe (GAPDH (Hs02758991_g1), ACTB (Hs01060665-g1), PITX1 (Hs00267528-m1), PITX2 (Hs04234069-mH), LHX3 (Hs01033412_m1), POMC (Hs01596743_m1), E-cadherin (Hs01023895_m1), EpCAM (Hs00901885_m1), RAX (Hs00429459-m1), TTF1 (Hs00968940-m1), NESTIN (Hs04187831-g1), SOX11 (Hs00846583_s1), LIN28A (Hs00702808-s1), NANOG (Hs04260366-g1), POU5F1 (Hs04260367-gH), manufactured by Thermo Fisher Scientific, were used. As a result, as shown in the Heat map of Fig. 18A, it was found that neural progenitor cell markers NESTIN and SOX11 were expressed in tissues 30, 60, and 100 days after the start of suspension culturing.

Then, cryosections were prepared from the tissue on day 103 after the start of suspension culturing and in the same manner as in Example 3. The sections were subjected to immunostaining using antibodies against NESTIN (mouse; 1:500; manufactured by R&D Systems Company) and SOX11 (sheep; 1:100; manufactured by R&D Systems Company), and cell nuclei were stained with DAPI. As a result, it was found that in a plurality of cell aggregates, NESTIN-positive and SOX11-positive cells were present in the cell layer inside the aggregates (Fig. 18B). From the above-mentioned results, it was shown that that pituitary tissue, hypothalamic tissue, and neural progenitor cells were present in the cell mass induced to differentiate from pluripotent stem cells (Fig. 18B). The scale bar at the bottom right of Fig. 18B, a, indicates 200 um and the scale bar at the bottom right of Figs. 18B, b-d, indicates 50 µm.

### [Example 16: Production and differentiation induction of pituitary organoid from human iPS cells by using culture vessel with divided microwells]

According to the steps shown in Fig. 19A, pituitary organoid was produced from human pluripotent stem cells by using a culture vessel with divided microwells. Human iPS cell line 1231A3 was used as the pluripotent stem cell. The cells to be used for differentiation induction were prepared in the same manner as in Example 1.

2 ml of a serum-free medium for differentiation induction described below was added to a 35 mm EZSPHERE Dish Type 905 (manufactured by Asahi Techno Glass Co., Ltd.) in advance and allowed to stand in a CO₂ incubator for about 1 hr, and removal of air bubbles in the microwell was confirmed using a microscope.

Thereafter, the prepared subconfluent human iPS cell 1231A3 strain was treated with 5 mM EDTA/PBS in the same manner as in the method described in Example 1. When collecting cells, a serum-free medium for differentiation induction was added, pipetting was performed, and the cells were scraped from the surface of the culture dish and dispersed into single cells. The cells were suspended in 3 ml of a serum-free medium at 1.8×10⁶ cells per dish of a 35 mm EZSPHERE Dish prepared in advance and suspension cultured under conditions of 37°C, 5% CO₂. As the serum-free medium therefor, a 5% KSR gfCDM was used (start of step (1)).

At the start of suspension culturing (day 0 after the start of suspension culturing, start of step (1)), Y-27632 (final concentration 10 µM), IWP-2 (final concentration 1 µM), SB431542 (final concentration 1 µM), JNK-IN-8 (final concentration 1 µM), and SAG (final concentration 100 nM) were added to the above-mentioned serum-free medium.

After confirming the formation of cell aggregates in the microwells on the bottom of the EZSPHERE Dish using a microscope on day 1 after the start of suspension culturing, a serum-free medium not containing Y-27632 but containing IWP-2, SB431542, JNK-IN-8, SAG (final concentration 700 nM), and BMP4 (final concentration 0.5 nM) was added by 1 ml per dish (start of step (2)).

Further, on day 3 after the start of suspension culturing, cell aggregates were collected using a wide-bore 1000 µl pipette tip and transferred to a 10 cm suspension culture dish. As the medium therefor, 12 ml of 5% KSR gfCDM not containing both Y-27632 and BMP4 but containing IWP-2, SB431542, JNK-IN-8, SAG (final concentration 700 nM) was used. Thereafter, on days 6, 10, 13, and 17 of culture, half of the medium was replaced by recovering 6 ml of the medium from the dish without sucking up the cell aggregates, and adding 6 ml of a new medium.

On day 29 after the start of suspension culturing, oblique illumination observation was performed using an inverted fluorescence microscope (BIOREVO BZ-X800, manufactured by Keyence Corporation) (Fig. 19B). As a result, pituitary organoid with placode-like pituitary tissue observed as a thick, highly transparent epithelium on the surface of the cell mass was formed. From the above-mentioned results, it was found that pituitary organoids with pituitary tissue on the surface can be produced from human pluripotent stem cells by using a culture vessel with divided microwells, as in the aforementioned case of using the 96-well microwell plate. Large-scale production of pituitary tissue becomes possible by using a culture vessel with divided microwells.

### [Example 17: Study of timing of addition of BMP under JNK inhibitor addition conditions in production of cell population containing pituitary tissue (pituitary organoid) from human iPS cell]

In Example 17, a cell population containing cells constituting pituitary tissue was produced according to the steps shown in Fig. 20A. Specifically, human iPS cells (1231A3 strain, obtained from RIKEN) were cultured under feeder-free conditions according to the method described in Scientific Reports, 4, 3594 (2014). As the feeder-free medium, StemFit medium was used and, as the feeder-free scaffold, Laminin 511-E8 was used.

Differentiation induction was performed under the same conditions as in Example 1, except that the addition conditions of BMP4 and the final concentration of IWP-2 were changed to 1 µM. As a condition for adding BMP4, step (2) was performed about 24 hr (day 1) and about 48 hr (day 2) after the start of differentiation induction.

On day 28 after the start of suspension culturing, oblique illumination observation was performed using an inverted fluorescence microscope (BIOREVO BZ-X800, manufactured by Keyence Corporation) (Fig. 20B). As a result, pituitary organoid with placode-like pituitary tissue observed as a thick, highly transparent epithelium on the surface of the cell mass was formed, under both BMP4 addition conditions of about 24 hr (day 1) and about 48 hr (day 2) after the start of differentiation induction. Comparing the above-mentioned conditions, the size of the pituitary organoids formed was more uniform, a large proportion of the surface of the cell mass was covered with pituitary tissue, and the proportion of cells other than pituitary was low, under the addition condition of BMP4 after 24 hr (day 1). From the above-mentioned results, one preferred embodiment of the addition timing of the BMP signal transduction pathway activating substance during the production of pituitary organoid under JNK inhibitor addition condition is shown to be after 12 hr and within 60 hr from the start of differentiation induction of human pluripotent stem cells.

This application is based on patent application No. 2021-162255 filed in Japan (filing date: September 30, 2021) and patent application No. 2022-116716 (filing date: July 21, 2022), the contents of which are incorporated in full herein.

## Claims

1. A method for producing a cell population containing pituitary tissue, comprising the following steps (1) and (2):
(1) a first step of culturing the pluripotent stem cells in the presence of a c-jun N-terminal kinase (JNK) signal transduction pathway inhibiting substance and a Wnt signal transduction pathway inhibiting substance to obtain a cell population,
(2) a second step of culturing the cell population obtained in the first step in the presence of a BMP signal transduction pathway activating substance and a Sonic hedgehog signal transduction pathway activating substance, thereby obtaining a cell population containing pituitary tissue.

2. The production method according to claim 1, wherein the pluripotent stem cell is subjected to the following step (a) before the first step:
(a) step a of culturing pluripotent stem cells in the absence of feeder cells and in a medium containing 1) a TGFβ family signal transduction pathway inhibiting substance and/or a Sonic hedgehog signal transduction pathway activating substance, and 2) a factor for maintaining an undifferentiated state.

3. The production method according to claim 1, wherein the culture in the first step is further in the presence of a Sonic hedgehog signal transduction pathway activating substance, and a period of the culture in the presence of a Sonic hedgehog signal transduction pathway activating substance in the first step and the second step is 30 days.

4. The production method according to claim 1, wherein the cell population obtained in the second step is subjected to the following step (3):
(3) a third step of culturing the cell population obtained in the second step in the absence of a Sonic hedgehog signal transduction pathway activating substance to obtain a cell population containing pituitary tissue.

5. The production method according to claim 4, wherein the cell population obtained in the second step is subjected to the following step (b) before the third step:
(b) step b of culturing the cell population obtained in the second step, in the presence of a BMP signal transduction pathway inhibiting substance.

6. The production method according to claim 1, wherein the JNK signal transduction pathway inhibiting substance comprises a JNK inhibitor.

7. The production method according to claim 1, wherein the JNK signal transduction pathway inhibiting substance comprises a Rac inhibitor.

8. The production method according to claim 1, wherein the Wnt signal transduction pathway inhibiting substance comprises a substance with inhibitory activity against non-classical Wnt pathway.

9. The production method according to any one of claims 1 to 8, wherein a TGF signal transduction pathway inhibiting substance is further present in any one or more of the first step, second step, step b, and the third step.

10. The production method according to any one of claims 1 to 8, wherein a TAK1 inhibiting substance is further present in any one or more of the first step, the second step, step b, and the third step.

11. The production method according to any one of claims 1 to 8, wherein a FGF signal transduction pathway activating substance is further present in any one or more of the second step, step b, and the third step.

12. The production method according to any one of claims 1 to 8, wherein a substance having the action of reducing oxidative stress is further present in any one or more of the second step, step b, and the third step.

13. The production method according to any one of claims 1 to 8, wherein an inhibiting substance against a stress response signal transduction pathway is further present in any one or more of the second step, step b, and the third step.

14. The production method according to any one of claims 1 to 8, wherein cells are cultivated while shaking in any one or more of the second step, step b, and the third step.

15. The production method according to any one of claims 1 to 8, wherein the cell population obtained in the first step is a cell aggregate.

16. The production method according to any one of claims 1 to 8, wherein any one or more of the first step, the second step, step b, and the third step is/are conducted in a culture vessel in which at least one well is formed, the well is divided into a plurality of microwells, and suspension cultured is performed so that one cell mass is formed in each microwell.

17. A method for producing a pituitary tissue, comprising collecting the pituitary tissue from a cell population containing a pituitary tissue, which is obtained by the production method according to any one of claims 1 to 8.
